# EUROPEAN PATENT APPLICATION

(11) **EP 2 832 855 A1**
(43) Date of publication of application: **04.02.2015**
(21) Application number: 13767251.5
(22) Date of filing: 29.03.2013
(51) Int. Cl.: C12N 15/09, A61K 39/395, A61K 45/00, A61P 19/02, A61P 19/10, A61P 29/00, A61P 35/00, A61P 35/04, C07K 16/18, C07K 16/46, C12N 1/15, C12N 1/19, C12N 1/21, C12N 5/10, C12P 21/08

(54) **NOVEL ANTI-SIGLEC15 ANTIBODY**

(30) Priority: 30.03.2012 JP 2012078841
(71) Applicant: Daiichi Sankyo Company, Limited, Toyko 103-8426 (JP)
(72) Inventor: HIRUMA, Yoshiharu, Tokyo 140-8710 (JP); HASEGAWA, Jun, Tokyo 140-8710 (JP)
(74) Representative: Arends, William Gerrit
(86) International application number: PCT/JP2013/059653
(87) International publication number: WO 2013/147212

(57) **Abstract**

Provided is a pharmaceutical composition for the treatment and/or prophylaxis of abnormal bone metabolism targeting a protein encoded by a gene strongly expressed in osteoclasts. Specifically provided is a pharmaceutical composition containing an antibody which specifically recognizes human Siglec-15 and has an activity of inhibiting osteoclast formation, and the like.

SEQUENCE LISTING

<110>  DAIICHI SANKYO COMPANY, LIMITED

<120>  Novel anti-Siglec-15 antibodies

<130>  FP1309

<150>  JP2012-078841
<151>  2012-03-30

<160>  49

<170>  PatentIn version 3.5

<210>  1
<211>  363
<212>  DNA
<213>  Rattus norvegicus

<220>
<221>  CDS
<222>  (1)..(363)

<400>  1
cag gtc cag ctg cag cag tct gga gct gag ctg aca aag cct ggc tct      48
Gln Val Gln Leu Gln Gln Ser Gly Ala Glu Leu Thr Lys Pro Gly Ser
1               5                   10                  15

tca gtg aag att tcc tgc aag act tct ggc tac acc ttt ccc agg tac      96
Ser Val Lys Ile Ser Cys Lys Thr Ser Gly Tyr Thr Phe Pro Arg Tyr
            20                  25                  30

gat gta agt tgg atc aag cag agg cct gga cag gcc ctt gag tgg att      144
Asp Val Ser Trp Ile Lys Gln Arg Pro Gly Gln Ala Leu Glu Trp Ile
        35                  40                  45

gga gtc att cat cca gga agt gga gga aca ggg tac aat gag aag ttc      192
Gly Val Ile His Pro Gly Ser Gly Gly Thr Gly Tyr Asn Glu Lys Phe
    50                  55                  60

aag gcc aag gcc aca ttg gct gtg gac aca tcc tcc agc aca gcc ttc      240
Lys Ala Lys Ala Thr Leu Ala Val Asp Thr Ser Ser Ser Thr Ala Phe
65                  70                  75                  80

atg caa ctc agc agc ctg aca cct gag gac act gcg gtc tat tac tgt      288
Met Gln Leu Ser Ser Leu Thr Pro Glu Asp Thr Ala Val Tyr Tyr Cys
                85                  90                  95

gca aga cgg ggc ctc aat tcg ggg tac tgg ttc ttt gac ttc tgg ggc      336
Ala Arg Arg Gly Leu Asn Ser Gly Tyr Trp Phe Phe Asp Phe Trp Gly
            100                 105                 110

cca gga acc ctg gtc act gtg tcc tca                                  363
Pro Gly Thr Leu Val Thr Val Ser Ser
        115                 120

<210>  2
<211>  121
<212>  PRT
<213>  Rattus norvegicus

<400>  2

Gln Val Gln Leu Gln Gln Ser Gly Ala Glu Leu Thr Lys Pro Gly Ser
1               5                   10                  15

Ser Val Lys Ile Ser Cys Lys Thr Ser Gly Tyr Thr Phe Pro Arg Tyr
            20                  25                  30

Asp Val Ser Trp Ile Lys Gln Arg Pro Gly Gln Ala Leu Glu Trp Ile
        35                  40                  45

Gly Val Ile His Pro Gly Ser Gly Gly Thr Gly Tyr Asn Glu Lys Phe
    50                  55                  60

Lys Ala Lys Ala Thr Leu Ala Val Asp Thr Ser Ser Ser Thr Ala Phe
65                  70                  75                  80

Met Gln Leu Ser Ser Leu Thr Pro Glu Asp Thr Ala Val Tyr Tyr Cys
                85                  90                  95

Ala Arg Arg Gly Leu Asn Ser Gly Tyr Trp Phe Phe Asp Phe Trp Gly
            100                 105                 110

Pro Gly Thr Leu Val Thr Val Ser Ser
        115                 120

<210>  3
<211>  327
<212>  DNA
<213>  Rattus norvegicus

<220>
<221>  CDS
<222>  (1)..(327)

<400>  3
gac att gtg atg act cag tct ccc aca tcc atg tcc ata tca gta gga      48
Asp Ile Val Met Thr Gln Ser Pro Thr Ser Met Ser Ile Ser Val Gly
1               5                   10                  15

gac agg gtc acc atg aac tgc aag gcc agt caa aat gtg ggt tct aat      96
Asp Arg Val Thr Met Asn Cys Lys Ala Ser Gln Asn Val Gly Ser Asn
            20                  25                  30

gta gac tgg tac caa cag aaa aca gga cag tct cct aaa ctg ctt atc      144
Val Asp Trp Tyr Gln Gln Lys Thr Gly Gln Ser Pro Lys Leu Leu Ile
        35                  40                  45

tac gaa tca acc aac cgg tac acg gga gtc cct gat cgc ttc aca ggc      192
Tyr Glu Ser Thr Asn Arg Tyr Thr Gly Val Pro Asp Arg Phe Thr Gly
    50                  55                  60

agt gga tct gga aca gac ttc act ttc acc atc agc aac gtg cag gct      240

Ser Gly Ser Gly Thr Asp Phe Thr Phe Thr Ile Ser Asn Val Gln Ala
65                  70                  75                  80

gaa gac ctg gct gtt tat tac tgt atg cag tct aac ttt ttt ccc ttc      288
Glu Asp Leu Ala Val Tyr Tyr Cys Met Gln Ser Asn Phe Phe Pro Phe
                85                  90                  95

acg ttc ggt tct ggg atc aag ctg gag atc aaa cgg gct                  327
Thr Phe Gly Ser Gly Ile Lys Leu Glu Ile Lys Arg Ala
            100                 105

<210>  4
<211>  109
<212>  PRT
<213>  Rattus norvegicus

<400>  4

Asp Ile Val Met Thr Gln Ser Pro Thr Ser Met Ser Ile Ser Val Gly
1               5                   10                  15

Asp Arg Val Thr Met Asn Cys Lys Ala Ser Gln Asn Val Gly Ser Asn
            20                  25                  30

Val Asp Trp Tyr Gln Gln Lys Thr Gly Gln Ser Pro Lys Leu Leu Ile
        35                  40                  45

Tyr Glu Ser Thr Asn Arg Tyr Thr Gly Val Pro Asp Arg Phe Thr Gly
    50                  55                  60

Ser Gly Ser Gly Thr Asp Phe Thr Phe Thr Ile Ser Asn Val Gln Ala
65                  70                  75                  80

Glu Asp Leu Ala Val Tyr Tyr Cys Met Gln Ser Asn Phe Phe Pro Phe
                85                  90                  95

Thr Phe Gly Ser Gly Ile Lys Leu Glu Ile Lys Arg Ala
            100                 105

<210>  5
<211>  449
<212>  DNA
<213>  Artificial Sequence

<220>
<223>  Nucleotide coding signal and constant regions of human kappa
       chain

<400>  5
gcctccggac tctagagcca ccatggtgct gcagacccag gtgttcatct ccctgctgct      60

gtggatctcc ggcgcgtacg gcgatatcgt gatgattaaa cgtacggtgg ccgccccctc      120

cgtgttcatc ttccccccct ccgacgagca gctgaagtcc ggcaccgcct ccgtggtgtg      180

cctgctgaat aacttctacc ccagagaggc caaggtgcag tggaaggtgg acaacgccct 240

gcagtccggg aactcccagg agagcgtgac cgagcaggac agcaaggaca gcacctacag 300

cctgagcagc accctgaccc tgagcaaagc cgactacgag aagcacaagg tgtacgcctg 360

cgaggtgacc caccagggcc tgagctcccc cgtcaccaag agcttcaaca ggggggagtg 420

ttagggcccc gtttaaacgg gggaggcta 449

<210> 6
<211> 1118
<212> DNA
<213> Artificial Sequence

<220>
<223> Nucleotide coding signal and constant regions of human IgG2 chain

<400> 6
gcctccggac tctagagcca ccatgaaaca cctgtggttc ttcctcctgc tggtggcagc 60

tcccagatgg gtgctgagcc aggtgcaatt gtgcaggcgg ttagctcagc cagcaccaag 120

ggcccttccg tgttccctct ggccccttgt agccgttcca ccagcgagtc caccgccgcc 180

cttggctgtc tggtgaagga ctacttccct gagcctgtga ccgtgagctg gaactccgga 240

gcccttacca gcggcgtgca caccttccct gccgtgctgc agtccagcgg cctttactcc 300

ctgagctccg tggtgaccgt gcctagctcc aacttcggca cccaaaccta cacctgtaac 360

gtggaccaca agcctagcaa caccaaggtg gacaagaccg tggagcgtaa gtgttgtgtg 420

gagtgtcctc cttgtcctgc ccctcctgtg gccggacctt ccgtgttcct tttccctcct 480

aagcctaagg acaccctgat gatcagccgt acccctgagg tgacctgtgt ggtggtggac 540

gtgtcccacg aggaccctga ggtgcagttc aactggtacg tggacggcgt ggaggtgcac 600

aacgccaaga ccaagcctcg tgaggagcaa ttcaacagca ccttccgtgt ggtgtccgtg 660

cttaccgtgg tgcaccaaga ctggctgaac ggcaaggagt acaagtgtaa ggtgagcaac 720

aagggacttc ctgcccctat cgagaagacc atctccaaga ccaagggcca acctcgtgag 780

cctcaagtgt acacccttcc tcctagccgt gaggagatga ccaagaacca agtgtccctt 840

acctgtctgg tgaagggctt ctaccctagc gacatcgccg tggagtggga gtccaacgga 900

caacctgaga acaactacaa gaccacccct cctatgcttg acagcgacgg ctccttcttc 960

ctgtacagca agctgaccgt ggacaagtcc cgttggcaac aaggcaacgt gttcagctgt 1020

tccgtgatgc acgaggccct gcacaaccac tacacccaaa agagcctttc cctgagccct 1080

ggaaagtgat atcgggcccg tttaaacggg ggaggcta 1118

<210> 7
<211> 1398
<212> DNA
<213> Artificial Sequence

<220>
<223>  Nucleotide coding heavy chain of chimeric #24A3


<220>
<221>  CDS
<222>  (1)..(1398)

<400>  7
atg aaa cac ctg tgg ttc ttc ctc ctg ctg gtg gca gct ccc aga tgg      48
Met Lys His Leu Trp Phe Phe Leu Leu Leu Val Ala Ala Pro Arg Trp
1               5                   10                  15

gtg ctg agc cag gtc cag ctg cag cag tct gga gct gag ctg aca aag      96
Val Leu Ser Gln Val Gln Leu Gln Gln Ser Gly Ala Glu Leu Thr Lys
            20                  25                  30

cct ggc tct tca gtg aag att tcc tgc aag act tct ggc tac acc ttt      144
Pro Gly Ser Ser Val Lys Ile Ser Cys Lys Thr Ser Gly Tyr Thr Phe
        35                  40                  45

ccc agg tac gat gta agt tgg atc aag cag agg cct gga cag gcc ctt      192
Pro Arg Tyr Asp Val Ser Trp Ile Lys Gln Arg Pro Gly Gln Ala Leu
    50                  55                  60

gag tgg att gga gtc att cat cca gga agt gga gga aca ggg tac aat      240
Glu Trp Ile Gly Val Ile His Pro Gly Ser Gly Gly Thr Gly Tyr Asn
65                  70                  75                  80

gag aag ttc aag gcc aag gcc aca ttg gct gtg gac aca tcc tcc agc      288
Glu Lys Phe Lys Ala Lys Ala Thr Leu Ala Val Asp Thr Ser Ser Ser
                85                  90                  95

aca gcc ttc atg caa ctc agc agc ctg aca cct gag gac act gcg gtc      336
Thr Ala Phe Met Gln Leu Ser Ser Leu Thr Pro Glu Asp Thr Ala Val
            100                 105                 110

tat tac tgt gca aga cgg ggc ctc aat tcg ggg tac tgg ttc ttt gac      384
Tyr Tyr Cys Ala Arg Arg Gly Leu Asn Ser Gly Tyr Trp Phe Phe Asp
        115                 120                 125

ttc tgg ggc cca gga acc ctg gtc act gtg agc tca gcc agc acc aag      432
Phe Trp Gly Pro Gly Thr Leu Val Thr Val Ser Ser Ala Ser Thr Lys
    130                 135                 140

ggc cct tcc gtg ttc cct ctg gcc cct tgt agc cgt tcc acc agc gag      480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct      528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc      576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg      624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac      672

Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt      720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga      768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc      816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag      864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac      912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt      960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag     1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag     1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac     1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt     1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg     1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg     1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac     1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac     1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct     1392
Glu Ala Leu His Asn His Tyr Thr Gln Lys Ser Leu Ser Leu Ser Pro
    450                 455                 460

gga aag                                                                 1398
Gly Lys
465


<210>  8
<211>  466
<212>  PRT
<213>  Artificial Sequence

<220>
<223>  Synthetic Construct

<400>  8

Met Lys His Leu Trp Phe Phe Leu Leu Leu Val Ala Ala Pro Arg Trp
1               5                   10                  15


Val Leu Ser Gln Val Gln Leu Gln Gln Ser Gly Ala Glu Leu Thr Lys
            20                  25                  30


Pro Gly Ser Ser Val Lys Ile Ser Cys Lys Thr Ser Gly Tyr Thr Phe
        35                  40                  45


Pro Arg Tyr Asp Val Ser Trp Ile Lys Gln Arg Pro Gly Gln Ala Leu
    50                  55                  60


Glu Trp Ile Gly Val Ile His Pro Gly Ser Gly Gly Thr Gly Tyr Asn
65                  70                  75                  80


Glu Lys Phe Lys Ala Lys Ala Thr Leu Ala Val Asp Thr Ser Ser Ser
                85                  90                  95


Thr Ala Phe Met Gln Leu Ser Ser Leu Thr Pro Glu Asp Thr Ala Val
            100                 105                 110


Tyr Tyr Cys Ala Arg Arg Gly Leu Asn Ser Gly Tyr Trp Phe Phe Asp
        115                 120                 125


Phe Trp Gly Pro Gly Thr Leu Val Thr Val Ser Ser Ala Ser Thr Lys
    130                 135                 140


Gly Pro Ser Val Phe Pro Leu Ala Pro Cys Ser Arg Ser Thr Ser Glu
145                 150                 155                 160


Ser Thr Ala Ala Leu Gly Cys Leu Val Lys Asp Tyr Phe Pro Glu Pro
                165                 170                 175


Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  9
<211>  702
<212>  DNA
<213>  Artificial Sequence

<220>
<223>  Nucleotide conding light chain of chimeric #24A3

<220>
<221>  CDS
<222>  (1)..(702)

<400>  9
atg gtg ctg cag acc cag gtg ttc atc tcc ctg ctg ctg tgg atc tcc      48
Met Val Leu Gln Thr Gln Val Phe Ile Ser Leu Leu Leu Trp Ile Ser
1               5                   10                  15

ggc gcg tac ggc gac att gtg atg act cag tct ccc aca tcc atg tcc      96
Gly Ala Tyr Gly Asp Ile Val Met Thr Gln Ser Pro Thr Ser Met Ser
            20                  25                  30

ata tca gta gga gac agg gtc acc atg aac tgc aag gcc agt caa aat      144
Ile Ser Val Gly Asp Arg Val Thr Met Asn Cys Lys Ala Ser Gln Asn
        35                  40                  45

gtg ggt tct aat gta gac tgg tac caa cag aaa aca gga cag tct cct      192
Val Gly Ser Asn Val Asp Trp Tyr Gln Gln Lys Thr Gly Gln Ser Pro
    50                  55                  60

aaa ctg ctt atc tac gaa tca acc aac cgg tac acg gga gtc cct gat      240
Lys Leu Leu Ile Tyr Glu Ser Thr Asn Arg Tyr Thr Gly Val Pro Asp
65                  70                  75                  80

cgc ttc aca ggc agt gga tct gga aca gac ttc act ttc acc atc agc      288
Arg Phe Thr Gly Ser Gly Ser Gly Thr Asp Phe Thr Phe Thr Ile Ser
                85                  90                  95

aac gtg cag gct gaa gac ctg gct gtt tat tac tgt atg cag tct aac      336
Asn Val Gln Ala Glu Asp Leu Ala Val Tyr Tyr Cys Met Gln Ser Asn
            100                 105                 110

ttt ttt ccc ttc acg ttc ggt tct ggg atc aag ctg gag atc aaa cgg      384
Phe Phe Pro Phe Thr Phe Gly Ser Gly Ile Lys Leu Glu Ile Lys Arg
        115                 120                 125

gct gtg gcc gcc ccc tcc gtg ttc atc ttc ccc ccc tcc gac gag cag      432
Ala Val Ala Ala Pro Ser Val Phe Ile Phe Pro Pro Ser Asp Glu Gln
    130                 135                 140

ctg aag tcc ggc acc gcc tcc gtg gtg tgc ctg ctg aat aac ttc tac      480
Leu Lys Ser Gly Thr Ala Ser Val Val Cys Leu Leu Asn Asn Phe Tyr
145                 150                 155                 160

ccc aga gag gcc aag gtg cag tgg aag gtg gac aac gcc ctg cag tcc     528
Pro Arg Glu Ala Lys Val Gln Trp Lys Val Asp Asn Ala Leu Gln Ser
                165                 170                 175

ggg aac tcc cag gag agc gtg acc gag cag gac agc aag gac agc acc     576
Gly Asn Ser Gln Glu Ser Val Thr Glu Gln Asp Ser Lys Asp Ser Thr
            180                 185                 190

tac agc ctg agc agc acc ctg acc ctg agc aaa gcc gac tac gag aag     624
Tyr Ser Leu Ser Ser Thr Leu Thr Leu Ser Lys Ala Asp Tyr Glu Lys
        195                 200                 205

cac aag gtg tac gcc tgc gag gtg acc cac cag ggc ctg agc tcc ccc     672
His Lys Val Tyr Ala Cys Glu Val Thr His Gln Gly Leu Ser Ser Pro
    210                 215                 220

gtc acc aag agc ttc aac agg ggg gag tgt                             702
Val Thr Lys Ser Phe Asn Arg Gly Glu Cys
225                 230


<210>  10
<211>  234
<212>  PRT
<213>  Artificial Sequence

<220>
<223>  Synthetic Construct

<400>  10

Met Val Leu Gln Thr Gln Val Phe Ile Ser Leu Leu Leu Trp Ile Ser
1               5                   10                  15


Gly Ala Tyr Gly Asp Ile Val Met Thr Gln Ser Pro Thr Ser Met Ser
            20                  25                  30


Ile Ser Val Gly Asp Arg Val Thr Met Asn Cys Lys Ala Ser Gln Asn
        35                  40                  45


Val Gly Ser Asn Val Asp Trp Tyr Gln Gln Lys Thr Gly Gln Ser Pro
    50                  55                  60


Lys Leu Leu Ile Tyr Glu Ser Thr Asn Arg Tyr Thr Gly Val Pro Asp
65                  70                  75                  80


Arg Phe Thr Gly Ser Gly Ser Gly Thr Asp Phe Thr Phe Thr Ile Ser
                85                  90                  95


Asn Val Gln Ala Glu Asp Leu Ala Val Tyr Tyr Cys Met Gln Ser Asn
            100                 105                 110


Phe Phe Pro Phe Thr Phe Gly Ser Gly Ile Lys Leu Glu Ile Lys Arg
        115                 120                 125

Ala Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  11
<211>  1398
<212>  DNA
<213>  Artificial Sequence

<220>
<223>  Nucleotide coding h#24A3-H1

<220>
<221>  CDS
<222>  (1)..(1398)

<400>  11
atg aaa cac ctg tgg ttc ttc ctc ctg ctg gtg gca gct ccc aga tgg        48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa        96
Val Leu Ser Gln Val Gln Leu Val Gln Ser Gly Ala Glu Val Lys Lys
            20                  25                  30

cct gga gcc agc gtg aag gtg tcc tgc aag gcc agc ggc tac acc ttc       144
Pro Gly Ala Ser Val Lys Val Ser Cys Lys Ala Ser Gly Tyr Thr Phe
        35                  40                  45

acc aga tac gac gtg tcc tgg gtg cgc cag gcc cca ggc cag gga ctg       192
Thr Arg Tyr Asp Val Ser Trp Val Arg Gln Ala Pro Gly Gln Gly Leu
    50                  55                  60

gaa tgg atg ggc gtg atc cac cct ggc agc ggc ggc acc ggc tac aac       240
Glu Trp Met Gly Val Ile His Pro Gly Ser Gly Gly Thr Gly Tyr Asn
65                  70                  75                  80

gag aag ttc aag gcc aga gtg acc atc acc gcc gac acc agc acc agc       288

Glu Lys Phe Lys Ala Arg Val Thr Ile Thr Ala Asp Thr Ser Thr Ser
                85                  90                  95

acc gcc tac atg gaa ctg agc agc ctg cgg agc gag gac acc gcc gtg      336
Thr Ala Tyr Met Glu Leu Ser Ser Leu Arg Ser Glu Asp Thr Ala Val
            100                 105                 110

tac tac tgc gcc aga cgg ggc ctg aac agc ggc tac tgg ttc ttc gac      384
Tyr Tyr Cys Ala Arg Arg Gly Leu Asn Ser Gly Tyr Trp Phe Phe Asp
        115                 120                 125

ttc tgg ggc cag ggc acc ctg gtg acc gtc agc tca gcc agc acc aag      432
Phe Trp Gly Gln Gly Thr Leu Val Thr Val Ser Ser Ala Ser Thr Lys
    130                 135                 140

ggc cct tcc gtg ttc cct ctg gcc cct tgt agc cgt tcc acc agc gag      480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct      528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc      576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg      624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac      672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt      720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga      768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc      816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag      864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac      912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt      960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag     1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag 1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac 1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt 1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg 1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg 1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac 1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac 1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct 1392
Glu Ala Leu His Asn His Tyr Thr Gln Lys Ser Leu Ser Leu Ser Pro
    450                 455                 460

gga aag 1398
Gly Lys
465

<210> 12
<211> 466
<212> PRT
<213> Artificial Sequence

<220>
<223> Synthetic Construct

<400> 12

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Arg Tyr Asp Val Ser Trp Val Arg Gln Ala Pro Gly Gln Gly Leu
    50                  55                  60

Glu Trp Met Gly Val Ile His Pro Gly Ser Gly Gly Thr Gly Tyr Asn
65                  70                  75                  80

Glu Lys Phe Lys Ala Arg Val Thr Ile Thr Ala Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Arg Gly Leu Asn Ser Gly Tyr Trp Phe Phe Asp
        115                 120                 125

Phe Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  13
<211>  1398
<212>  DNA
<213>  Artificial Sequence

<220>
<223>  Nucleotide coding h#24A3-H2

<220>
<221>  CDS
<222>  (1)..(1398)

<400>  13
atg aaa cac ctg tgg ttc ttc ctc ctg ctg gtg gca gct ccc aga tgg      48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa      96
Val Leu Ser Gln Val Gln Leu Val Gln Ser Gly Ala Glu Val Lys Lys
            20                  25                  30

cct gga gcc agc gtg aag gtg tcc tgc aag gcc agc ggc tac acc ttc 144
Pro Gly Ala Ser Val Lys Val Ser Cys Lys Ala Ser Gly Tyr Thr Phe
        35                  40                  45

ccc aga tac gac gtg tcc tgg gtg cgc cag gcc cca ggc cag gga ctg 192
Pro Arg Tyr Asp Val Ser Trp Val Arg Gln Ala Pro Gly Gln Gly Leu
    50                  55                  60

gaa tgg atg ggc gtg atc cac cct ggc agc ggc ggc acc ggc tac aac 240
Glu Trp Met Gly Val Ile His Pro Gly Ser Gly Gly Thr Gly Tyr Asn
65                  70                  75                  80

gag aag ttc aag gcc aga gtg acc atc acc gcc gac acc agc acc agc 288
Glu Lys Phe Lys Ala Arg Val Thr Ile Thr Ala Asp Thr Ser Thr Ser
                85                  90                  95

acc gcc tac atg gaa ctg agc agc ctg cgg agc gag gac acc gcc gtg 336
Thr Ala Tyr Met Glu Leu Ser Ser Leu Arg Ser Glu Asp Thr Ala Val
            100                 105                 110

tac tac tgc gcc aga cgg ggc ctg aac agc ggc tac tgg ttc ttc gac 384
Tyr Tyr Cys Ala Arg Arg Gly Leu Asn Ser Gly Tyr Trp Phe Phe Asp
        115                 120                 125

ttc tgg ggc cag ggc acc ctg gtg acc gtc agc tca gcc agc acc aag 432
Phe Trp Gly Gln Gly Thr Leu Val Thr Val Ser Ser Ala Ser Thr Lys
    130                 135                 140

ggc cct tcc gtg ttc cct ctg gcc cct tgt agc cgt tcc acc agc gag 480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct 528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc 576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg 624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac 672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt 720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga 768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc 816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag 864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac 912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt 960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag 1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag 1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac 1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt 1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg 1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg 1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac 1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac 1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct 1392
Glu Ala Leu His Asn His Tyr Thr Gln Lys Ser Leu Ser Leu Ser Pro
    450                 455                 460

gga aag 1398
Gly Lys
465

<210> 14
<211> 466
<212> PRT
<213> Artificial Sequence

<220>
<223> Synthetic Construct

<400> 14

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30
Pro Gly Ala Ser Val Lys Val Ser Cys Lys Ala Ser Gly Tyr Thr Phe
        35                  40                  45
Pro Arg Tyr Asp Val Ser Trp Val Arg Gln Ala Pro Gly Gln Gly Leu
    50                  55                  60
Glu Trp Met Gly Val Ile His Pro Gly Ser Gly Gly Thr Gly Tyr Asn
65                  70                  75                  80
Glu Lys Phe Lys Ala Arg Val Thr Ile Thr Ala Asp Thr Ser Thr Ser
                85                  90                  95
Thr Ala Tyr Met Glu Leu Ser Ser Leu Arg Ser Glu Asp Thr Ala Val
            100                 105                 110
Tyr Tyr Cys Ala Arg Arg Gly Leu Asn Ser Gly Tyr Trp Phe Phe Asp
        115                 120                 125
Phe Trp Gly Gln Gly Thr Leu Val Thr Val Ser Ser Ala Ser Thr Lys
    130                 135                 140
Gly Pro Ser Val Phe Pro Leu Ala Pro Cys Ser Arg Ser Thr Ser Glu
145                 150                 155                 160
Ser Thr Ala Ala Leu Gly Cys Leu Val Lys Asp Tyr Phe Pro Glu Pro
                165                 170                 175
Val Thr Val Ser Trp Asn Ser Gly Ala Leu Thr Ser Gly Val His Thr
            180                 185                 190
Phe Pro Ala Val Leu Gln Ser Ser Gly Leu Tyr Ser Leu Ser Ser Val
        195                 200                 205
Val Thr Val Pro Ser Ser Asn Phe Gly Thr Gln Thr Tyr Thr Cys Asn
    210                 215                 220
Val Asp His Lys Pro Ser Asn Thr Lys Val Asp Lys Thr Val Glu Arg
225                 230                 235                 240
Lys Cys Cys Val Glu Cys Pro Pro Cys Pro Ala Pro Pro Val Ala Gly
                245                 250                 255
Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  15
<211>  1398
<212>  DNA
<213>  Artificial Sequence

<220>
<223>  Nucleotide coding h#24A3-H3

<220>
<221>  CDS
<222>  (1)..(1398)

<400>  15
atg aaa cac ctg tgg ttc ttc ctc ctg ctg gtg gca gct ccc aga tgg      48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa      96
Val Leu Ser Gln Val Gln Leu Val Gln Ser Gly Ala Glu Val Lys Lys
            20                  25                  30

cct gga gcc agc gtg aag gtg tcc tgc aag gcc agc ggc tac acc ttc      144
Pro Gly Ala Ser Val Lys Val Ser Cys Lys Ala Ser Gly Tyr Thr Phe
        35                  40                  45

ccc aga tac gac gtg agt tgg atc cgg cag gct cca gga cag ggc ctg      192
Pro Arg Tyr Asp Val Ser Trp Ile Arg Gln Ala Pro Gly Gln Gly Leu
    50                  55                  60

gaa tgg atc ggc gtg atc cac cct ggc agc ggc ggc acc ggc tac aac      240
Glu Trp Ile Gly Val Ile His Pro Gly Ser Gly Gly Thr Gly Tyr Asn
65                  70                  75                  80

gag aag ttc aag gcc aga gcc acc atc acc gtg gac acc agc acc agc      288
Glu Lys Phe Lys Ala Arg Ala Thr Ile Thr Val Asp Thr Ser Thr Ser
                85                  90                  95

acc gcc tac atg gaa ctg agc agc ctg cgg agc gag gac acc gcc gtg      336
Thr Ala Tyr Met Glu Leu Ser Ser Leu Arg Ser Glu Asp Thr Ala Val
            100                 105                 110

tac tac tgc gcc aga cgg ggc ctg aac agc ggc tac tgg ttc ttc gac      384
Tyr Tyr Cys Ala Arg Arg Gly Leu Asn Ser Gly Tyr Trp Phe Phe Asp
        115                 120                 125

ttc tgg ggc cag ggc acc ctg gtg acc gtc agc tca gcc agc acc aag      432
Phe Trp Gly Gln Gly Thr Leu Val Thr Val Ser Ser Ala Ser Thr Lys
    130                 135                 140

ggc cct tcc gtg ttc cct ctg gcc cct tgt agc cgt tcc acc agc gag      480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct      528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc      576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg      624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac      672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt      720
Val Asp His Lys Pro Ser Asn Thr Lys Val Asp Lys Thr Val Glu Arg

225                 230                 235                 240

aag tgt tgt gtg gag tgt cct cct tgt cct gcc cct cct gtg gcc gga         768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc         816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag         864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac         912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt         960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag        1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag        1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac        1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt        1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg        1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg        1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac        1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac        1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct        1392
Glu Ala Leu His Asn His Tyr Thr Gln Lys Ser Leu Ser Leu Ser Pro
    450                 455                 460

gga aag                                                                1398
Gly Lys
465

<210>  16
<211>  466
<212>  PRT
<213>  Artificial Sequence

<220>
<223>  Synthetic Construct

<400>  16

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Pro Arg Tyr Asp Val Ser Trp Ile Arg Gln Ala Pro Gly Gln Gly Leu
    50                  55                  60

Glu Trp Ile Gly Val Ile His Pro Gly Ser Gly Gly Thr Gly Tyr Asn
65                  70                  75                  80

Glu Lys Phe Lys Ala Arg Ala Thr Ile Thr Val Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Arg Gly Leu Asn Ser Gly Tyr Trp Phe Phe Asp
        115                 120                 125

Phe Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn

210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210> 17
<211> 1398
<212> DNA
<213> Artificial Sequence

<220>
<223> Nucleotide coding h#24A3-H4

<220>
<221> CDS
<222> (1)..(1398)

<400> 17
atg aaa cac ctg tgg ttc ttc ctc ctg ctg gtg gca gct ccc aga tgg 48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa 96
Val Leu Ser Gln Val Gln Leu Val Gln Ser Gly Ala Glu Val Lys Lys
            20                  25                  30

cct gga gcc agc gtg aag gtg tcc tgc aag acc agc ggc tac acc ttc 144
Pro Gly Ala Ser Val Lys Val Ser Cys Lys Thr Ser Gly Tyr Thr Phe
        35                  40                  45

ccc aga tac gac gtg tcc tgg atc aag cag gcc cca gga cag ggc ctg 192
Pro Arg Tyr Asp Val Ser Trp Ile Lys Gln Ala Pro Gly Gln Gly Leu
    50                  55                  60

gaa tgg atc ggc gtg atc cac cct ggc agc ggc ggc acc ggc tac aac 240
Glu Trp Ile Gly Val Ile His Pro Gly Ser Gly Gly Thr Gly Tyr Asn
65                  70                  75                  80

gag aag ttc aag gcc aga gcc aca ctg acc gtg gac acc agc acc agc 288
Glu Lys Phe Lys Ala Arg Ala Thr Leu Thr Val Asp Thr Ser Thr Ser
                85                  90                  95

acc gcc tac atg gaa ctg agc agc ctg cgg agc gag gac acc gcc gtg 336
Thr Ala Tyr Met Glu Leu Ser Ser Leu Arg Ser Glu Asp Thr Ala Val
            100                 105                 110

tac tac tgc gcc aga cgg ggc ctg aac agc ggc tac tgg ttc ttc gac 384
Tyr Tyr Cys Ala Arg Arg Gly Leu Asn Ser Gly Tyr Trp Phe Phe Asp
        115                 120                 125

ttc tgg ggc cag ggc acc ctg gtg acc gtc agc tca gcc agc acc aag 432
Phe Trp Gly Gln Gly Thr Leu Val Thr Val Ser Ser Ala Ser Thr Lys
    130                 135                 140

ggc cct tcc gtg ttc cct ctg gcc cct tgt agc cgt tcc acc agc gag 480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct 528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc 576

Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg      624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac      672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt      720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga      768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc      816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag      864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac      912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt      960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag     1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag     1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac     1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt     1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg     1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg     1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac     1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac 1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct 1392
Glu Ala Leu His Asn His Tyr Thr Gln Lys Ser Leu Ser Leu Ser Pro
    450                 455                 460

gga aag 1398
Gly Lys
465

<210> 18
<211> 466
<212> PRT
<213> Artificial Sequence

<220>
<223> Synthetic Construct

<400> 18

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Thr Ser Gly Tyr Thr Phe
        35                  40                  45

Pro Arg Tyr Asp Val Ser Trp Ile Lys Gln Ala Pro Gly Gln Gly Leu
    50                  55                  60

Glu Trp Ile Gly Val Ile His Pro Gly Ser Gly Gly Thr Gly Tyr Asn
65                  70                  75                  80

Glu Lys Phe Lys Ala Arg Ala Thr Leu Thr Val Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Arg Gly Leu Asn Ser Gly Tyr Trp Phe Phe Asp
        115                 120                 125

Phe Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  19
<211>  1398
<212>  DNA
<213>  Artificial Sequence

<220>
<223>  Nucleotide coding h#24A3-H5

<220>
<221>  CDS
<222>  (1)..(1398)

<400>  19
atg aaa cac ctg tgg ttc ttc ctc ctg ctg gtg gca gct ccc aga tgg      48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa      96
Val Leu Ser Gln Val Gln Leu Val Gln Ser Gly Ala Glu Val Lys Lys
            20                  25                  30

cct gga gcc agc gtg aag gtg tcc tgc aag acc agc ggc tac acc ttc      144
Pro Gly Ala Ser Val Lys Val Ser Cys Lys Thr Ser Gly Tyr Thr Phe
        35                  40                  45

ccc aga tac gac gtg tcc tgg atc aag cag cgg cca gga cag gct ctg      192
Pro Arg Tyr Asp Val Ser Trp Ile Lys Gln Arg Pro Gly Gln Ala Leu
    50                  55                  60

gaa tgg atc ggc gtg atc cac cct ggc agc ggc ggc acc ggc tac aac      240
Glu Trp Ile Gly Val Ile His Pro Gly Ser Gly Gly Thr Gly Tyr Asn
65                  70                  75                  80

gag aag ttc aag gcc aag gct acc ctg gcc gtg gac acc agc acc agc      288
Glu Lys Phe Lys Ala Lys Ala Thr Leu Ala Val Asp Thr Ser Thr Ser
                85                  90                  95

acc gcc tac atg gaa ctg agc agc ctg cgg agc gag gac acc gcc gtg      336
Thr Ala Tyr Met Glu Leu Ser Ser Leu Arg Ser Glu Asp Thr Ala Val
            100                 105                 110

tac tac tgc gcc aga cgg ggc ctg aac agc ggc tac tgg ttc ttc gac      384
Tyr Tyr Cys Ala Arg Arg Gly Leu Asn Ser Gly Tyr Trp Phe Phe Asp
        115                 120                 125

ttc tgg ggc cct ggc acc ctg gtg acc gtc agc tca gcc agc acc aag 432
Phe Trp Gly Pro Gly Thr Leu Val Thr Val Ser Ser Ala Ser Thr Lys
    130                 135                 140

ggc cct tcc gtg ttc cct ctg gcc cct tgt agc cgt tcc acc agc gag 480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct 528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc 576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg 624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac 672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt 720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga 768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc 816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag 864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac 912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt 960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag 1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag 1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac 1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt 1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg 1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg 1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac 1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac 1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct 1392
Glu Ala Leu His Asn His Tyr Thr Gln Lys Ser Leu Ser Leu Ser Pro
    450                 455                 460

gga aag 1398
Gly Lys
465

<210> 20
<211> 466
<212> PRT
<213> Artificial Sequence

<220>
<223> Synthetic Construct

<400> 20

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Thr Ser Gly Tyr Thr Phe
        35                  40                  45

Pro Arg Tyr Asp Val Ser Trp Ile Lys Gln Arg Pro Gly Gln Ala Leu
    50                  55                  60

Glu Trp Ile Gly Val Ile His Pro Gly Ser Gly Gly Thr Gly Tyr Asn
65                  70                  75                  80

Glu Lys Phe Lys Ala Lys Ala Thr Leu Ala Val Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Arg Gly Leu Asn Ser Gly Tyr Trp Phe Phe Asp
        115                 120                 125

Phe Trp Gly Pro Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  21
<211>  1398
<212>  DNA
<213>  Artificial Sequence

<220>
<223>  Nucleotide coding h#24A3-H6

<220>
<221>  CDS
<222>  (1)..(1398)

<400>  21
atg aaa cac ctg tgg ttc ttc ctc ctg ctg gtg gca gct ccc aga tgg        48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa        96
Val Leu Ser Gln Val Gln Leu Val Gln Ser Gly Ala Glu Val Lys Lys
            20                  25                  30

cct gga gcc agc gtg aag gtg tcc tgc aag acc agc ggc tac acc ttc       144
Pro Gly Ala Ser Val Lys Val Ser Cys Lys Thr Ser Gly Tyr Thr Phe
        35                  40                  45

ccc aga tac gac gtg tcc tgg atc aag cag cgg cca ggc cag gcc ctg       192
Pro Arg Tyr Asp Val Ser Trp Ile Lys Gln Arg Pro Gly Gln Ala Leu
    50                  55                  60

gaa tgg atc ggc gtg atc cac cct ggc agc ggc ggc acc ggc tac aac       240
Glu Trp Ile Gly Val Ile His Pro Gly Ser Gly Gly Thr Gly Tyr Asn
65                  70                  75                  80

gag aag ttc aag gcc aag gct acc ctg gcc gtg gac acc agc acc agc 288
Glu Lys Phe Lys Ala Lys Ala Thr Leu Ala Val Asp Thr Ser Thr Ser
                85                  90                  95

acc gcc ttc atg gaa ctg agc agc ctg cgg agc gag gac acc gcc gtg 336
Thr Ala Phe Met Glu Leu Ser Ser Leu Arg Ser Glu Asp Thr Ala Val
            100                 105                 110

tac tac tgc gcc aga cgg ggc ctg aac agc ggc tac tgg ttc ttc gac 384
Tyr Tyr Cys Ala Arg Arg Gly Leu Asn Ser Gly Tyr Trp Phe Phe Asp
        115                 120                 125

ttc tgg ggc cct ggc acc ctg gtg acc gtc agc tca gcc agc acc aag 432
Phe Trp Gly Pro Gly Thr Leu Val Thr Val Ser Ser Ala Ser Thr Lys
    130                 135                 140

ggc cct tcc gtg ttc cct ctg gcc cct tgt agc cgt tcc acc agc gag 480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct 528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc 576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg 624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac 672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt 720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga 768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc 816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag 864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac 912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt 960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag 1008
Val Val Ser Val Leu Thr Val Val His Gln Asp Trp Leu Asn Gly Lys

325                 330                 335

gag tac aag tgt aag gtg agc aac aag gga ctt cct gcc cct atc gag      1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac      1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt      1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg      1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg      1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac      1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac      1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct      1392
Glu Ala Leu His Asn His Tyr Thr Gln Lys Ser Leu Ser Leu Ser Pro
    450                 455                 460

gga aag                                                              1398
Gly Lys
465


<210>  22
<211>  466
<212>  PRT
<213>  Artificial Sequence

<220>
<223>  Synthetic Construct

<400>  22

Met Lys His Leu Trp Phe Phe Leu Leu Leu Val Ala Ala Pro Arg Trp
1               5                   10                  15


Val Leu Ser Gln Val Gln Leu Val Gln Ser Gly Ala Glu Val Lys Lys
            20                  25                  30


Pro Gly Ala Ser Val Lys Val Ser Cys Lys Thr Ser Gly Tyr Thr Phe
        35                  40                  45


Pro Arg Tyr Asp Val Ser Trp Ile Lys Gln Arg Pro Gly Gln Ala Leu
    50                  55                  60

Glu Trp Ile Gly Val Ile His Pro Gly Ser Gly Gly Thr Gly Tyr Asn
65                  70                  75                  80

Glu Lys Phe Lys Ala Lys Ala Thr Leu Ala Val Asp Thr Ser Thr Ser
                85                  90                  95

Thr Ala Phe Met Glu Leu Ser Ser Leu Arg Ser Glu Asp Thr Ala Val
            100                 105                 110

Tyr Tyr Cys Ala Arg Arg Gly Leu Asn Ser Gly Tyr Trp Phe Phe Asp
        115                 120                 125

Phe Trp Gly Pro Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg

305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  23
<211>  702
<212>  DNA
<213>  Artificial Sequence

<220>
<223>  Nucleotide coding h#24A3-L1

<220>
<221>  CDS
<222>  (1)..(702)

<400>  23
atg gtg ctg cag acc cag gtg ttc atc tcc ctg ctg ctg tgg atc tcc        48
Met Val Leu Gln Thr Gln Val Phe Ile Ser Leu Leu Leu Trp Ile Ser
1               5                   10                  15

ggc gcg tac ggc gac atc gtg atg acc cag agc ccc gac agc ctg gcc        96
Gly Ala Tyr Gly Asp Ile Val Met Thr Gln Ser Pro Asp Ser Leu Ala

20                  25                  30

gtg tct ctg ggc gag aga gcc acc atc aac tgc aag gcc agc cag aac          144
Val Ser Leu Gly Glu Arg Ala Thr Ile Asn Cys Lys Ala Ser Gln Asn
        35                  40                  45

gtg ggc agc aac gtg gac tgg tat cag cag aag ccc ggc cag ccc ccc          192
Val Gly Ser Asn Val Asp Trp Tyr Gln Gln Lys Pro Gly Gln Pro Pro
    50                  55                  60

aag ctg ctg atc tac gag agc acc aac cgg tac acc ggc gtg ccc gac          240
Lys Leu Leu Ile Tyr Glu Ser Thr Asn Arg Tyr Thr Gly Val Pro Asp
65                  70                  75                  80

aga ttc agc ggc agc ggc tcc ggc acc gac ttc acc ctg acc atc agc          288
Arg Phe Ser Gly Ser Gly Ser Gly Thr Asp Phe Thr Leu Thr Ile Ser
                85                  90                  95

agt ctg cag gcc gag gac gtg gcc gtg tac tac tgt atg cag agc aac          336
Ser Leu Gln Ala Glu Asp Val Ala Val Tyr Tyr Cys Met Gln Ser Asn
            100                 105                 110

ttc ttc cca ttc acc ttc ggc cag ggc acc aag gtg gaa atc aag cgt          384
Phe Phe Pro Phe Thr Phe Gly Gln Gly Thr Lys Val Glu Ile Lys Arg
        115                 120                 125

acg gtg gcc gcc ccc tcc gtg ttc atc ttc ccc ccc tcc gac gag cag          432
Thr Val Ala Ala Pro Ser Val Phe Ile Phe Pro Pro Ser Asp Glu Gln
    130                 135                 140

ctg aag tcc ggc acc gcc tcc gtg gtg tgc ctg ctg aat aac ttc tac          480
Leu Lys Ser Gly Thr Ala Ser Val Val Cys Leu Leu Asn Asn Phe Tyr
145                 150                 155                 160

ccc aga gag gcc aag gtg cag tgg aag gtg gac aac gcc ctg cag tcc          528
Pro Arg Glu Ala Lys Val Gln Trp Lys Val Asp Asn Ala Leu Gln Ser
                165                 170                 175

ggg aac tcc cag gag agc gtg acc gag cag gac agc aag gac agc acc          576
Gly Asn Ser Gln Glu Ser Val Thr Glu Gln Asp Ser Lys Asp Ser Thr
            180                 185                 190

tac agc ctg agc agc acc ctg acc ctg agc aaa gcc gac tac gag aag          624
Tyr Ser Leu Ser Ser Thr Leu Thr Leu Ser Lys Ala Asp Tyr Glu Lys
        195                 200                 205

cac aag gtg tac gcc tgc gag gtg acc cac cag ggc ctg agc tcc ccc          672
His Lys Val Tyr Ala Cys Glu Val Thr His Gln Gly Leu Ser Ser Pro
    210                 215                 220

gtc acc aag agc ttc aac agg ggg gag tgt                                  702
Val Thr Lys Ser Phe Asn Arg Gly Glu Cys
225                 230


<210>  24
<211>  234
<212>  PRT
<213>  Artificial Sequence

<220>
<223>  Synthetic Construct

<400>  24

Met Val Leu Gln Thr Gln Val Phe Ile Ser Leu Leu Leu Trp Ile Ser
1               5                   10                  15

Gly Ala Tyr Gly Asp Ile Val Met Thr Gln Ser Pro Asp Ser Leu Ala
            20                  25                  30

Val Ser Leu Gly Glu Arg Ala Thr Ile Asn Cys Lys Ala Ser Gln Asn
        35                  40                  45

Val Gly Ser Asn Val Asp Trp Tyr Gln Gln Lys Pro Gly Gln Pro Pro
    50                  55                  60

Lys Leu Leu Ile Tyr Glu Ser Thr Asn Arg Tyr Thr Gly Val Pro Asp
65                  70                  75                  80

Arg Phe Ser Gly Ser Gly Ser Gly Thr Asp Phe Thr Leu Thr Ile Ser
                85                  90                  95

Ser Leu Gln Ala Glu Asp Val Ala Val Tyr Tyr Cys Met Gln Ser Asn
            100                 105                 110

Phe Phe Pro Phe Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  25

<211>  702
<212>  DNA
<213>  Artificial Sequence

<220>
<223>  Nucleotide coding h#24A3-L2


<220>
<221>  CDS
<222>  (1)..(702)

<400>  25
atg gtg ctg cag acc cag gtg ttc atc tcc ctg ctg ctg tgg atc tcc        48
Met Val Leu Gln Thr Gln Val Phe Ile Ser Leu Leu Leu Trp Ile Ser
1               5                   10                  15

ggc gcg tac ggc gac atc gtg atg acc cag agc ccc gac agc ctg gcc        96
Gly Ala Tyr Gly Asp Ile Val Met Thr Gln Ser Pro Asp Ser Leu Ala
            20                  25                  30

gtg tct ctg ggc gag aga gcc acc atc aac tgc aag gcc agc cag aac       144
Val Ser Leu Gly Glu Arg Ala Thr Ile Asn Cys Lys Ala Ser Gln Asn
        35                  40                  45

gtg ggc agc aac gtg gac tgg tat cag cag aag ccc ggc cag agc ccc       192
Val Gly Ser Asn Val Asp Trp Tyr Gln Gln Lys Pro Gly Gln Ser Pro
    50                  55                  60

aag ctg ctg atc tac gag agc acc aac cgg tac acc ggc gtg ccc gac       240
Lys Leu Leu Ile Tyr Glu Ser Thr Asn Arg Tyr Thr Gly Val Pro Asp
65                  70                  75                  80

aga ttc agc ggc agc ggc tcc ggc acc gac ttc acc ttc acc atc agc       288
Arg Phe Ser Gly Ser Gly Ser Gly Thr Asp Phe Thr Phe Thr Ile Ser
                85                  90                  95

agt ctg cag gcc gag gac gtg gcc gtg tac tac tgt atg cag agc aac       336
Ser Leu Gln Ala Glu Asp Val Ala Val Tyr Tyr Cys Met Gln Ser Asn
            100                 105                 110

ttc ttc cca ttc acc ttc ggc cag ggc acc aag gtg gaa atc aag cgt       384
Phe Phe Pro Phe Thr Phe Gly Gln Gly Thr Lys Val Glu Ile Lys Arg
        115                 120                 125

acg gtg gcc gcc ccc tcc gtg ttc atc ttc ccc ccc tcc gac gag cag       432
Thr Val Ala Ala Pro Ser Val Phe Ile Phe Pro Pro Ser Asp Glu Gln
    130                 135                 140

ctg aag tcc ggc acc gcc tcc gtg gtg tgc ctg ctg aat aac ttc tac       480
Leu Lys Ser Gly Thr Ala Ser Val Val Cys Leu Leu Asn Asn Phe Tyr
145                 150                 155                 160

ccc aga gag gcc aag gtg cag tgg aag gtg gac aac gcc ctg cag tcc       528
Pro Arg Glu Ala Lys Val Gln Trp Lys Val Asp Asn Ala Leu Gln Ser
                165                 170                 175

ggg aac tcc cag gag agc gtg acc gag cag gac agc aag gac agc acc       576
Gly Asn Ser Gln Glu Ser Val Thr Glu Gln Asp Ser Lys Asp Ser Thr
            180                 185                 190

tac agc ctg agc agc acc ctg acc ctg agc aaa gcc gac tac gag aag       624
Tyr Ser Leu Ser Ser Thr Leu Thr Leu Ser Lys Ala Asp Tyr Glu Lys

195                 200                 205

cac aag gtg tac gcc tgc gag gtg acc cac cag ggc ctg agc tcc ccc        672
His Lys Val Tyr Ala Cys Glu Val Thr His Gln Gly Leu Ser Ser Pro
    210                 215                 220

gtc acc aag agc ttc aac agg ggg gag tgt                                702
Val Thr Lys Ser Phe Asn Arg Gly Glu Cys
225                 230


<210>  26
<211>  234
<212>  PRT
<213>  Artificial Sequence

<220>
<223>  Synthetic Construct

<400>  26

Met Val Leu Gln Thr Gln Val Phe Ile Ser Leu Leu Leu Trp Ile Ser
1               5                   10                  15


Gly Ala Tyr Gly Asp Ile Val Met Thr Gln Ser Pro Asp Ser Leu Ala
            20                  25                  30


Val Ser Leu Gly Glu Arg Ala Thr Ile Asn Cys Lys Ala Ser Gln Asn
        35                  40                  45


Val Gly Ser Asn Val Asp Trp Tyr Gln Gln Lys Pro Gly Gln Ser Pro
    50                  55                  60


Lys Leu Leu Ile Tyr Glu Ser Thr Asn Arg Tyr Thr Gly Val Pro Asp
65                  70                  75                  80


Arg Phe Ser Gly Ser Gly Ser Gly Thr Asp Phe Thr Phe Thr Ile Ser
                85                  90                  95


Ser Leu Gln Ala Glu Asp Val Ala Val Tyr Tyr Cys Met Gln Ser Asn
            100                 105                 110


Phe Phe Pro Phe Thr Phe Gly Gln Gly Thr Lys Val Glu Ile Lys Arg
        115                 120                 125


Thr Val Ala Ala Pro Ser Val Phe Ile Phe Pro Pro Ser Asp Glu Gln
    130                 135                 140


Leu Lys Ser Gly Thr Ala Ser Val Val Cys Leu Leu Asn Asn Phe Tyr
145                 150                 155                 160


Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  27
<211>  702
<212>  DNA
<213>  Artificial Sequence

<220>
<223>  Nucleotide coding h#24A3-L3

<220>
<221>  CDS
<222>  (1)..(702)

<400>  27
atg gtg ctg cag acc cag gtg ttc atc tcc ctg ctg ctg tgg atc tcc      48
Met Val Leu Gln Thr Gln Val Phe Ile Ser Leu Leu Leu Trp Ile Ser
1               5                   10                  15

ggc gcg tac ggc gac atc gtg atg acc cag agc ccc gac agc ctg gcc      96
Gly Ala Tyr Gly Asp Ile Val Met Thr Gln Ser Pro Asp Ser Leu Ala
            20                  25                  30

gtg tct ctg ggc gag aga gcc acc atc aac tgc aag gcc agc cag aac      144
Val Ser Leu Gly Glu Arg Ala Thr Ile Asn Cys Lys Ala Ser Gln Asn
        35                  40                  45

gtg ggc agc aac gtg gac tgg tat cag cag aag ccc ggc cag agc ccc      192
Val Gly Ser Asn Val Asp Trp Tyr Gln Gln Lys Pro Gly Gln Ser Pro
    50                  55                  60

aag ctg ctg atc tac gag agc acc aac cgg tac acc ggc gtg ccc gac      240
Lys Leu Leu Ile Tyr Glu Ser Thr Asn Arg Tyr Thr Gly Val Pro Asp
65                  70                  75                  80

aga ttc aca ggc agc ggc agc ggc acc gac ttc acc ttc acc atc agc      288
Arg Phe Thr Gly Ser Gly Ser Gly Thr Asp Phe Thr Phe Thr Ile Ser
                85                  90                  95

agt ctg cag gcc gag gac gtg gcc gtg tac tac tgt atg cag agc aac      336
Ser Leu Gln Ala Glu Asp Val Ala Val Tyr Tyr Cys Met Gln Ser Asn
            100                 105                 110

ttc ttc cca ttc acc ttc ggc cag ggc acc aag gtg gaa atc aag cgt      384
Phe Phe Pro Phe Thr Phe Gly Gln Gly Thr Lys Val Glu Ile Lys Arg
        115                 120                 125

acg gtg gcc gcc ccc tcc gtg ttc atc ttc ccc ccc tcc gac gag cag 432
Thr Val Ala Ala Pro Ser Val Phe Ile Phe Pro Pro Ser Asp Glu Gln
    130                 135                 140

ctg aag tcc ggc acc gcc tcc gtg gtg tgc ctg ctg aat aac ttc tac 480
Leu Lys Ser Gly Thr Ala Ser Val Val Cys Leu Leu Asn Asn Phe Tyr
145                 150                 155                 160

ccc aga gag gcc aag gtg cag tgg aag gtg gac aac gcc ctg cag tcc 528
Pro Arg Glu Ala Lys Val Gln Trp Lys Val Asp Asn Ala Leu Gln Ser
                165                 170                 175

ggg aac tcc cag gag agc gtg acc gag cag gac agc aag gac agc acc 576
Gly Asn Ser Gln Glu Ser Val Thr Glu Gln Asp Ser Lys Asp Ser Thr
            180                 185                 190

tac agc ctg agc agc acc ctg acc ctg agc aaa gcc gac tac gag aag 624
Tyr Ser Leu Ser Ser Thr Leu Thr Leu Ser Lys Ala Asp Tyr Glu Lys
        195                 200                 205

cac aag gtg tac gcc tgc gag gtg acc cac cag ggc ctg agc tcc ccc 672
His Lys Val Tyr Ala Cys Glu Val Thr His Gln Gly Leu Ser Ser Pro
    210                 215                 220

gtc acc aag agc ttc aac agg ggg gag tgt 702
Val Thr Lys Ser Phe Asn Arg Gly Glu Cys
225                 230

<210> 28
<211> 234
<212> PRT
<213> Artificial Sequence

<220>
<223> Synthetic Construct

<400> 28

Met Val Leu Gln Thr Gln Val Phe Ile Ser Leu Leu Leu Trp Ile Ser
1               5                   10                  15

Gly Ala Tyr Gly Asp Ile Val Met Thr Gln Ser Pro Asp Ser Leu Ala
            20                  25                  30

Val Ser Leu Gly Glu Arg Ala Thr Ile Asn Cys Lys Ala Ser Gln Asn
        35                  40                  45

Val Gly Ser Asn Val Asp Trp Tyr Gln Gln Lys Pro Gly Gln Ser Pro
    50                  55                  60

Lys Leu Leu Ile Tyr Glu Ser Thr Asn Arg Tyr Thr Gly Val Pro Asp
65                  70                  75                  80

Arg Phe Thr Gly Ser Gly Ser Gly Thr Asp Phe Thr Phe Thr Ile Ser
                85                  90                  95

Ser Leu Gln Ala Glu Asp Val Ala Val Tyr Tyr Cys Met Gln Ser Asn
                100                 105                 110

Phe Phe Pro Phe Thr Phe Gly Gln Gly Thr Lys Val Glu Ile Lys Arg
            115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
                180                 185                 190

Tyr Ser Leu Ser Ser Thr Leu Thr Leu Ser Lys Ala Asp Tyr Glu Lys
            195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  29
<211>  702
<212>  DNA
<213>  Artificial Sequence

<220>
<223>  Nucleotide coding h#24A3-L4

<220>
<221>  CDS
<222>  (1)..(702)

<400>  29
atg gtg ctg cag acc cag gtg ttc atc tcc ctg ctg ctg tgg atc tcc      48
Met Val Leu Gln Thr Gln Val Phe Ile Ser Leu Leu Leu Trp Ile Ser
1               5                   10                  15

ggc gcg tac ggc gac atc gtg atg acc cag agc ccc gac agc ctg gcc      96
Gly Ala Tyr Gly Asp Ile Val Met Thr Gln Ser Pro Asp Ser Leu Ala
            20                  25                  30

gtg tct ctg ggc gag aga gcc acc atc aac tgc aag gcc agc cag aac      144
Val Ser Leu Gly Glu Arg Ala Thr Ile Asn Cys Lys Ala Ser Gln Asn
        35                  40                  45

gtg ggc agc aac gtg gac tgg tat cag cag aaa acc ggc cag agc ccc 192
Val Gly Ser Asn Val Asp Trp Tyr Gln Gln Lys Thr Gly Gln Ser Pro
    50                  55                  60

aag ctg ctg atc tac gag agc acc aac cgg tac acc ggc gtg ccc gac 240
Lys Leu Leu Ile Tyr Glu Ser Thr Asn Arg Tyr Thr Gly Val Pro Asp
65                  70                  75                  80

aga ttc aca ggc agc ggc agc ggc acc gac ttc acc ttc acc atc agc 288
Arg Phe Thr Gly Ser Gly Ser Gly Thr Asp Phe Thr Phe Thr Ile Ser
                85                  90                  95

agt ctg cag gcc gag gac gtg gcc gtg tac tac tgt atg cag agc aac 336
Ser Leu Gln Ala Glu Asp Val Ala Val Tyr Tyr Cys Met Gln Ser Asn
            100                 105                 110

ttc ttc ccc ttc acc ttc ggc tcc ggc acc aag gtg gaa atc aag cgt 384
Phe Phe Pro Phe Thr Phe Gly Ser Gly Thr Lys Val Glu Ile Lys Arg
        115                 120                 125

acg gtg gcc gcc ccc tcc gtg ttc atc ttc ccc ccc tcc gac gag cag 432
Thr Val Ala Ala Pro Ser Val Phe Ile Phe Pro Pro Ser Asp Glu Gln
    130                 135                 140

ctg aag tcc ggc acc gcc tcc gtg gtg tgc ctg ctg aat aac ttc tac 480
Leu Lys Ser Gly Thr Ala Ser Val Val Cys Leu Leu Asn Asn Phe Tyr
145                 150                 155                 160

ccc aga gag gcc aag gtg cag tgg aag gtg gac aac gcc ctg cag tcc 528
Pro Arg Glu Ala Lys Val Gln Trp Lys Val Asp Asn Ala Leu Gln Ser
                165                 170                 175

ggg aac tcc cag gag agc gtg acc gag cag gac agc aag gac agc acc 576
Gly Asn Ser Gln Glu Ser Val Thr Glu Gln Asp Ser Lys Asp Ser Thr
            180                 185                 190

tac agc ctg agc agc acc ctg acc ctg agc aaa gcc gac tac gag aag 624
Tyr Ser Leu Ser Ser Thr Leu Thr Leu Ser Lys Ala Asp Tyr Glu Lys
        195                 200                 205

cac aag gtg tac gcc tgc gag gtg acc cac cag ggc ctg agc tcc ccc 672
His Lys Val Tyr Ala Cys Glu Val Thr His Gln Gly Leu Ser Ser Pro
    210                 215                 220

gtc acc aag agc ttc aac agg ggg gag tgt 702
Val Thr Lys Ser Phe Asn Arg Gly Glu Cys
225                 230

<210> 30
<211> 234
<212> PRT
<213> Artificial Sequence

<220>
<223> Synthetic Construct

<400> 30

Met Val Leu Gln Thr Gln Val Phe Ile Ser Leu Leu Leu Trp Ile Ser
1               5                   10                  15

Gly Ala Tyr Gly Asp Ile Val Met Thr Gln Ser Pro Asp Ser Leu Ala
                20                  25                  30

Val Ser Leu Gly Glu Arg Ala Thr Ile Asn Cys Lys Ala Ser Gln Asn
            35                  40                  45

Val Gly Ser Asn Val Asp Trp Tyr Gln Gln Lys Thr Gly Gln Ser Pro
    50                  55                  60

Lys Leu Leu Ile Tyr Glu Ser Thr Asn Arg Tyr Thr Gly Val Pro Asp
65                  70                  75                  80

Arg Phe Thr Gly Ser Gly Ser Gly Thr Asp Phe Thr Phe Thr Ile Ser
                85                  90                  95

Ser Leu Gln Ala Glu Asp Val Ala Val Tyr Tyr Cys Met Gln Ser Asn
            100                 105                 110

Phe Phe Pro Phe Thr Phe Gly Ser Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  31
<211>  5
<212>  PRT
<213>  Rattus norvegicus

<400>  31

Arg Tyr Asp Val Ser
1               5

<210>  32
<211>  17
<212>  PRT
<213>  Rattus norvegicus

<400>  32

Val Ile His Pro Gly Ser Gly Gly Thr Gly Tyr Asn Glu Lys Phe Lys
1               5                   10                  15

Ala

<210>  33
<211>  12
<212>  PRT
<213>  Rattus norvegicus

<400>  33

Arg Gly Leu Asn Ser Gly Tyr Trp Phe Phe Asp Phe
1               5                   10

<210>  34
<211>  11
<212>  PRT
<213>  Rattus norvegicus

<400>  34

Lys Ala Ser Gln Asn Val Gly Ser Asn Val Asp
1               5                   10

<210>  35
<211>  7
<212>  PRT
<213>  Rattus norvegicus

<400>  35

Glu Ser Thr Asn Arg Tyr Thr
1               5

<210>  36
<211>  9
<212>  PRT
<213>  Rattus norvegicus

<400>  36

Met Gln Ser Asn Phe Phe Pro Phe Thr
1               5

<210>  37
<211>  1398
<212>  DNA
<213>  Artificial Sequence

<220>
<223>  Nucleotide coding h#24A3-H2b


<220>
<221>  CDS
<222>  (1)..(1398)

<400>  37
atg aaa cac ctg tgg ttc ttc ctc ctg ctg gtg gca gct ccc aga tgg      48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa      96
Val Leu Ser Gln Val Gln Leu Val Gln Ser Gly Ala Glu Val Lys Lys
            20                  25                  30

cct gga gcc agc gtg aag gtg tcc tgc aag gcc agc ggc tac acc ttc      144
Pro Gly Ala Ser Val Lys Val Ser Cys Lys Ala Ser Gly Tyr Thr Phe
        35                  40                  45

ccc aga tac gac gtg tcc tgg gtg cgc cag gcc cca ggc cag gga ctg      192
Pro Arg Tyr Asp Val Ser Trp Val Arg Gln Ala Pro Gly Gln Gly Leu
    50                  55                  60

gaa tgg atg ggc gtg atc cac cct ggc agc ggc ggc acc ggc tac aac      240
Glu Trp Met Gly Val Ile His Pro Gly Ser Gly Gly Thr Gly Tyr Asn
65                  70                  75                  80

gag aag ttc aag gcc aga gtg acc atc acc gcc gac acc agc acc agc      288
Glu Lys Phe Lys Ala Arg Val Thr Ile Thr Ala Asp Thr Ser Thr Ser
                85                  90                  95

acc gcc tac atg gaa ctg agc agc ctg cgg agc gag gac acc gcc gtg      336
Thr Ala Tyr Met Glu Leu Ser Ser Leu Arg Ser Glu Asp Thr Ala Val
            100                 105                 110

tac tac tgc gcc aga cgg ggc ctg aac aag ggc tac tgg ttc ttc gac      384
Tyr Tyr Cys Ala Arg Arg Gly Leu Asn Lys Gly Tyr Trp Phe Phe Asp
        115                 120                 125

ttc tgg ggc cag ggc acc ctg gtg acc gtc agc tca gcc agc acc aag      432
Phe Trp Gly Gln Gly Thr Leu Val Thr Val Ser Ser Ala Ser Thr Lys
    130                 135                 140

ggc cct tcc gtg ttc cct ctg gcc cct tgt agc cgt tcc acc agc gag      480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct      528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc      576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg      624

Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac 672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt 720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga 768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc 816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag 864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac 912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt 960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag 1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag 1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac 1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt 1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg 1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg 1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac 1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac 1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct 1392
Glu Ala Leu His Asn His Tyr Thr Gln Lys Ser Leu Ser Leu Ser Pro
    450                 455                 460

gga aag 1398
Gly Lys
465

<210> 38
<211> 466
<212> PRT
<213> Artificial Sequence

<220>
<223> Synthetic Construct

<400> 38

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Pro Arg Tyr Asp Val Ser Trp Val Arg Gln Ala Pro Gly Gln Gly Leu
    50                  55                  60

Glu Trp Met Gly Val Ile His Pro Gly Ser Gly Gly Thr Gly Tyr Asn
65                  70                  75                  80

Glu Lys Phe Lys Ala Arg Val Thr Ile Thr Ala Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Arg Gly Leu Asn Lys Gly Tyr Trp Phe Phe Asp
        115                 120                 125

Phe Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
        180                 185                 190

Phe Pro Ala Val Leu Gln Ser Ser Gly Leu Tyr Ser Leu Ser Ser Val
    195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  39
<211>  1398
<212>  DNA
<213>  Artificial Sequence

<220>
<223>  Nucleotide coding h#24A3-H2c

<220>
<221>  CDS
<222>  (1)..(1398)

<400>  39
atg aaa cac ctg tgg ttc ttc ctc ctg ctg gtg gca gct ccc aga tgg        48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa        96
Val Leu Ser Gln Val Gln Leu Val Gln Ser Gly Ala Glu Val Lys Lys
            20                  25                  30

cct gga gcc agc gtg aag gtg tcc tgc aag gcc agc ggc tac acc ttc       144
Pro Gly Ala Ser Val Lys Val Ser Cys Lys Ala Ser Gly Tyr Thr Phe
        35                  40                  45

ccc aga tac gac gtg tcc tgg gtg cgc cag gcc cca ggc cag gga ctg       192
Pro Arg Tyr Asp Val Ser Trp Val Arg Gln Ala Pro Gly Gln Gly Leu
    50                  55                  60

gaa tgg atc ggc gtg atc cac cct ggc agc ggc ggc acc ggc tac aac       240
Glu Trp Ile Gly Val Ile His Pro Gly Ser Gly Gly Thr Gly Tyr Asn
65                  70                  75                  80

gag aag ttc aag gcc aga gtg acc atc acc gcc gac acc agc acc agc       288
Glu Lys Phe Lys Ala Arg Val Thr Ile Thr Ala Asp Thr Ser Thr Ser
                85                  90                  95

acc gcc tac atg gaa ctg agc agc ctg cgg agc gag gac acc gcc gtg       336
Thr Ala Tyr Met Glu Leu Ser Ser Leu Arg Ser Glu Asp Thr Ala Val
            100                 105                 110

tac tac tgc gcc aga cgg ggc ctg aac aag ggc tac tgg ttc ttc gac       384
Tyr Tyr Cys Ala Arg Arg Gly Leu Asn Lys Gly Tyr Trp Phe Phe Asp
        115                 120                 125

ttc tgg ggc cag ggc acc ctg gtg acc gtc agc tca gcc agc acc aag       432
Phe Trp Gly Gln Gly Thr Leu Val Thr Val Ser Ser Ala Ser Thr Lys
    130                 135                 140

ggc cct tcc gtg ttc cct ctg gcc cct tgt agc cgt tcc acc agc gag 480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct 528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc 576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg 624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac 672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt 720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga 768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc 816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag 864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac 912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt 960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag 1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag 1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac 1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt 1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg 1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg        1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac        1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac        1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct        1392
Glu Ala Leu His Asn His Tyr Thr Gln Lys Ser Leu Ser Leu Ser Pro
    450                 455                 460

gga aag                                                                1398
Gly Lys
465


<210>  40
<211>  466
<212>  PRT
<213>  Artificial Sequence

<220>
<223>  Synthetic Construct

<400>  40

Met Lys His Leu Trp Phe Phe Leu Leu Leu Val Ala Ala Pro Arg Trp
1               5                   10                  15


Val Leu Ser Gln Val Gln Leu Val Gln Ser Gly Ala Glu Val Lys Lys
            20                  25                  30


Pro Gly Ala Ser Val Lys Val Ser Cys Lys Ala Ser Gly Tyr Thr Phe
        35                  40                  45


Pro Arg Tyr Asp Val Ser Trp Val Arg Gln Ala Pro Gly Gln Gly Leu
    50                  55                  60


Glu Trp Ile Gly Val Ile His Pro Gly Ser Gly Gly Thr Gly Tyr Asn
65                  70                  75                  80


Glu Lys Phe Lys Ala Arg Val Thr Ile Thr Ala Asp Thr Ser Thr Ser
                85                  90                  95


Thr Ala Tyr Met Glu Leu Ser Ser Leu Arg Ser Glu Asp Thr Ala Val
            100                 105                 110


Tyr Tyr Cys Ala Arg Arg Gly Leu Asn Lys Gly Tyr Trp Phe Phe Asp
        115                 120                 125

Phe Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  41
<211>  1398
<212>  DNA
<213>  Artificial Sequence

<220>
<223>  Nucleotide coding h#24A3-H2d

<220>
<221>  CDS
<222>  (1)..(1398)

<400>  41
atg aaa cac ctg tgg ttc ttc ctc ctg ctg gtg gca gct ccc aga tgg      48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa      96
Val Leu Ser Gln Val Gln Leu Val Gln Ser Gly Ala Glu Val Lys Lys
            20                  25                  30

cct gga gcc agc gtg aag gtg tcc tgc aag gcc agc ggc tac acc ttc      144
Pro Gly Ala Ser Val Lys Val Ser Cys Lys Ala Ser Gly Tyr Thr Phe
        35                  40                  45

ccc aga tac gac gtg tcc tgg gtg cgc cag gcc cca ggc cag gga ctg      192
Pro Arg Tyr Asp Val Ser Trp Val Arg Gln Ala Pro Gly Gln Gly Leu
    50                  55                  60

gaa tgg atg ggc gtg atc cac cct ggc agc ggc ggc acc ggc tac aac      240
Glu Trp Met Gly Val Ile His Pro Gly Ser Gly Gly Thr Gly Tyr Asn
65                  70                  75                  80

gag aag ttc aag gcc aga gcc acc atc acc gcc gac acc agc acc agc      288
Glu Lys Phe Lys Ala Arg Ala Thr Ile Thr Ala Asp Thr Ser Thr Ser
                85                  90                  95

acc gcc tac atg gaa ctg agc agc ctg cgg agc gag gac acc gcc gtg 336
Thr Ala Tyr Met Glu Leu Ser Ser Leu Arg Ser Glu Asp Thr Ala Val
            100                 105                 110

tac tac tgc gcc aga cgg ggc ctg aac aag ggc tac tgg ttc ttc gac 384
Tyr Tyr Cys Ala Arg Arg Gly Leu Asn Lys Gly Tyr Trp Phe Phe Asp
        115                 120                 125

ttc tgg ggc cag ggc acc ctg gtg acc gtc agc tca gcc agc acc aag 432
Phe Trp Gly Gln Gly Thr Leu Val Thr Val Ser Ser Ala Ser Thr Lys
    130                 135                 140

ggc cct tcc gtg ttc cct ctg gcc cct tgt agc cgt tcc acc agc gag 480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct 528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc 576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg 624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac 672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt 720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga 768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc 816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag 864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac 912
Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt 960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag 1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag 1056
Glu Tyr Lys Cys Lys Val Ser Asn Lys Gly Leu Pro Ala Pro Ile Glu

340                 345                 350

aag acc atc tcc aag acc aag ggc caa cct cgt gag cct caa gtg tac       1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt       1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg       1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg       1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac       1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac       1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct       1392
Glu Ala Leu His Asn His Tyr Thr Gln Lys Ser Leu Ser Leu Ser Pro
    450                 455                 460

gga aag                                                               1398
Gly Lys
465

<210>  42
<211>  466
<212>  PRT
<213>  Artificial Sequence

<220>
<223>  Synthetic Construct

<400>  42

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Pro Arg Tyr Asp Val Ser Trp Val Arg Gln Ala Pro Gly Gln Gly Leu
    50                  55                  60

Glu Trp Met Gly Val Ile His Pro Gly Ser Gly Gly Thr Gly Tyr Asn
65                  70                  75                  80

Glu Lys Phe Lys Ala Arg Ala Thr Ile Thr Ala Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Arg Gly Leu Asn Lys Gly Tyr Trp Phe Phe Asp
        115                 120                 125

Phe Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys

325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  43
<211>  1398
<212>  DNA
<213>  Artificial Sequence

<220>
<223>  Nucleotide coding h#24A3-H2e

<220>
<221>  CDS
<222>  (1)..(1398)

<400>  43
atg aaa cac ctg tgg ttc ttc ctc ctg ctg gtg gca gct ccc aga tgg      48
Met Lys His Leu Trp Phe Phe Leu Leu Leu Val Ala Ala Pro Arg Trp
1               5                   10                  15

gtg ctg agc cag gtg cag ctg gtg cag tct ggc gcc gaa gtg aag aaa      96
Val Leu Ser Gln Val Gln Leu Val Gln Ser Gly Ala Glu Val Lys Lys
            20                  25                  30

cct gga gcc agc gtg aag gtg tcc tgc aag gcc agc ggc tac acc ttc      144
Pro Gly Ala Ser Val Lys Val Ser Cys Lys Ala Ser Gly Tyr Thr Phe

35                  40                  45

ccc aga tac gac gtg tcc tgg gtg cgc cag gcc cca ggc cag gga ctg        192
Pro Arg Tyr Asp Val Ser Trp Val Arg Gln Ala Pro Gly Gln Gly Leu
    50                  55                  60

gaa tgg atg ggc gtg atc cac cct ggc agc ggc ggc acc ggc tac aac        240
Glu Trp Met Gly Val Ile His Pro Gly Ser Gly Gly Thr Gly Tyr Asn
65                  70                  75                  80

gag aag ttc aag gcc aga gtg acc atc acc gtg gac acc agc acc agc        288
Glu Lys Phe Lys Ala Arg Val Thr Ile Thr Val Asp Thr Ser Thr Ser
                85                  90                  95

acc gcc tac atg gaa ctg agc agc ctg cgg agc gag gac acc gcc gtg        336
Thr Ala Tyr Met Glu Leu Ser Ser Leu Arg Ser Glu Asp Thr Ala Val
            100                 105                 110

tac tac tgc gcc aga cgg ggc ctg aac aag ggc tac tgg ttc ttc gac        384
Tyr Tyr Cys Ala Arg Arg Gly Leu Asn Lys Gly Tyr Trp Phe Phe Asp
        115                 120                 125

ttc tgg ggc cag ggc acc ctg gtg acc gtc agc tca gcc agc acc aag        432
Phe Trp Gly Gln Gly Thr Leu Val Thr Val Ser Ser Ala Ser Thr Lys
    130                 135                 140

ggc cct tcc gtg ttc cct ctg gcc cct tgt agc cgt tcc acc agc gag        480
Gly Pro Ser Val Phe Pro Leu Ala Pro Cys Ser Arg Ser Thr Ser Glu
145                 150                 155                 160

tcc acc gcc gcc ctt ggc tgt ctg gtg aag gac tac ttc cct gag cct        528
Ser Thr Ala Ala Leu Gly Cys Leu Val Lys Asp Tyr Phe Pro Glu Pro
                165                 170                 175

gtg acc gtg agc tgg aac tcc gga gcc ctt acc agc ggc gtg cac acc        576
Val Thr Val Ser Trp Asn Ser Gly Ala Leu Thr Ser Gly Val His Thr
            180                 185                 190

ttc cct gcc gtg ctg cag tcc agc ggc ctt tac tcc ctg agc tcc gtg        624
Phe Pro Ala Val Leu Gln Ser Ser Gly Leu Tyr Ser Leu Ser Ser Val
        195                 200                 205

gtg acc gtg cct agc tcc aac ttc ggc acc caa acc tac acc tgt aac        672
Val Thr Val Pro Ser Ser Asn Phe Gly Thr Gln Thr Tyr Thr Cys Asn
    210                 215                 220

gtg gac cac aag cct agc aac acc aag gtg gac aag acc gtg gag cgt        720
Val Asp His Lys Pro Ser Asn Thr Lys Val Asp Lys Thr Val Glu Arg
225                 230                 235                 240

aag tgt tgt gtg gag tgt cct cct tgt cct gcc cct cct gtg gcc gga        768
Lys Cys Cys Val Glu Cys Pro Pro Cys Pro Ala Pro Pro Val Ala Gly
                245                 250                 255

cct tcc gtg ttc ctt ttc cct cct aag cct aag gac acc ctg atg atc        816
Pro Ser Val Phe Leu Phe Pro Pro Lys Pro Lys Asp Thr Leu Met Ile
            260                 265                 270

agc cgt acc cct gag gtg acc tgt gtg gtg gtg gac gtg tcc cac gag        864
Ser Arg Thr Pro Glu Val Thr Cys Val Val Val Asp Val Ser His Glu
        275                 280                 285

gac cct gag gtg cag ttc aac tgg tac gtg gac ggc gtg gag gtg cac        912

Asp Pro Glu Val Gln Phe Asn Trp Tyr Val Asp Gly Val Glu Val His
    290                 295                 300

aac gcc aag acc aag cct cgt gag gag caa ttc aac agc acc ttc cgt      960
Asn Ala Lys Thr Lys Pro Arg Glu Glu Gln Phe Asn Ser Thr Phe Arg
305                 310                 315                 320

gtg gtg tcc gtg ctt acc gtg gtg cac caa gac tgg ctg aac ggc aag      1008
Val Val Ser Val Leu Thr Val Val His Gln Asp Trp Leu Asn Gly Lys
                325                 330                 335

gag tac aag tgt aag gtg agc aac aag gga ctt cct gcc cct atc gag      1056
Glu Tyr Lys Cys Lys Val Ser Asn Lys Gly Leu Pro Ala Pro Ile Glu
            340                 345                 350

aag acc atc tcc aag acc aag ggc caa cct cgt gag cct caa gtg tac      1104
Lys Thr Ile Ser Lys Thr Lys Gly Gln Pro Arg Glu Pro Gln Val Tyr
        355                 360                 365

acc ctt cct cct agc cgt gag gag atg acc aag aac caa gtg tcc ctt      1152
Thr Leu Pro Pro Ser Arg Glu Glu Met Thr Lys Asn Gln Val Ser Leu
    370                 375                 380

acc tgt ctg gtg aag ggc ttc tac cct agc gac atc gcc gtg gag tgg      1200
Thr Cys Leu Val Lys Gly Phe Tyr Pro Ser Asp Ile Ala Val Glu Trp
385                 390                 395                 400

gag tcc aac gga caa cct gag aac aac tac aag acc acc cct cct atg      1248
Glu Ser Asn Gly Gln Pro Glu Asn Asn Tyr Lys Thr Thr Pro Pro Met
                405                 410                 415

ctt gac agc gac ggc tcc ttc ttc ctg tac agc aag ctg acc gtg gac      1296
Leu Asp Ser Asp Gly Ser Phe Phe Leu Tyr Ser Lys Leu Thr Val Asp
            420                 425                 430

aag tcc cgt tgg caa caa ggc aac gtg ttc agc tgt tcc gtg atg cac      1344
Lys Ser Arg Trp Gln Gln Gly Asn Val Phe Ser Cys Ser Val Met His
        435                 440                 445

gag gcc ctg cac aac cac tac acc caa aag agc ctt tcc ctg agc cct      1392
Glu Ala Leu His Asn His Tyr Thr Gln Lys Ser Leu Ser Leu Ser Pro
    450                 455                 460

gga aag                                                              1398
Gly Lys
465


<210>  44
<211>  466
<212>  PRT
<213>  Artificial Sequence

<220>
<223>  Synthetic Construct

<400>  44

Met Lys His Leu Trp Phe Phe Leu Leu Leu Val Ala Ala Pro Arg Trp
1               5                   10                  15


Val Leu Ser Gln Val Gln Leu Val Gln Ser Gly Ala Glu Val Lys Lys

20                  25                  30

Pro Gly Ala Ser Val Lys Val Ser Cys Lys Ala Ser Gly Tyr Thr Phe
        35                  40                  45

Pro Arg Tyr Asp Val Ser Trp Val Arg Gln Ala Pro Gly Gln Gly Leu
    50                  55                  60

Glu Trp Met Gly Val Ile His Pro Gly Ser Gly Gly Thr Gly Tyr Asn
65                  70                  75                  80

Glu Lys Phe Lys Ala Arg Val Thr Ile Thr Val Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Arg Gly Leu Asn Lys Gly Tyr Trp Phe Phe Asp
        115                 120                 125

Phe Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  45
<211>  702
<212>  DNA
<213>  Artificial Sequence

<220>
<223>  Nucleotide coding h#24A3-L2b

<220>
<221>  CDS

<222>  (1)..(702)

<400>  45
atg gtg ctg cag acc cag gtg ttc atc tcc ctg ctg ctg tgg atc tcc      48
Met Val Leu Gln Thr Gln Val Phe Ile Ser Leu Leu Leu Trp Ile Ser
1               5                   10                  15

ggc gcg tac ggc gac atc gtg atg acc cag agc ccc gac agc ctg gcc      96
Gly Ala Tyr Gly Asp Ile Val Met Thr Gln Ser Pro Asp Ser Leu Ala
            20                  25                  30

gtg tct ctg ggc gag aga gcc acc atc aac tgc aag gcc agc cag aac      144
Val Ser Leu Gly Glu Arg Ala Thr Ile Asn Cys Lys Ala Ser Gln Asn
        35                  40                  45

gtg ggc agc aac gtg gac tgg tat cag cag aag ccc ggc cag agc ccc      192
Val Gly Ser Asn Val Asp Trp Tyr Gln Gln Lys Pro Gly Gln Ser Pro
    50                  55                  60

aag ctg ctg atc tac gag agc acc aac cgg tac acc ggc gtg ccc gac      240
Lys Leu Leu Ile Tyr Glu Ser Thr Asn Arg Tyr Thr Gly Val Pro Asp
65                  70                  75                  80

aga ttc agc ggc agc ggc tcc ggc acc gac ttc acc ctg acc atc agc      288
Arg Phe Ser Gly Ser Gly Ser Gly Thr Asp Phe Thr Leu Thr Ile Ser
                85                  90                  95

agt ctg cag gcc gag gac gtg gcc gtg tac tac tgt atg cag agc aac      336
Ser Leu Gln Ala Glu Asp Val Ala Val Tyr Tyr Cys Met Gln Ser Asn
            100                 105                 110

ttc ttc cca ttc acc ttc ggc cag ggc acc aag gtg gaa atc aag cgt      384
Phe Phe Pro Phe Thr Phe Gly Gln Gly Thr Lys Val Glu Ile Lys Arg
        115                 120                 125

acg gtg gcc gcc ccc tcc gtg ttc atc ttc ccc ccc tcc gac gag cag      432
Thr Val Ala Ala Pro Ser Val Phe Ile Phe Pro Pro Ser Asp Glu Gln
    130                 135                 140

ctg aag tcc ggc acc gcc tcc gtg gtg tgc ctg ctg aat aac ttc tac      480
Leu Lys Ser Gly Thr Ala Ser Val Val Cys Leu Leu Asn Asn Phe Tyr
145                 150                 155                 160

ccc aga gag gcc aag gtg cag tgg aag gtg gac aac gcc ctg cag tcc      528
Pro Arg Glu Ala Lys Val Gln Trp Lys Val Asp Asn Ala Leu Gln Ser
                165                 170                 175

ggg aac tcc cag gag agc gtg acc gag cag gac agc aag gac agc acc      576
Gly Asn Ser Gln Glu Ser Val Thr Glu Gln Asp Ser Lys Asp Ser Thr
            180                 185                 190

tac agc ctg agc agc acc ctg acc ctg agc aaa gcc gac tac gag aag      624
Tyr Ser Leu Ser Ser Thr Leu Thr Leu Ser Lys Ala Asp Tyr Glu Lys
        195                 200                 205

cac aag gtg tac gcc tgc gag gtg acc cac cag ggc ctg agc tcc ccc      672
His Lys Val Tyr Ala Cys Glu Val Thr His Gln Gly Leu Ser Ser Pro
    210                 215                 220

gtc acc aag agc ttc aac agg ggg gag tgt                              702
Val Thr Lys Ser Phe Asn Arg Gly Glu Cys
225                 230

<210>  46
<211>  234
<212>  PRT
<213>  Artificial Sequence

<220>
<223>  Synthetic Construct

<400>  46

Met Val Leu Gln Thr Gln Val Phe Ile Ser Leu Leu Leu Trp Ile Ser
1               5                   10                  15

Gly Ala Tyr Gly Asp Ile Val Met Thr Gln Ser Pro Asp Ser Leu Ala
            20                  25                  30

Val Ser Leu Gly Glu Arg Ala Thr Ile Asn Cys Lys Ala Ser Gln Asn
        35                  40                  45

Val Gly Ser Asn Val Asp Trp Tyr Gln Gln Lys Pro Gly Gln Ser Pro
    50                  55                  60

Lys Leu Leu Ile Tyr Glu Ser Thr Asn Arg Tyr Thr Gly Val Pro Asp
65                  70                  75                  80

Arg Phe Ser Gly Ser Gly Ser Gly Thr Asp Phe Thr Leu Thr Ile Ser
                85                  90                  95

Ser Leu Gln Ala Glu Asp Val Ala Val Tyr Tyr Cys Met Gln Ser Asn
            100                 105                 110

Phe Phe Pro Phe Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  47
<211>  702
<212>  DNA
<213>  Artificial Sequence

<220>
<223>  Nucleotide coding h#24A3-L3b

<220>
<221>  CDS
<222>  (1)..(702)

<400>  47
atg gtg ctg cag acc cag gtg ttc atc tcc ctg ctg ctg tgg atc tcc      48
Met Val Leu Gln Thr Gln Val Phe Ile Ser Leu Leu Leu Trp Ile Ser
1               5                   10                  15

ggc gcg tac ggc gac atc gtg atg acc cag agc ccc gac agc ctg gcc      96
Gly Ala Tyr Gly Asp Ile Val Met Thr Gln Ser Pro Asp Ser Leu Ala
            20                  25                  30

gtg tct ctg ggc gag aga gcc acc atc aac tgc aag gcc agc cag aac      144
Val Ser Leu Gly Glu Arg Ala Thr Ile Asn Cys Lys Ala Ser Gln Asn
        35                  40                  45

gtg ggc agc aac gtg gac tgg tat cag cag aag ccc ggc cag agc ccc      192
Val Gly Ser Asn Val Asp Trp Tyr Gln Gln Lys Pro Gly Gln Ser Pro
    50                  55                  60

aag ctg ctg atc tac gag agc acc aac cgg tac acc ggc gtg ccc gac      240
Lys Leu Leu Ile Tyr Glu Ser Thr Asn Arg Tyr Thr Gly Val Pro Asp
65                  70                  75                  80

aga ttc aca ggc agc ggc agc ggc acc gac ttc acc ctg acc atc agc      288
Arg Phe Thr Gly Ser Gly Ser Gly Thr Asp Phe Thr Leu Thr Ile Ser
                85                  90                  95

agt ctg cag gcc gag gac gtg gcc gtg tac tac tgt atg cag agc aac      336
Ser Leu Gln Ala Glu Asp Val Ala Val Tyr Tyr Cys Met Gln Ser Asn
            100                 105                 110

ttc ttc cca ttc acc ttc ggc cag ggc acc aag gtg gaa atc aag cgt      384
Phe Phe Pro Phe Thr Phe Gly Gln Gly Thr Lys Val Glu Ile Lys Arg
        115                 120                 125

acg gtg gcc gcc ccc tcc gtg ttc atc ttc ccc ccc tcc gac gag cag      432
Thr Val Ala Ala Pro Ser Val Phe Ile Phe Pro Pro Ser Asp Glu Gln
    130                 135                 140

ctg aag tcc ggc acc gcc tcc gtg gtg tgc ctg ctg aat aac ttc tac      480
Leu Lys Ser Gly Thr Ala Ser Val Val Cys Leu Leu Asn Asn Phe Tyr
145                 150                 155                 160

ccc aga gag gcc aag gtg cag tgg aag gtg gac aac gcc ctg cag tcc      528

Pro Arg Glu Ala Lys Val Gln Trp Lys Val Asp Asn Ala Leu Gln Ser
                165                 170                 175

ggg aac tcc cag gag agc gtg acc gag cag gac agc aag gac agc acc       576
Gly Asn Ser Gln Glu Ser Val Thr Glu Gln Asp Ser Lys Asp Ser Thr
            180                 185                 190

tac agc ctg agc agc acc ctg acc ctg agc aaa gcc gac tac gag aag       624
Tyr Ser Leu Ser Ser Thr Leu Thr Leu Ser Lys Ala Asp Tyr Glu Lys
        195                 200                 205

cac aag gtg tac gcc tgc gag gtg acc cac cag ggc ctg agc tcc ccc       672
His Lys Val Tyr Ala Cys Glu Val Thr His Gln Gly Leu Ser Ser Pro
    210                 215                 220

gtc acc aag agc ttc aac agg ggg gag tgt                               702
Val Thr Lys Ser Phe Asn Arg Gly Glu Cys
225                 230


<210>  48
<211>  234
<212>  PRT
<213>  Artificial Sequence

<220>
<223>  Synthetic Construct

<400>  48

Met Val Leu Gln Thr Gln Val Phe Ile Ser Leu Leu Leu Trp Ile Ser
1               5                   10                  15


Gly Ala Tyr Gly Asp Ile Val Met Thr Gln Ser Pro Asp Ser Leu Ala
            20                  25                  30


Val Ser Leu Gly Glu Arg Ala Thr Ile Asn Cys Lys Ala Ser Gln Asn
        35                  40                  45


Val Gly Ser Asn Val Asp Trp Tyr Gln Gln Lys Pro Gly Gln Ser Pro
    50                  55                  60


Lys Leu Leu Ile Tyr Glu Ser Thr Asn Arg Tyr Thr Gly Val Pro Asp
65                  70                  75                  80


Arg Phe Thr Gly Ser Gly Ser Gly Thr Asp Phe Thr Leu Thr Ile Ser
                85                  90                  95


Ser Leu Gln Ala Glu Asp Val Ala Val Tyr Tyr Cys Met Gln Ser Asn
            100                 105                 110


Phe Phe Pro Phe Thr Phe Gly Gln Gly Thr Lys Val Glu Ile Lys Arg
        115                 120                 125


Thr Val Ala Ala Pro Ser Val Phe Ile Phe Pro Pro Ser Asp Glu Gln

130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  49
<211>  12
<212>  PRT
<213>  Artificial Sequence

<220>
<223>  CDRH3 of #24A3

<400>  49

Arg Gly Leu Asn Lys Gly Tyr Trp Phe Phe Asp Phe
1               5                   10

## Description

### Technical Field

The present invention relates to a substance useful as a therapeutic and/or prophylactic agent for abnormal bone metabolism, and a method for the treatment and/or prophylaxis of abnormal bone metabolism.

### Background Art

Bone is known to be a dynamic organ which is continuously remodeled by repeated formation and resorption so as to change its own morphology and maintain blood calcium levels. Healthy bone maintains an equilibrium between bone formation by osteoblasts and bone resorption by osteoclasts, and bone mass is maintained constant. In contrast, when the equilibrium between bone formation and bone resorption is lost, abnormal bone metabolism such as osteoporosis occurs (see, for example, Non Patent Literature 1 and 2).

As factors which regulate bone metabolism, many systemic hormones and local cytokines have been reported, and these factors collaborate with one another to form and maintain bone (see, for example, Non Patent Literature 1 and 3). As a change in bone tissue due to aging, the occurrence of osteoporosis is widely known, but the mechanism of its occurrence encompasses various factors such as a decrease in secretion of sex hormones and abnormality in the receptors for the hormones; variation in cytokine expression locally in bone; expression of aging genes; and osteoclast or osteoblast differentiation failure or dysfunction, and thus it is difficult to consider it as a simple age-related physiological phenomenon. Primary osteoporosis is largely divided into postmenopausal osteoporosis due to a decrease in secretion of estrogen and senile osteoporosis due to aging, but advancement of basic research on the mechanisms of regulation of bone formation and bone resorption is essential to elucidate the mechanism of its occurrence and to develop a therapeutic agent therefor.

Osteoclasts are multinucleated cells derived from hematopoietic stem cells, and by releasing chloride ions and hydrogen ions on a bone surface to which osteoclasts adhere, osteoclasts acidify the gap between the bone surface and the osteoclasts and also secrete cathepsin K which is an acid protease or the like (see, for example, Non Patent Literature 4). This causes degradation of calcium phosphate, activation of acid proteases and degradation of bone matrix proteins, resulting in bone resorption.

Osteoclast precursor cells have been found to be differentiated into osteoclasts by stimulation with RANKL (receptor activator of NF-κB ligand) expressed on the cell membrane of osteoblasts/stromal cells present on the surface of bone (see, for example, Non Patent Literature 5 and 6). It has been revealed that: RANKL is a membrane protein produced by osteoblasts/stromal cells, its expression is regulated by a bone resorption factor, RANKL induces differentiation of osteoclast precursor cells into mature multinucleated osteoclasts, and the like (see, for example, Non Patent Literature 5 and 7). Further, knockout mice devoid of RANKL have been found to develop an osteopetrosis-like disease, and therefore, RANKL has been proved to be a physiological osteoclast differentiation-inducing factor (see, for example, Non Patent Literature 8).

As medicaments for treating bone metabolism diseases or shortening the duration of treatment, bisphosphonates, active vitamin D₃, calcitonin and derivatives thereof, hormones such as estradiol, SERMs (selective estrogen receptor modulators), ipriflavone, vitamin K₂ (menatetrenone), PTH, calcium preparations, and the like are used. However, these medicaments are not always satisfactory in terms of therapeutic outcome and the development of a medicament with a more potent therapeutic effect has been demanded.

The cell membranes of immune cells are covered with a dense coating of various glycans such as sialylated glycans which are recognized by various glycan-binding proteins. Sialic-acid-binding immunoglobulin-like lectins (hereinafter referred to as "Siglecs") are a family of type I membrane proteins which recognize sialylated glycans and bind thereto. Many Siglecs are expressed on the cell membranes of immune cells and recognize sialic acid similarly present on the cell membranes of immune cells and regulate cell interaction or cell function and are considered to be involved in immune responses (see, for example, Non Patent Literature 9). However, there are also a lot of Siglec molecules whose physiological functions have not been elucidated yet. Siglec-15 (Sialic-acid binding immunoglobulin-like lectin 15) is a molecule which has been newly reported to belong to the Siglecs (see, for example, Non Patent Literature 10) and is identical to a molecule called CD33L3 (CD33 molecule-like 3). This molecule is highly evolutionarily conserved from fish to humans and has been found to be strongly expressed in dendritic cells and/or macrophages of human spleen and lymph nodes. Further, as a result of a binding test using a sialic acid probe, it has also been found that human Siglec-15 binds to Neu5Acα2-6GalNAc and that mouse Siglec-15 binds further to Neu5Acα2-3Galβ1-4Glc, and the like (see, for example, Non Patent Literature 10). Until recently, the physiological role of Siglec-15 had not been revealed, however, it has been reported that the expression of Siglec-15 increases with the differentiation and maturation of osteoclasts, and the differentiation of osteoclasts is inhibited by decreasing the expression of Siglec-15 by RNA interference (see, for example, PTL 1). Further, the effect of an anti-Siglec-15 antibody on osteoclast differentiation has been revealed for the first time in PTL 2 (published on April 16, 2009) and PTL 3 (published on October 14, 2010). Further, also in PTL 4, an antibody which inhibits the differentiation of osteoclasts has been disclosed, however, a search for an antibody which has a more potent effect has been continued.

### Prior Art Documents

### Patent Literature

Patent Literature 1: WO 07/093042
Patent Literature 2: WO 09/48072
Patent Literature 3: WO 10/117011
Patent Literature 4: WO 11/041894

### Non Patent Literature

Non Patent Literature 1 : Endocrinological Review, (1992) 13, pp. 66-80
Non Patent Literature 2: Principles of Bone Biology, Academic Press, New York, (1996) pp. 87-102
Non Patent Literature 3: Endocrinological Review, (1996) 17, pp. 308-332
Non Patent Literature 4: American Journal of Physiology, (1991) 260, C1315-C1324
Non Patent Literature 5: Proceedings of the National Academy of Science of the United States of America, (1998) 95, pp. 3597-3602
Non Patent Literature 6: Cell, (1998) 93, pp. 165-176
Non Patent Literature 7: Journal of Bone and Mineral Research, (1998) 23, S222
Non Patent Literature 8: Nature, (1999) 397, pp. 315-323
Non Patent Literature 9: Nature Reviews Immunology, (2007) 7, pp. 255-266
Non Patent Literature 10: Glycobiology, (2007) 17, pp. 838-846

### Summary of Invention

### Technical Problem of the Invention

An object of the invention is to provide a gene which is specifically expressed in various forms of abnormal bone metabolism which are seen in osteoporosis, rheumatoid arthritis, cancer metastasis to bone or the like, a substance which inhibits the differentiation and maturation of osteoclasts and the activity thereof, and a therapeutic and/or prophylactic agent for abnormal bone metabolism.

### Means for solving the Problem

The present inventors studied to elucidate the mechanism of osteoclast differentiation, maturation and activation in order to find a substance having a therapeutic and/or prophylactic effect on abnormal bone metabolism. As a result, the present inventors found that the expression of the Siglec-15 gene increases with the differentiation and maturation of osteoclasts, and also found that the differentiation of osteoclasts is inhibited by an antibody which specifically binds to Siglec-15. Further, the present inventors humanized a rat anti-mouse Siglec-15 antibody that had been obtained, and thus completed the invention.

Specifically, the invention includes the following inventions.
(1) An antibody or an antigen binding fragment of the antibody, characterized in that:
   the heavy chain sequence contains a variable region having CDRH1, CDRH2, and CDRH3, and the CDRH1 comprises an amino acid sequence represented by SEQ ID NO: 31, the CDRH2 comprises an amino acid sequence represented by SEQ ID NO: 32, and the CDRH3 comprises either an amino acid sequence represented by SEQ ID NO: 33 or an amino acid sequence including substitution of one to three amino acids therein; and
   the light chain sequence contains a variable region having CDRL1, CDRL2, and CDRL3, and the CDRL1 comprises an amino acid sequence represented by SEQ ID NO: 34, the CDRL2 comprises an amino acid sequence represented by SEQ ID NO: 35, and the CDRL3 comprises an amino acid sequence represented by SEQ ID NO: 36.
(2) An antibody or an antigen binding fragment of the antibody, characterized in that:
   the heavy chain sequence contains a variable region having CDRH1, CDRH2, and CDRH3, and the CDRH1 comprises an amino acid sequence represented by SEQ ID NO: 31, the CDRH2 comprises an amino acid sequence represented by SEQ ID NO: 32, and the CDRH3 comprises an amino acid sequence represented by SEQ ID NO: 33 or an amino acid sequence represented by SEQ ID NO: 49; and
   the light chain sequence contains a variable region having CDRL1, CDRL2, and CDRL3, and the CDRL1 comprises an amino acid sequence represented by SEQ ID NO: 34, the CDRL2 comprises an amino acid sequence represented by SEQ ID NO: 35, and the CDRL3 comprises an amino acid sequence represented by SEQ ID NO: 36.
(3) The antibody or an antigen binding fragment of the antibody according to (1) or (2), characterized by comprising a heavy chain variable region sequence comprising amino acid residues 1 to 121 of an amino acid sequence represented by SEQ ID NO: 2 and a light chain variable region sequence comprising amino acid residues 1 to 109 of an amino acid sequence represented by SEQ ID NO: 4.
(4) The antigen binding fragment of the antibody according to (1) to (3), which is selected from the group consisting of Fab, F(ab')2, Fab' and Fv.
(5) The antibody according to (1) to (3), characterized by being an scFv.
(6) The antibody or an antigen binding fragment of the antibody according to any one of (1) to (4), characterized in that the antibody is a chimeric antibody.
(7) The antibody or an antigen binding fragment of the antibody according to (6), characterized by comprising a heavy chain sequence comprising amino acid residues 20 to 466 of an amino acid sequence represented by SEQ ID NO: 8 and a light chain sequence comprising amino acid residues 21 to 234 of an amino acid sequence represented by SEQ ID NO: 10.
(8) The antibody or an antigen binding fragment of the antibody according to (1), (2) or (4), characterized in that the antibody is humanized.
(9) The antibody according to (6) or (8), wherein the heavy chain has a constant region of a human immunoglobulin G2 heavy chain and the light chain has a constant region of a human immunoglobulin κ light chain.
(10) An antibody which inhibits osteoclast formation and/or osteoclastic bone resorption or an antigen binding fragment of the antibody, characterized by comprising:
   (a) a heavy chain variable region sequence selected from the group consisting of the following amino acid sequences:
      a1) an amino acid sequence comprising amino acid residues 20 to 140 of an amino acid sequence represented by SEQ ID NO: 12;
      a2) an amino acid sequence comprising amino acid residues 20 to 140 of an amino acid sequence represented by SEQ ID NO: 14;
      a3) an amino acid sequence comprising amino acid residues 20 to 140 of an amino acid sequence represented by SEQ ID NO: 16;
      a4) an amino acid sequence comprising amino acid residues 20 to 140 of an amino acid sequence represented by SEQ ID NO: 18;
      a5) an amino acid sequence comprising amino acid residues 20 to 140 of an amino acid sequence represented by SEQ ID NO: 20;
      a6) an amino acid sequence comprising amino acid residues 20 to 140 of an amino acid sequence represented by SEQ ID NO: 22;
      a7) an amino acid sequence comprising amino acid residues 20 to 140 of an amino acid sequence represented by SEQ ID NO: 38;
      a8) an amino acid sequence comprising amino acid residues 20 to 140 of an amino acid sequence represented by SEQ ID NO: 40;
      a9) an amino acid sequence comprising amino acid residues 20 to 140 of an amino acid sequence represented by SEQ ID NO: 42;
      a10) an amino acid sequence comprising amino acid residues 20 to 140 of an amino acid sequence represented by SEQ ID NO: 44;
      a11) an amino acid sequence having a homology of at least 95% with any one of the amino acid sequences selected from a1) to a10) ;
      a12) an amino acid sequence having a homology of at least 99% with any one of the amino acid sequences selected from a1) to a10); and
      a13) an amino acid sequence including substitution, deletion, or addition of one to several amino acid residues in any one of the amino acid sequences selected from a1) to a10); and
   (b) a light chain variable region sequence selected from the group consisting of the following amino acid sequences:
      b1) an amino acid sequence comprising amino acid residues 21 to 129 of an amino acid sequence represented by SEQ ID NO: 24;
      b2) an amino acid sequence comprising amino acid residues 21 to 129 of an amino acid sequence represented by SEQ ID NO: 26;
      b3) an amino acid sequence comprising amino acid residues 21 to 129 of an amino acid sequence represented by SEQ ID NO: 28;
      b4) an amino acid sequence comprising amino acid residues 21 to 129 of an amino acid sequence represented by SEQ ID NO: 30;
      b5) an amino acid sequence comprising amino acid residues 21 to 129 of an amino acid sequence represented by SEQ ID NO: 46;
      b6) an amino acid sequence comprising amino acid residues 21 to 129 of an amino acid sequence represented by SEQ ID NO: 48;
      b7) an amino acid sequence having a homology of at least 95% with any one of the amino acid sequences selected from b1) to b6);
      b8) an amino acid sequence having a homology of at least 99% with any one of the amino acid sequences selected from b1) to b6); and
      b9) an amino acid sequence including substitution, deletion, or addition of one to several amino acid residues in any one of the amino acid sequences selected from b1) to b6).
(11) The antibody or an antigen binding fragment of the antibody according to (10), characterized by comprising a heavy chain variable region sequence comprising amino acid residues 20 to 140 of an amino acid sequence represented by SEQ ID NO: 14 and a light chain variable region sequence comprising amino acid residues 21 to 129 of an amino acid sequence represented by SEQ ID NO: 24.
(12) The antibody or an antigen binding fragment of the antibody according to (10), characterized by comprising a heavy chain variable region sequence comprising amino acid residues 20 to 140 of an amino acid sequence represented by SEQ ID NO: 14 and a light chain variable region sequence comprising amino acid residues 21 to 129 of an amino acid sequence represented by SEQ ID NO: 26.
(13) The antibody or an antigen binding fragment of the antibody according to (10), characterized by comprising a heavy chain variable region sequence comprising amino acid residues 20 to 140 of an amino acid sequence represented by SEQ ID NO: 14 and a light chain variable region sequence comprising amino acid residues 21 to 129 of an amino acid sequence represented by SEQ ID NO: 28.
(14) The antibody or an antigen binding fragment of the antibody according to (10), characterized by comprising a heavy chain variable region sequence comprising amino acid residues 20 to 140 of an amino acid sequence represented by SEQ ID NO: 38 and a light chain variable region sequence comprising amino acid residues 21 to 129 of an amino acid sequence represented by SEQ ID NO: 48.
(15) The antibody or an antigen binding fragment of the antibody according to (10), characterized by comprising a heavy chain variable region sequence comprising amino acid residues 20 to 140 of an amino acid sequence represented by SEQ ID NO: 40 and a light chain variable region sequence comprising amino acid residues 21 to 129 of an amino acid sequence represented by SEQ ID NO: 46.
(16) The antibody or an antigen binding fragment of the antibody according to (10), characterized by comprising a heavy chain variable region sequence comprising amino acid residues 20 to 140 of an amino acid sequence represented by SEQ ID NO: 42 and a light chain variable region sequence comprising amino acid residues 21 to 129 of an amino acid sequence represented by SEQ ID NO: 24.
(17) The antibody or an antigen binding fragment of the antibody according to (10), characterized by comprising a heavy chain variable region sequence comprising amino acid residues 20 to 140 of an amino acid sequence represented by SEQ ID NO: 44 and a light chain variable region sequence comprising amino acid residues 21 to 129 of an amino acid sequence represented by SEQ ID NO: 24.
(18) The antibody or an antigen binding fragment of the antibody according to (10), characterized by comprising a heavy chain sequence comprising amino acid residues 20 to 466 of an amino acid sequence represented by SEQ ID NO: 14 and a light chain sequence comprising amino acid residues 21 to 234 of an amino acid sequence represented by SEQ ID NO: 24.
(19) The antibody or an antigen binding fragment of the antibody according to (10), characterized by comprising a heavy chain sequence comprising amino acid residues 20 to 466 of an amino acid sequence represented by SEQ ID NO: 14 and a light chain sequence comprising amino acid residues 21 to 234 of an amino acid sequence represented by SEQ ID NO: 26.
(20) The antibody or an antigen binding fragment of the antibody according to (10), characterized by comprising a heavy chain sequence comprising amino acid residues 20 to 466 of an amino acid sequence represented by SEQ ID NO: 14 and a light chain sequence comprising amino acid residues 21 to 234 of an amino acid sequence represented by SEQ ID NO: 28.
(21) The antibody or an antigen binding fragment of the antibody according to (10), characterized by comprising a heavy chain sequence comprising amino acid residues 20 to 466 of an amino acid sequence represented by SEQ ID NO: 38 and a light chain sequence comprising amino acid residues 21 to 234 of an amino acid sequence represented by SEQ ID NO: 48.
(22) The antibody or an antigen binding fragment of the antibody according to (10), characterized by comprising a heavy chain sequence comprising amino acid residues 20 to 466 of an amino acid sequence represented by SEQ ID NO: 40 and a light chain sequence comprising amino acid residues 21 to 234 of an amino acid sequence represented by SEQ ID NO: 46.
(23) The antibody or a functional fragment of the antibody according to (10), characterized by comprising a heavy chain sequence comprising amino acid residues 20 to 466 of an amino acid sequence represented by SEQ ID NO: 42 and a light chain sequence comprising amino acid residues 21 to 234 of an amino acid sequence represented by SEQ ID NO: 24.
(24) The antibody or an antigen binding fragment of the antibody according to (10), characterized by comprising a heavy chain sequence comprising amino acid residues 20 to 466 of an amino acid sequence represented by SEQ ID NO: 44 and a light chain sequence comprising amino acid residues 21 to 234 of an amino acid sequence represented by SEQ ID NO: 24.
(25) The antibody according to any one of (11) to (24), wherein the antibody comprises a heavy chain including a deletion of one to several amino acids from the carboxyl group-terminal end thereof.
(26) A pharmaceutical composition, characterized by comprising at least one of the antibodies or antigen binding fragments of the antibodies according to (1) to (25).
(27) The pharmaceutical composition according to (26), characterized by being a therapeutic and/or prophylactic agent for abnormal bone metabolism.
(28) A pharmaceutical composition for the treatment and/or prophylaxis of abnormal bone metabolism, characterized by comprising at least one of the antibodies or antigen binding fragments of the antibodies according to (1) to (25) and at least one selected from the group consisting of bisphosphonates, active vitamin D₃, calcitonin and derivatives thereof, hormones such as estradiol, SERMs (selective estrogen receptor modulators), ipriflavone, vitamin K₂ (menatetrenone), calcium preparations, PTH (parathyroid hormone), nonsteroidal anti-inflammatory agents, soluble TNF receptors, anti-TNF-α antibodies or antigen binding fragments of the antibodies, anti-PTHrP (parathyroid hormone-related protein) antibodies or antigen binding fragments of the antibodies, IL-1 receptor antagonists, anti-IL-6 receptor antibodies or antigen binding fragments of the antibodies, anti-RANKL antibodies or antigen binding fragments of the antibodies, and OCIF (osteoclastogenesis inhibitory factor).
(29) The pharmaceutical composition according to (27) or (28), wherein the abnormal bone metabolism is selected from the group consisting of osteoporosis, bone destruction accompanying rheumatoid arthritis, cancerous hypercalcemia, bone destruction accompanying multiple myeloma or cancer metastasis to bone, giant cell tumor, osteopenia, tooth loss due to periodontitis, osteolysis around a prosthetic joint, bone destruction in chronic osteomyelitis, bone Paget's disease, renal osteodystrophy, and osteogenesis imperfecta.
(30) The pharmaceutical composition according to (29), characterized in that the abnormal bone metabolism is osteoporosis, bone destruction accompanying rheumatoid arthritis, or bone destruction accompanying cancer metastasis to bone.
(31) The pharmaceutical composition according to (30), characterized in that the abnormal bone metabolism is osteoporosis.
(32) The pharmaceutical composition according to (31), characterized in that the osteoporosis is postmenopausal osteoporosis, senile osteoporosis, secondary osteoporosis due to the use of a therapeutic agent such as a steroid or an immunosuppressant, or osteoporosis accompanying rheumatoid arthritis.
(33) A method for the treatment and/or prophylaxis of abnormal bone metabolism, characterized by administering at least one of the antibodies or antigen binding fragments of the antibodies according to (1) to (25) or the pharmaceutical composition according to (27) or (28).
(34) A method for the treatment and/or prophylaxis of abnormal bone metabolism, characterized by simultaneously or successively administering at least one of the antibodies or antigen binding fragments of the antibodies according to (1) to (25) or the pharmaceutical composition according to (28) and at least one selected from the group consisting of bisphosphonates, active vitamin D₃, calcitonin and derivatives thereof, hormones such as estradiol, SERMs (selective estrogen receptor modulators), ipriflavone, vitamin K₂ (menatetrenone), calcium preparations, PTH (parathyroid hormone), nonsteroidal anti-inflammatory agents, soluble TNF receptors, anti-TNF-α antibodies or antigen binding fragments of the antibodies, anti-PTHrP (parathyroid hormone-related protein) antibodies or antigen binding fragments of the antibodies, IL-1 receptor antagonists, anti-IL-6 receptor antibodies or antigen binding fragments of the antibodies, anti-RANKL antibodies or antigen binding fragments of the antibodies, and OCIF (osteoclastogenesis inhibitory factor).
(35) The method for the treatment and/or prophylaxis according to (33) or (34), characterized in that the abnormal bone metabolism is osteoporosis, bone destruction accompanying rheumatoid arthritis, or bone destruction accompanying cancer metastasis to bone.
(36) The method for the treatment and/or prophylaxis according to (35), characterized in that the abnormal bone metabolism is osteoporosis.
(37) The method for the treatment and/or prophylaxis according to (36), characterized in that the osteoporosis is postmenopausal osteoporosis, senile osteoporosis, secondary osteoporosis due to the use of a therapeutic agent such as a steroid or an immunosuppressant, or osteoporosis accompanying rheumatoid arthritis.
(38) A polynucleotide encoding the antibody according to any one of (1) to (25).
(39) The polynucleotide according to (38), characterized by comprising a nucleotide sequence comprising nucleotides 1 to 363 of a nucleotide sequence represented by SEQ ID NO: 1 and a nucleotide sequence comprising nucleotides 1 to 327 of a nucleotide sequence represented by SEQ ID NO: 3.
(40) The polynucleotide according to (39), characterized by comprising a nucleotide sequence comprising nucleotides 58 to 1398 of a nucleotide sequence represented by SEQ ID NO: 7 and a nucleotide sequence comprising nucleotides 61 to 702 of a nucleotide sequence represented by SEQ ID NO: 9.
(41) The polynucleotide according to (38), characterized by comprising:
   (a) a polynucleotide selected from the group consisting of the following nucleotide sequences:
      a1) a nucleotide sequence comprising nucleotides 58 to 420 of a nucleotide sequence represented by SEQ ID NO: 11;
      a2) a nucleotide sequence comprising nucleotides 58 to 420 of a nucleotide sequence represented by SEQ ID NO: 13;
      a3) a nucleotide sequence comprising nucleotides 58 to 420 of a nucleotide sequence represented by SEQ ID NO: 15;
      a4) a nucleotide sequence comprising nucleotides 58 to 420 of a nucleotide sequence represented by SEQ ID NO: 17;
      a5) a nucleotide sequence comprising nucleotides 58 to 420 of a nucleotide sequence represented by SEQ ID NO: 19;
      a6) a nucleotide sequence comprising nucleotides 58 to 420 of a nucleotide sequence represented by SEQ ID NO: 21;
      a7) a nucleotide sequence comprising nucleotides 58 to 420 of a nucleotide sequence represented by SEQ ID NO: 37;
      a8) a nucleotide sequence comprising nucleotides 58 to 420 of a nucleotide sequence represented by SEQ ID NO: 39;
      a9) a nucleotide sequence comprising nucleotides 58 to 420 of a nucleotide sequence represented by SEQ ID NO: 41;
      a10) a nucleotide sequence comprising nucleotides 58 to 420 of a nucleotide sequence represented by SEQ ID NO: 43;
      a11) an amino acid sequence having a homology of at least 95% with any one of the nucleotide sequences selected from a1) to a10) ;
      a12) an amino acid sequence having a homology of at least 99% with any one of the nucleotide sequences selected from a1) to a10) ;
      a13) a nucleotide sequence of a polynucleotide which hybridizes to a polynucleotide comprising a nucleotide sequence complementary to any one of the nucleotide sequences selected from a1) to a10) under stringent conditions; and
      a14) a nucleotide sequence including substitution, deletion, or addition of one to several amino acid residues in any one of the nucleotide sequences selected from a1) to a10) ; and
   (b) a polynucleotide selected from the group consisting of the following nucleotide sequences:
      b1) a nucleotide sequence comprising nucleotides 61 to 387 of a nucleotide sequence represented by SEQ ID NO: 23;
      b2) a nucleotide sequence comprising nucleotides 61 to 387 of a nucleotide sequence represented by SEQ ID NO: 25;
      b3) a nucleotide sequence comprising nucleotides 61 to 387 of a nucleotide sequence represented by SEQ ID NO: 27;
      b4) a nucleotide sequence comprising nucleotides 61 to 387 of a nucleotide sequence represented by SEQ ID NO: 28;
      b5) a nucleotide sequence comprising nucleotides 61 to 387 of a nucleotide sequence represented by SEQ ID NO: 45;
      b6) a nucleotide sequence comprising nucleotides 61 to 387 of a nucleotide sequence represented by SEQ ID NO: 47;
      b7) an amino acid sequence having a homology of at least 95% with any one of the nucleotide sequences selected from b1) to b6) ;
      b8) an amino acid sequence having a homology of at least 99% with any one of the nucleotide sequences selected from b1) to b6) ;
      b9) a nucleotide sequence of a polynucleotide which hybridizes to a polynucleotide comprising a nucleotide sequence complementary to any one of the nucleotide sequences selected from b1) to b6) under stringent conditions; and
      b10) a nucleotide sequence including substitution, deletion, or addition of one to several nucleotides in any one of the nucleotide sequences selected from b1) to b6).
(42) The polynucleotide according to (41), characterized by comprising a polynucleotide comprising a nucleotide sequence comprising nucleotides 58 to 420 of a nucleotide sequence represented by SEQ ID NO: 13, and a polynucleotide comprising a nucleotide sequence comprising nucleotides 61 to 387 of a nucleotide sequence represented by SEQ ID NO: 23.
(43) The polynucleotide according to (41), characterized by comprising a polynucleotide comprising a nucleotide sequence comprising nucleotides 58 to 420 of a nucleotide sequence represented by SEQ ID NO: 13, and a polynucleotide comprising a nucleotide sequence comprising nucleotides 61 to 387 of a nucleotide sequence represented by SEQ ID NO: 25.
(44) The polynucleotide according to (41), characterized by comprising a polynucleotide comprising a nucleotide sequence comprising nucleotides 58 to 420 of a nucleotide sequence represented by SEQ ID NO: 13, and a polynucleotide comprising a nucleotide sequence comprising nucleotides 61 to 387 of a nucleotide sequence represented by SEQ ID NO: 27.
(45) The polynucleotide according to (41), characterized by comprising a polynucleotide comprising a nucleotide sequence comprising nucleotides 58 to 420 of a nucleotide sequence represented by SEQ ID NO: 37, and a polynucleotide comprising a nucleotide sequence comprising nucleotides 61 to 387 of a nucleotide sequence represented by SEQ ID NO: 47.
(46) The polynucleotide according to (41), characterized by comprising a polynucleotide comprising a nucleotide sequence comprising nucleotides 58 to 420 of a nucleotide sequence represented by SEQ ID NO: 39, and a polynucleotide comprising a nucleotide sequence comprising nucleotides 61 to 387 of a nucleotide sequence represented by SEQ ID NO: 45.
(47) The polynucleotide according to (41), characterized by comprising a polynucleotide comprising a nucleotide sequence comprising nucleotides 58 to 420 of a nucleotide sequence represented by SEQ ID NO: 41, and a polynucleotide comprising a nucleotide sequence comprising nucleotides 61 to 387 of a nucleotide sequence represented by SEQ ID NO: 23.
(48) The polynucleotide according to (41), characterized by comprising a polynucleotide comprising a nucleotide sequence comprising nucleotides 58 to 420 of a nucleotide sequence represented by SEQ ID NO: 43, and a polynucleotide comprising a nucleotide sequence comprising nucleotides 61 to 387 of a nucleotide sequence represented by SEQ ID NO: 23.
(49) The polynucleotide according to (41), characterized by comprising a polynucleotide comprising a nucleotide sequence comprising nucleotides 58 to 1398 of a nucleotide sequence represented by SEQ ID NO: 13, and a polynucleotide comprising a nucleotide sequence comprising nucleotides 61 to 702 of a nucleotide sequence represented by SEQ ID NO: 23.
(50) The polynucleotide according to (41), characterized by comprising a polynucleotide comprising a nucleotide sequence comprising nucleotides 58 to 1398 of a nucleotide sequence represented by SEQ ID NO: 13, and a polynucleotide comprising a nucleotide sequence comprising nucleotides 61 to 702 of a nucleotide sequence represented by SEQ ID NO: 25.
(51) The polynucleotide according to (41), characterized by comprising a polynucleotide comprising a nucleotide sequence comprising nucleotides 58 to 1398 of a nucleotide sequence represented by SEQ ID NO: 13, and a polynucleotide comprising a nucleotide sequence comprising nucleotides 61 to 702 of a nucleotide sequence represented by SEQ ID NO: 27.
(52) The polynucleotide according to (41), characterized by comprising a polynucleotide comprising a nucleotide sequence comprising nucleotides 58 to 1398 of a nucleotide sequence represented by SEQ ID NO: 37, and a polynucleotide comprising a nucleotide sequence comprising nucleotides 61 to 702 of a nucleotide sequence represented by SEQ ID NO: 47.
(53) The polynucleotide according to (41), characterized by comprising a polynucleotide comprising a nucleotide sequence comprising nucleotides 58 to 1398 of a nucleotide sequence represented by SEQ ID NO: 39, and a polynucleotide comprising a nucleotide sequence comprising nucleotides 61 to 702 of a nucleotide sequence represented by SEQ ID NO: 45.
(54) The polynucleotide according to (41), characterized by comprising a polynucleotide comprising a nucleotide sequence comprising nucleotides 58 to 1398 of a nucleotide sequence represented by SEQ ID NO: 41, and a polynucleotide comprising a nucleotide sequence comprising nucleotides 61 to 702 of a nucleotide sequence represented by SEQ ID NO: 23.
(55) The polynucleotide according to (41), characterized by comprising a polynucleotide comprising a nucleotide sequence comprising nucleotides 58 to 1398 of a nucleotide sequence represented by SEQ ID NO: 43, and a polynucleotide comprising a nucleotide sequence comprising nucleotides 61 to 702 of a nucleotide sequence represented by SEQ ID NO: 23.
(56) A vector, comprising any one of the polynucleotides according to (38) to (55).
(57) A transformed host cell, comprising any one of the polynucleotides according to (38) to (55).
(58) A transformed host cell, comprising the vector according to (56).
(59) A method of producing the antibody according to any one of (1) to (25), comprising culturing the host cell according to (57) or (58), and purifying the antibody from the resulting culture product.

### Effects of Invention

According to the invention, a therapeutic and/or prophylactic agent for abnormal bone metabolism whose mechanism of action is to inhibit the differentiation and maturation of osteoclasts and the bone resorption activity thereof can be obtained.

### Brief Description of Drawings

[Fig. 1] Fig. 1 shows a cloned nucleotide sequence of a rat #24A3 heavy chain variable region and an amino acid sequence thereof.
[Fig. 2] Fig. 2 shows a cloned nucleotide sequence of a rat #24A3 light chain variable region and an amino acid sequence thereof.
[Fig. 3] Fig. 3 shows a nucleotide sequence of a human chimeric #24A3 antibody heavy chain and an amino acid sequence thereof.
[Fig. 4] Fig. 4 shows a nucleotide sequence of a human chimeric #24A3 antibody light chain and an amino acid sequence thereof.
[Fig. 5] Fig. 5 shows a nucleotide sequence of h#24A3-H1 and an amino acid sequence thereof.
[Fig. 6] Fig. 6 shows a nucleotide sequence of h#24A3-H2 and an amino acid sequence thereof.
[Fig. 7] Fig. 7 shows a nucleotide sequence of h#24A3-H3 and an amino acid sequence thereof.
[Fig. 8] Fig. 8 shows a nucleotide sequence of h#24A3-H4 and an amino acid sequence thereof.
[Fig. 9] Fig. 9 shows a nucleotide sequence of h#24A3-H5 and an amino acid sequence thereof.
[Fig. 10] Fig. 10 shows a nucleotide sequence of h#24A3-H6 and an amino acid sequence thereof.
[Fig. 11] Fig. 11 shows a nucleotide sequence of h#24A3-L1 and an amino acid sequence thereof.
[Fig. 12] Fig. 12 shows a nucleotide sequence of h#24A3-L2 and an amino acid sequence thereof.
[Fig. 13] Fig. 13 shows a nucleotide sequence of h#24A3-L3 and an amino acid sequence thereof.
[Fig. 14] Fig. 14 shows a nucleotide sequence of h#24A3-L4 and an amino acid sequence thereof.
[Fig. 15] Fig. 15 shows amino acid sequences of the respective CDR sequences of a rat #24A3 antibody.
[Fig. 16] Fig. 16 shows a graph depicting the inhibition of differentiation of normal human osteoclasts by a human chimeric antibody of a rat anti-human Siglec-15 monoclonal antibody #24A3 (N=6).
[Fig. 17] Fig. 17 shows a nucleotide sequence of h#24A3-H2b and an amino acid sequence thereof.
[Fig. 18] Fig. 18 shows a nucleotide sequence of h#24A3-H2c and an amino acid sequence thereof.
[Fig. 19] Fig. 19 shows a nucleotide sequence of h#24A3-H2d and an amino acid sequence thereof.
[Fig. 20] Fig. 20 shows a nucleotide sequence of h#24A3-H2e and an amino acid sequence thereof.
[Fig. 21] Fig. 21 shows a nucleotide sequence of h#24A3-L2b and an amino acid sequence thereof.
[Fig. 22] Fig. 22 shows a nucleotide sequence of h#24A3-L3b and an amino acid sequence thereof.
[Fig. 23] Fig. 23 shows graphs depicting the inhibition of differentiation of normal human osteoclasts by a humanized antibody of a rat anti-human Siglec-15 monoclonal antibody #24A3 (N=6).
[Fig. 24] Fig. 24 is a thermogram obtained for determining the thermal stability of the h#24A3-H2d/L1 antibody.
[Fig. 25] Fig. 25 is a thermogram obtained for determining the thermal stability of the h#24A3-H2e/L1 antibody.
[Fig. 26] Fig. 26 is a thermogram obtained for determining the thermal stability of the h#24A3-H2b/L3b antibody.
[Fig. 27] Fig. 27 is a thermogram obtained for determining the thermal stability of the h#24A3-H2c/L2b antibody.

### Description of Embodiments

The term "gene" as used herein includes not only DNA, but also mRNA, cDNA, and cRNA.

The term "polynucleotide" as used herein is used with the same meaning as a "nucleic acid" and also includes DNA, RNA, probes, oligonucleotides, and primers.

The terms "polypeptide" and "protein" as used herein are used without distinction.

The term "RNA fraction" as used herein refers to a fraction containing RNA.

The term "cell" as used herein includes cells in an animal individual and cultured cells.

The term "Siglec-15" as used herein is used with the same meaning as "Siglec-15 protein".

The term "osteoclast formation" as used herein is used with the same meaning as "osteoclast differentiation" or "osteoclast maturation".

The term "antigen binding fragment of an antibody" as used herein is used with the same meaning as "functional fragment of an antibody" and refers to a partial fragment of an antibody having an activity of binding to an antigen and includes Fab, F(ab')2, Fv, scFv, diabodies, linear antibodies, polyspecific antibodies formed from antibody fragments, and the like. The term also encompasses Fab' which is a monovalent fragment in a variable region of an antibody obtained by treating F(ab')2 under reducing conditions. However, the term is not limited to these molecules as long as the fragment has a binding affinity for an antigen. Further, these antigen binding fragments include not only a fragment obtained by treating a full-length molecule of an antibody protein with an appropriate enzyme, but also a protein produced in an appropriate host cell using a genetically modified antibody gene.

The term "epitope" as used herein refers to a partial peptide or a partial tertiary structure of Siglec-15 to which a specific anti-Siglec-15 antibody binds. The above-mentioned epitope which is a partial peptide of Siglec-15 can be determined by methods well known to those skilled in the art such as an immunoassay. However, the following method can be employed, for example. Various partial structures of Siglec-15 are produced. In the production of the partial structures, a known oligopeptide synthesis technique can be used. For example, a series of polypeptides having appropriately reduced lengths obtained by sequentially shortening Siglec-15 from the C terminus or N terminus are produced using a genetic recombination technique known to those skilled in the art. Thereafter, the reactivity of an antibody against these polypeptides is examined and a recognition site is roughly determined. Then, peptides having shorter lengths are synthesized and the reactivity with these peptides is examined, whereby the epitope can be determined. Further, the epitope which is a partial tertiary structure of Siglec-15 to which a specific anti-Siglec-15 antibody binds can be determined by specifying the amino acid residues of Siglec-15 adjacent to the antibody by X-ray crystal structure analysis. If a second anti-Siglec-15 antibody binds to a partial peptide or a partial tertiary structure to which a first anti-Siglec-15 antibody binds, it can be determined that the first antibody and the second antibody share the same epitope. Further, by confirming that the second anti-Siglec-15 antibody competes with the first anti-Siglec-15 antibody for the binding to Siglec-15 (that is, the second antibody inhibits the binding between Siglec-15 and the first antibody), it can be determined that the first antibody and the second antibody share the same epitope even if the specific sequence or structure of the epitope has not been determined. Further, when the first antibody and the second antibody bind to the same epitope and also the first antibody has a special effect such as an antigen-neutralizing activity, the second antibody can be expected to have the same activity.

It is known that each heavy and light chain of an antibody molecule has three complementarity determining regions (CDRs). The complementarity determining region is also called the hypervariable domain, and is present in a variable region of each heavy and light chain of an antibody. It is a site which has particularly high variability in its primary structure, and there are three separate CDRs in the primary structure of each heavy and light polypeptide chain. In this specification, as the complementarity determining regions of an antibody, the complementarity determining regions of the heavy chain are represented by CDRH1, CDRH2, and CDRH3 from the amino-terminal end of the amino acid sequence of the heavy chain, and the complementarity determining regions of the light chain are represented by CDRL1, CDRL2, and CDRL3 from the amino-terminal end of the amino acid sequence of the light chain. These sites are proximate to one another in the tertiary structure and determine the specificity for an antigen to which the antibody binds.

The phrase "hybridization is performed under stringent conditions" as used herein refers to hybridization being performed under conditions under which identification can be achieved by performing hybridization at 68°C in a commercially available hybridization solution, ExpressHyb Hybridization Solution (manufactured by Clontech Laboratories, Inc.) or performing hybridization at 68°C in the presence of 0.7 to 1.0 M NaCl using a filter having DNA immobilized thereon, followed by performing washing at 68°C using 0.1 to 2 x SSC solution (1 x SSC solution is composed of 150 mM NaCl and 15 mM sodium citrate) or under conditions equivalent thereto.

The term "several" in the specification in the phrase "one to several" and "one or several" as used herein refers to 2 to 10, preferably 10 or fewer, 5 or 6 or fewer, or 2 or 3.

### 1. Siglec-15

The present inventors have found that the Siglec-15 gene is specifically expressed in giant cell tumors and have also found that the expression level of the Siglec-15 gene increases when a monocyte-derived cell line differentiates into osteoclasts.

Siglec-15 to be used in the invention is directly purified from monocytes or bone marrow cells of a human, non-human mammal (such as a guinea pig, rat, mouse, rabbit, pig, sheep, cattle, or monkey) or chicken and can then be used, or is prepared from a cell membrane fraction of the above-mentioned cells and can then be used. Further, Siglec-15 can be obtained by in vitro synthesis or production in a host cell through genetic engineering. Specifically, in such genetic engineering production, Siglec-15 cDNA is integrated into a vector capable of expressing Siglec-15 cDNA, and Siglec-15 is synthesized in a solution containing enzymes, substrates, and energy substances required for transcription and translation, or another prokaryotic or eukaryotic host cell is transformed to express Siglec-15, whereby the protein can be obtained.

The nucleotide sequence of human Siglec-15 cDNA has been registered in GenBank with an accession number of NM_213602. The nucleotide sequence of mouse Siglec-15 cDNA has been registered in GenBank with an accession number of XM_884636. Incidentally, Siglec-15 is sometimes called CD33 antigen-like 3, CD33 molecule-like 3, CD33-like 3, or CD33L3, and all of these represent the same molecule.

Siglec-15 cDNA can be obtained by, for example, a so-called PCR method in which a polymerase chain reaction (hereinafter referred to as "PCR") is performed using a cDNA library expressing Siglec-15 cDNA as a template and primers which specifically amplify Siglec-15 cDNA (Saiki, R. K., et al., Science, (1988) 239, 487-49).

Incidentally, a polynucleotide which hybridizes to a polynucleotide comprising a nucleotide sequence complementary to a nucleotide sequence encoding human or mouse Siglec-15 under stringent conditions and encodes a protein having a biological activity comparable to that of Siglec-15 is also regarded as Siglec-15 cDNA. Further, a polynucleotide which is a splicing variant transcribed from the human or mouse Siglec-15 locus or a polynucleotide which hybridizes to a sequence complementary thereto under stringent conditions and encodes a protein having a biological activity comparable to that of Siglec-15 is also regarded as Siglec-15 cDNA.

Further, a protein which comprises an amino acid sequence including substitution, deletion or addition of one or several amino acids in an amino acid sequence of human or mouse Siglec-15 or an amino acid sequence obtained by removing the signal sequence from this sequence and which has a biological activity comparable to that of Siglec-15 is also regarded as Siglec-15. Further, a protein which comprises an amino acid sequence encoded by a splicing variant transcribed from the human or mouse Siglec-15 locus or an amino acid sequence including substitution, deletion or addition of one or several amino acids therein and which has a biological activity comparable to that of Siglec-15 is also regarded as Siglec-15.

### 2. Detection of abnormal bone metabolism

An analysis of the expression level of the Siglec-15 gene in a group of test samples from various human bone tissues showed that the expression level of the gene significantly increases in giant cell tumor (GCT) which is a bone tumor with a large number of osteoclast-like multinucleated giant cells arising and is characterized by clinical findings of osteolytic bone destruction (Bullough et al., Atlas of Orthopedic Pathology 2nd edition, pp. 17.6-17.8, Lippincott Williams & Wilkins Publishers (1992)).

It was also found that the expression level of the Siglec-15 gene increases when a monocyte-derived cell line is differentiated into osteoclasts.

Accordingly, Siglec-15 is considered to be associated with human pathologies such as GCT in which bone resorption is increased. In other words, measurement of the expression level of the Siglec-15 gene and/or Siglec-15 in each cell and/or each tissue enables determination of the state of abnormal bone metabolism accompanied by overexpression of Siglec-15. The term "abnormal bone metabolism" as used herein refers to a disorder characterized by net bone loss, and specific examples thereof include, but are not limited to, osteoporosis (postmenopausal osteoporosis, senile osteoporosis, secondary osteoporosis due to the use of a therapeutic agent such as a steroid or an immunosuppressant, or osteoporosis accompanying rheumatoid arthritis), bone destruction accompanying rheumatoid arthritis, cancerous hypercalcemia, bone destruction accompanying multiple myeloma or cancer metastasis to bone, giant cell tumor, osteopenia, tooth loss due to periodontitis, osteolysis around a prosthetic joint, bone destruction in chronic osteomyelitis, bone Paget's disease, renal osteodystrophy, and osteogenesis imperfecta.

In the invention, the "test sample" to be used for examining the expression level of the Siglec-15 gene and/or Siglec-15 refers to a sample of tissue from bone marrow, bone, prostate, testis, penis, bladder, kidney, oral cavity, pharynx, lip, tongue, gingiva, nasopharynx, esophagus, stomach, small intestine, large intestine, colon, liver, gallbladder, pancreas, nose, lung, soft tissue, skin, breast, uterus, ovary, brain, thyroid, lymph node, muscle, fat tissue or the like, or blood, a body fluid, an excretion, or the like obtained from a test subject, a clinical specimen, etc., however, in the invention, blood or bone marrow is more preferred.

As regards RANKL, which is known to be associated with osteoclast differentiation, a knockout mouse has been produced, and the phenotype when the function of RANKL has been lost has been analyzed (Young-Yun Kong, et. al., Nature (1999) 397, pp. 315-323). By producing a knockout mouse devoid of Siglec-15 in the same manner as above, the phenotype when the function of Siglec-15 has been lost can be analyzed.

### 3. Production of anti-Siglec-15 antibody

The antibody of the invention, which is against Siglec-15, can be obtained by immunizing an animal with Siglec-15 or an arbitrary polypeptide selected from the amino acid sequence of Siglec-15, and collecting and purifying the antibody produced in vivo according to common procedures. The biological species of Siglec-15 to be used as an antigen is not limited to being human, and an animal can be immunized with Siglec-15 derived from an animal other than humans such as a mouse or a rat. In this case, by examining the cross-reactivity between an antibody binding to the obtained heterologous Siglec-15 and human Siglec-15, an antibody applicable to a human disease can be selected.

Further, a monoclonal antibody can be obtained by fusing antibody-producing cells which produce an antibody against Siglec-15 with myeloma cells to establish a hybridoma according to a known method (for example, Kohler and Milstein, Nature, (1975) 256, pp. 495-497; Kennet, R. ed., Monoclonal Antibodies, pp. 365-367, Plenum Press, N.Y. (1980)). A specific example of such a method is described in WO 09/48072 (published on April 16, 2009) and WO 10/117011 (published on October 14, 2010). However, the method of obtaining a monoclonal antibody corresponds to a field which has been well established, and is not limited to the above specific example.

Incidentally, Siglec-15 to be used as an antigen can be obtained by genetic engineering whereby a host cell is caused to produce the Siglec-15 gene. Specifically, a vector capable of expressing the Siglec-15 gene is produced, the resulting vector is introduced into a host cell to express the gene, and then the expressed Siglec-15 is purified.

Examples of the hybridoma strain thus established include hybridoma #24A3 described in this specification. Incidentally, in this specification, the antibody produced by the hybridoma #24A3 is referred to as the "#24A3 antibody" or simply "#24A3". Further, in this specification, antibodies other than the #24A3 antibody are also represented by their antibody names in the same manner. A partial fragment containing the heavy chain variable region of the #24A3 antibody has an amino acid sequence comprising amino acid residues 1 to 121 of SEQ ID NO: 2 in the Sequence Listing. Further, a partial fragment containing the light chain variable region sequence of the #24A3 antibody has an amino acid sequence comprising amino acid residues 1 to 109 of SEQ ID NO: 4 in the Sequence Listing.

The antibody of the invention includes not only the above-mentioned monoclonal antibody against Siglec-15 but also a recombinant antibody obtained by artificial modification for the purpose of decreasing heterologous antigenicity to humans or the like such as a chimeric antibody, a humanized antibody and a human antibody. These antibodies can be produced using known methods.

As the chimeric antibody, an antibody in which antibody variable and constant regions are derived from different species, for example, a chimeric antibody in which a mouse- or rat-derived antibody variable region is connected to a human-derived constant region can be exemplified (see Proc. Natl. Acad. Sci. USA, 81, 6851-6855, (1984)). As one example of the chimeric antibody derived from a rat anti-mouse antibody #24A3, an antibody comprising a heavy chain having an amino acid sequence comprising amino acid residues 20 to 466 of SEQ ID NO: 8 in the Sequence Listing and a light chain having an amino acid sequence comprising amino acid residues 21 to 234 of SEQ ID NO: 10 in the Sequence Listing can be exemplified.

As the humanized antibody, an antibody obtained by integrating only the complementarity determining regions (CDRs) into a human-derived antibody (see Nature (1986) 321, pp. 522-525), and an antibody obtained by grafting part of the amino acid residues of the framework as well as the CDR sequences to a human antibody by a CDR-grafting method (WO 90/07861) can be exemplified.

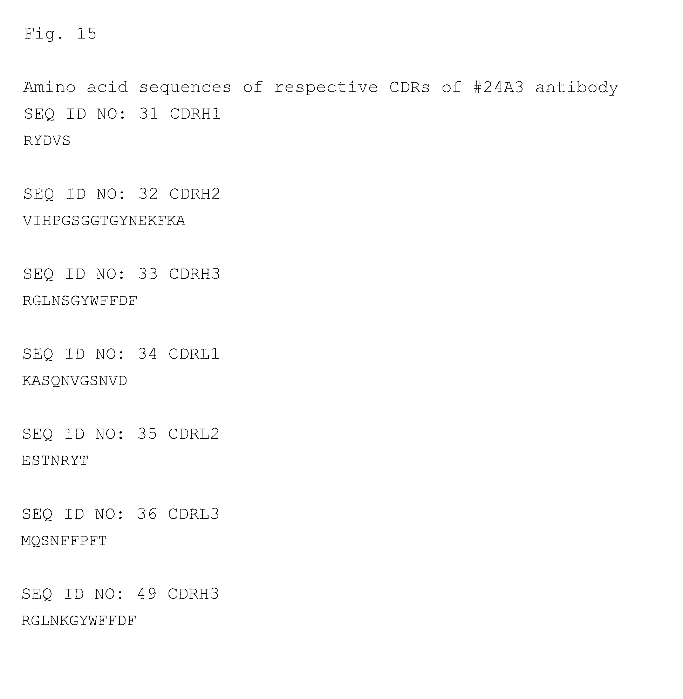

The humanized antibody derived from the #24A3 antibody is included in the antibody of the invention as long as the humanized antibody has all 6 types of CDR sequences of #24A3 and has the activity of inhibiting osteoclast formation. Incidentally, the heavy chain variable region of the #24A3 antibody has CDRH1 (RYDVS) comprising an amino acid sequence represented by SEQ ID NO: 31, CDRH2 (VIHPGSGGTGYNEKFKA) comprising an amino acid sequence represented by SEQ ID NO: 32, and CDRH3 (RGLNSGYWFFDF) comprising an amino acid sequence represented by SEQ ID NO: 33. Further, the light chain variable region of the #24A3 antibody has CDRL1 (KASQNVGSNVD) comprising an amino acid sequence represented by SEQ ID NO: 34, CDRL2 (ESTNRYT) comprising an amino acid sequence represented by SEQ ID NO: 35, and CDRL3 (MQSNFFPFT) comprising an amino acid sequence represented by SEQ ID NO: 36. The amino acid sequences of these CDRs are also shown in Fig. 15.

Further, a CDR-modified humanized antibody including substitution of one to three amino acid residues in each CDR with another amino acid residue is also included in the antibody of the invention as long as it has the activity of inhibiting osteoclast formation. As an example of the substitution of an amino acid in CDRH3, CDRH3 including substitution of one amino acid in the amino acid sequence represented by SEQ ID NO: 33 can be exemplified. Preferred is CDRH3 comprising an amino acid sequence (RGLNKGYWFFDF) represented by SEQ ID NO: 49. The amino acid sequence of this CDR is also shown in Fig. 15.

As an example of the humanized antibody of a rat antibody #24A3, an arbitrary combination of a heavy chain containing a heavy chain variable region comprising an amino acid sequence comprising amino acid residues 20 to 140 of SEQ ID NO: 12 in the Sequence Listing, amino acid residues 20 to 140 of SEQ ID NO: 14, amino acid residues 20 to 140 of SEQ ID NO: 16, amino acid residues 20 to 140 of SEQ ID NO: 18, amino acid residues 20 to 140 of SEQ ID NO: 20, amino acid residues 20 to 140 of SEQ ID NO: 22, amino acid residues 20 to 140 of SEQ ID NO: 38, amino acid residues 20 to 140 of SEQ ID NO: 40, amino acid residues 20 to 140 of SEQ ID NO: 42, or amino acid residues 20 to 140 of SEQ ID NO: 44 and a light chain containing a light chain variable region comprising an amino acid sequence comprising amino acid residues 21 to 129 of SEQ ID NO: 24, amino acid residues 21 to 129 of SEQ ID NO: 26, amino acid residues 21 to 129 of SEQ ID NO: 28, amino acid residues 21 to 129 of SEQ ID NO: 30, amino acid residues 21 to 129 of SEQ ID NO: 46, or amino acid residues 21 to 129 of SEQ ID NO: 48 can be exemplified.

As a preferred combination, an antibody comprising a heavy chain containing a heavy chain variable region comprising an amino acid sequence comprising amino acid residues 20 to 140 of SEQ ID NO: 12 and a light chain containing a light chain variable region comprising an amino acid sequence comprising amino acid residues 21 to 129 of SEQ ID NO: 24, an antibody comprising a heavy chain containing a heavy chain variable region comprising an amino acid sequence comprising amino acid residues 20 to 140 of SEQ ID NO: 12 and a light chain containing a light chain variable region comprising an amino acid sequence comprising amino acid residues 21 to 129 of SEQ ID NO: 26, an antibody comprising a heavy chain containing a heavy chain variable region comprising an amino acid sequence comprising amino acid residues 20 to 140 of SEQ ID NO: 12 and a light chain containing a light chain variable region comprising an amino acid sequence comprising amino acid residues 21 to 129 of SEQ ID NO: 28, an antibody comprising a heavy chain containing a heavy chain variable region comprising an amino acid sequence comprising amino acid residues 20 to 140 of SEQ ID NO: 14 and a light chain containing a light chain variable region comprising an amino acid sequence comprising amino acid residues 21 to 129 of SEQ ID NO: 24, an antibody comprising a heavy chain containing a heavy chain variable region comprising an amino acid sequence comprising amino acid residues 20 to 140 of SEQ ID NO: 14 and a light chain containing a light chain variable region comprising an amino acid sequence comprising amino acid residues 21 to 129 of SEQ ID NO: 26, an antibody comprising a heavy chain containing a heavy chain variable region comprising an amino acid sequence comprising amino acid residues 20 to 140 of SEQ ID NO: 14 and a light chain containing a light chain variable region comprising an amino acid sequence comprising amino acid residues 21 to 129 of SEQ ID NO: 28, an antibody comprising a heavy chain containing a heavy chain variable region comprising an amino acid sequence comprising amino acid residues 20 to 140 of SEQ ID NO: 16 and a light chain containing a light chain variable region comprising an amino acid sequence comprising amino acid residues 21 to 129 of SEQ ID NO: 24, an antibody comprising a heavy chain containing a heavy chain variable region comprising an amino acid sequence comprising amino acid residues 20 to 140 of SEQ ID NO: 16 and a light chain containing a light chain variable region comprising an amino acid sequence comprising amino acid residues 21 to 129 of SEQ ID NO: 26, an antibody comprising a heavy chain containing a heavy chain variable region comprising an amino acid sequence comprising amino acid residues 20 to 140 of SEQ ID NO: 16 and a light chain containing a light chain variable region comprising an amino acid sequence comprising amino acid residues 21 to 129 of SEQ ID NO: 28, an antibody comprising a heavy chain containing a heavy chain variable region comprising an amino acid sequence comprising amino acid residues 20 to 140 of SEQ ID NO: 18 and a light chain containing a light chain variable region comprising an amino acid sequence comprising amino acid residues 21 to 129 of SEQ ID NO: 24, an antibody comprising a heavy chain containing a heavy chain variable region comprising an amino acid sequence comprising amino acid residues 20 to 140 of SEQ ID NO: 18 and a light chain containing a light chain variable region comprising an amino acid sequence comprising amino acid residues 21 to 129 of SEQ ID NO: 26, an antibody comprising a heavy chain containing a heavy chain variable region comprising an amino acid sequence comprising amino acid residues 20 to 140 of SEQ ID NO: 18 and a light chain containing a light chain variable region comprising an amino acid sequence comprising amino acid residues 21 to 129 of SEQ ID NO: 28, an antibody comprising a heavy chain containing a heavy chain variable region comprising an amino acid sequence comprising amino acid residues 20 to 140 of SEQ ID NO: 20 and a light chain containing a light chain variable region comprising an amino acid sequence comprising amino acid residues 21 to 129 of SEQ ID NO: 24, an antibody comprising a heavy chain containing a heavy chain variable region comprising an amino acid sequence comprising amino acid residues 20 to 140 of SEQ ID NO: 20 and a light chain containing a light chain variable region comprising an amino acid sequence comprising amino acid residues 21 to 129 of SEQ ID NO: 26, an antibody comprising a heavy chain containing a heavy chain variable region comprising an amino acid sequence comprising amino acid residues 20 to 140 of SEQ ID NO: 20 and a light chain containing a light chain variable region comprising an amino acid sequence comprising amino acid residues 21 to 129 of SEQ ID NO: 28, or an antibody comprising a heavy chain containing a heavy chain variable region comprising an amino acid sequence comprising amino acid residues 20 to 140 of SEQ ID NO: 22 and a light chain containing a light chain variable region comprising an amino acid sequence comprising amino acid residues 21 to 129 of SEQ ID NO: 30 can be exemplified.

As a more preferred combination, an antibody comprising a heavy chain having an amino acid sequence comprising amino acid residues 20 to 466 of SEQ ID NO: 12 and a light chain having an amino acid sequence comprising amino acid residues 21 to 234 of SEQ ID NO: 24, an antibody comprising a heavy chain having an amino acid sequence comprising amino acid residues 20 to 466 of SEQ ID NO: 12 and a light chain having an amino acid sequence comprising amino acid residues 21 to 234 of SEQ ID NO: 26, an antibody comprising a heavy chain having an amino acid sequence comprising amino acid residues 20 to 466 of SEQ ID NO: 12 and a light chain having an amino acid sequence comprising amino acid residues 21 to 234 of SEQ ID NO: 28, an antibody comprising a heavy chain having an amino acid sequence comprising amino acid residues 20 to 466 of SEQ ID NO: 14 and a light chain having an amino acid sequence comprising amino acid residues 21 to 234 of SEQ ID NO: 24, an antibody comprising a heavy chain having an amino acid sequence comprising amino acid residues 20 to 466 of SEQ ID NO: 14 and a light chain having an amino acid sequence comprising amino acid residues 21 to 234 of SEQ ID NO: 26, an antibody comprising a heavy chain having an amino acid sequence comprising amino acid residues 20 to 466 of SEQ ID NO: 14 and a light chain having an amino acid sequence comprising amino acid residues 21 to 234 of SEQ ID NO: 28, an antibody comprising a heavy chain having an amino acid sequence comprising amino acid residues 20 to 466 of SEQ ID NO: 16 and a light chain having an amino acid sequence comprising amino acid residues 21 to 234 of SEQ ID NO: 24, an antibody comprising a heavy chain having an amino acid sequence comprising amino acid residues 20 to 466 of SEQ ID NO: 16 and a light chain having an amino acid sequence comprising amino acid residues 21 to 234 of SEQ ID NO: 26, an antibody comprising a heavy chain having an amino acid sequence comprising amino acid residues 20 to 466 of SEQ ID NO: 16 and a light chain having an amino acid sequence comprising amino acid residues 21 to 234 of SEQ ID NO: 28, an antibody comprising a heavy chain having an amino acid sequence comprising amino acid residues 20 to 466 of SEQ ID NO: 18 and a light chain having an amino acid sequence comprising amino acid residues 21 to 234 of SEQ ID NO: 24, an antibody comprising a heavy chain having an amino acid sequence comprising amino acid residues 20 to 466 of SEQ ID NO: 18 and a light chain having an amino acid sequence comprising amino acid residues 21 to 234 of SEQ ID NO: 26, an antibody comprising a heavy chain having an amino acid sequence comprising amino acid residues 20 to 466 of SEQ ID NO: 18 and a light chain having an amino acid sequence comprising amino acid residues 21 to 234 of SEQ ID NO: 28, an antibody comprising a heavy chain having an amino acid sequence comprising amino acid residues 20 to 466 of SEQ ID NO: 20 and a light chain having an amino acid sequence comprising amino acid residues 21 to 234 of SEQ ID NO: 24, an antibody comprising a heavy chain having an amino acid sequence comprising amino acid residues 20 to 466 of SEQ ID NO: 20 and a light chain having an amino acid sequence comprising amino acid residues 21 to 234 of SEQ ID NO: 26, an antibody comprising a heavy chain having an amino acid sequence comprising amino acid residues 20 to 466 of SEQ ID NO: 20 and a light chain having an amino acid sequence comprising amino acid residues 21 to 234 of SEQ ID NO: 28, or an antibody comprising a heavy chain having an amino acid sequence comprising amino acid residues 20 to 466 of SEQ ID NO: 22 and a light chain having an amino acid sequence comprising amino acid residues 21 to 234 of SEQ ID NO: 30 can be exemplified.

As a further more preferred combination, an antibody comprising a heavy chain containing a heavy chain variable region comprising an amino acid sequence comprising amino acid residues 20 to 140 of SEQ ID NO: 14 and a light chain containing a light chain variable region comprising an amino acid sequence comprising amino acid residues 21 to 129 of SEQ ID NO: 24, an antibody comprising a heavy chain containing a heavy chain variable region comprising an amino acid sequence comprising amino acid residues 20 to 140 of SEQ ID NO: 14 and a light chain containing a light chain variable region comprising an amino acid sequence comprising amino acid residues 21 to 129 of SEQ ID NO: 26, an antibody comprising a heavy chain containing a heavy chain variable region comprising an amino acid sequence comprising amino acid residues 20 to 140 of SEQ ID NO: 14 and a light chain containing a light chain variable region comprising an amino acid sequence comprising amino acid residues 21 to 129 of SEQ ID NO: 28, an antibody comprising a heavy chain containing a heavy chain variable region comprising an amino acid sequence comprising amino acid residues 20 to 140 of SEQ ID NO: 38 and a light chain containing a light chain variable region comprising an amino acid sequence comprising amino acid residues 21 to 129 of SEQ ID NO: 48, an antibody comprising a heavy chain containing a heavy chain variable region comprising an amino acid sequence comprising amino acid residues 20 to 140 of SEQ ID NO: 40 and a light chain containing a light chain variable region comprising an amino acid sequence comprising amino acid residues 21 to 129 of SEQ ID NO: 46, an antibody comprising a heavy chain containing a heavy chain variable region comprising an amino acid sequence comprising amino acid residues 20 to 140 of SEQ ID NO: 42 and a light chain containing a light chain variable region comprising an amino acid sequence comprising amino acid residues 21 to 129 of SEQ ID NO: 24, or an antibody comprising a heavy chain containing a heavy chain variable region comprising an amino acid sequence comprising amino acid residues 20 to 140 of SEQ ID NO: 44 and a light chain containing a light chain variable region comprising an amino acid sequence comprising amino acid residues 21 to 129 of SEQ ID NO: 24 can be exemplified.

As the most preferred combination, an antibody comprising a heavy chain having an amino acid sequence comprising amino acid residues 20 to 466 of SEQ ID NO: 14 and a light chain having an amino acid sequence comprising amino acid residues 21 to 234 of SEQ ID NO: 24, an antibody comprising a heavy chain having an amino acid sequence comprising amino acid residues 20 to 466 of SEQ ID NO: 14 and a light chain having an amino acid sequence comprising amino acid residues 21 to 234 of SEQ ID NO: 26, an antibody comprising a heavy chain having an amino acid sequence comprising amino acid residues 20 to 466 of SEQ ID NO: 14 and a light chain having an amino acid sequence comprising amino acid residues 21 to 234 of SEQ ID NO: 28, an antibody comprising a heavy chain having an amino acid sequence comprising amino acid residues 20 to 466 of SEQ ID NO: 38 and a light chain having an amino acid sequence comprising amino acid residues 21 to 234 of SEQ ID NO: 48, an antibody comprising a heavy chain having an amino acid sequence comprising amino acid residues 20 to 466 of SEQ ID NO: 40 and a light chain having an amino acid sequence comprising amino acid residues 21 to 234 of SEQ ID NO: 46, an antibody comprising a heavy chain having an amino acid sequence comprising amino acid residues 20 to 466 of SEQ ID NO: 42 and a light chain having an amino acid sequence comprising amino acid residues 21 to 234 of SEQ ID NO: 24, or an antibody comprising a heavy chain having an amino acid sequence comprising amino acid residues 20 to 466 of SEQ ID NO: 44 and a light chain having an amino acid sequence comprising amino acid residues 21 to 234 of SEQ ID NO: 24 can be exemplified.

Further, the antibody of the invention includes a human antibody. A human anti-Siglec-15 antibody refers to a human antibody encoded only by a gene sequence of an antibody derived from a human chromosome. The human anti-Siglec-15 antibody can be obtained by a method using a human antibody-producing mouse having a human chromosome fragment containing heavy and light chain genes of a human antibody (see Tomizuka, K. et al., Nature Genetics (1997) 16, pp. 133-143; Kuroiwa, Y. et al., Nucl. Acids Res. (1998) 26, pp. 3447-3448; Yoshida, H. et al., Animal Cell Technology: Basic and Applied Aspects vol. 10, pp. 69-73 (Kitagawa, Y., Matsuda, T. and Iijima, S. eds.), Kluwer Academic Publishers, 1999; Tomizuka, K. et al., Proc. Natl. Acad. Sci. USA (2000) 97, pp. 722-727, etc.).

Such a human antibody-producing mouse can be created specifically as follows. A genetically modified animal in which the endogenous immunoglobulin heavy and light chain gene loci have been disrupted, and instead, the human immunoglobulin heavy and light chain gene loci have been introduced via a yeast artificial chromosome (YAC) vector or the like is created by producing a knockout animal and a transgenic animal and mating these animals.

Further, according to a genetic engineering technique, by using cDNAs encoding such a heavy chain and a light chain of a human antibody, respectively, and preferably a vector containing the cDNAs, eukaryotic cells are transformed, and a transformant which produces a recombinant human monoclonal antibody is cultured, whereby the antibody can also be obtained from the culture supernatant. Here, as the host, for example, eukaryotic cells, preferably mammalian cells such as CHO cells, lymphocytes or myeloma cells can be used.

Further, a method of obtaining a phage display-derived human antibody screened from a human antibody library (see Wormstone, I. M. et al., Investigative Ophthalmology & Visual Science. (2002) 43 (7), pp. 2301-2308; Carmen, S. et al., Briefings in Functional Genomics and Proteomics (2002), 1 (2), pp. 189-203; Siriwardena, D. et al., Opthalmology (2002) 109 (3), pp. 427-431, etc.) is also known.

For example, a phage display method in which a variable region of a human antibody is expressed on the surface of a phage as a single-chain antibody (scFv), and a phage which binds to an antigen is selected (Nature Biotechnology (2005), 23, (9), pp. 1105-1116) can be used. By analyzing the gene of the phage selected based on the binding to an antigen, a DNA sequence encoding the variable region of a human antibody which binds to the antigen can be determined. If the DNA sequence of scFv which binds to an antigen is determined, a human antibody can be obtained by preparing an expression vector having the sequence and introducing the vector into an appropriate host to express it (WO 92/01047, WO 92/20791, WO 93/06213, WO 93/11236, WO 93/19172, WO 95/01438, WO 95/15388, Annu. Rev. Immunol (1994) 12, pp. 433-455, Nature Biotechnology (2005) 23 (9), pp. 1105-1116).

The above antibodies can be evaluated for the property of binding to an antigen by the method described in Example 3 or 10, etc., and a preferred antibody can be selected. As one example of another index by which the properties of antibodies are compared, the stability of antibodies can be exemplified. Differential scanning calorimetry (DSC) is a method capable of rapidly and accurately measuring a thermal denaturation midpoint temperature (Tm) which is a favorable index of relative structural stability of proteins. By measuring Tm values using DSC and comparing the values, a difference in thermal stability can be compared. It is known that the storage stability of antibodies shows some correlation with the thermal stability of antibodies (Lori Burton, et. al. , Pharmaceutical Development and Technology (2007) 12, pp. 265-273), and a preferred antibody can be selected by using thermal stability as an index. Examples of other indices for selecting antibodies are as follows: the yield in an appropriate host cell is high; and the aggregability in an aqueous solution is low. For example, an antibody which shows the highest yield does not always show high thermal stability, and therefore, it is necessary to select an antibody most suitable for administering to humans by making a comprehensive evaluation based on the above-mentioned indices.

Further, a method in which the full-length heavy and light chain sequences of an antibody are ligated using an appropriate linker, whereby a single-chain immunoglobulin is obtained is also known (Lee, H-S, et. al., Molecular Immunology (1999) 36, pp. 61-71; Shirrmann, T. et. al., mAbs (2010), 2, (1) pp. 1-4). By dimerizing such a single-chain immunoglobulin, the resulting dimer can have a structure and an activity similar to those of an antibody which is a tetramer itself. Further, the antibody of the invention may be an antibody which has a single heavy chain variable region and does not have a light chain sequence. Such an antibody is called a single domain antibody (sdAb) or a nanobody, and in fact, it is observed in camels and llamas and has been reported to have antigen binding affinity (Muyldemans S. et. al., Protein Eng. (1994) 7(9), 1129-35, Hamers-Casterman C. et. al., Nature (1993) 363 (6428) 446-8). The above-mentioned antibodies can also be regarded as a type of antigen binding fragment of the antibody according to the invention.

Further, by controlling glycosylation in which a glycan is bound to the antibody of the invention, it is possible to enhance antibody-dependent cytotoxic activity. As regards techniques for controlling the glycosylation of antibodies, WO 99/54342, WO 00/61739, WO 02/31140, etc. are known. However, the techniques are not limited thereto.

In cases where an antibody is produced by first isolating an antibody gene and then introducing the gene into an appropriate host, a combination of an appropriate host and an appropriate expression vector can be used. Specific examples of the antibody gene include a combination of a gene encoding a heavy chain sequence of an antibody described in this specification and a gene encoding a light chain sequence thereof. When a host cell is transformed, it is possible to insert the heavy chain sequence gene and the light chain sequence gene into the same expression vector, and also into different expression vectors separately. In cases where eukaryotic cells are used as the host, animal cells, plant cells, and eukaryotic microorganisms can be used. As the animal cells, (1) mammalian cells, for example, dihydrofolate reductase-deficient strains (Urlaub, G. and Chasin, L. A., Proc. Natl. Acad. Sci. USA (1980) 77, pp. 4126-4220) of simian COS cells (Gluzman, Y., Cell, (1981) 23, pp. 175-182, ATCC CRL-1650), murine fibroblasts NIH3T3 (ATCC No. CRL-1658), and Chinese hamster ovarian cells (CHO cells; ATCC: CCL-61) can be exemplified. Further, in cases where prokaryotic cells are used, for example, Escherichia coli and Bacillus subtilis can be exemplified. By introducing a target antibody gene into these cells through transformation, and culturing the thus transformed cells in vitro, the antibody can be obtained. In the above-mentioned culture method, the yield may sometimes vary depending on the sequence of the antibody, and therefore, it is possible to select one which is easily produced as a pharmaceutical by using the yield as an index among the antibodies having a comparable binding activity.

There is no limitation to the isotype of the antibody of the invention, and examples thereof include IgG (IgG1, IgG2, IgG3, IgG4), IgM, IgA (IgA1, IgA2), IgD, and IgE, and preferred examples thereof include IgG and IgM, and more preferred examples thereof include IgG1 and IgG2.

Further, the antibody of the invention may be an antigen binding fragment of the antibody having an antigen binding site of the antibody or a modified fragment thereof. The fragment of the antibody can be obtained by treating the antibody with a protease such as papain or pepsin, or modifying the antibody gene according to a genetic engineering technique and expressing the modified gene in suitable cultured cells. Among these antibody fragments, a fragment having all or some of the functions of the full-length molecule of the antibody can be called an antigen binding fragment of the antibody. As the functions of the antibody, generally an antigen binding activity, an activity of neutralizing the activity of an antigen, an activity of increasing the activity of an antigen, an antibody-dependent cytotoxic activity, a complement-dependent cytotoxic activity, and a complement-dependent cellular cytotoxic activity can be exemplified. The function of the antigen binding fragment of the antibody according to the invention is binding activity to Siglec-15, preferably the activity of inhibiting the formation of osteoclasts, more preferably the activity of inhibiting the process of cell fusion of osteoclasts.

Examples of the fragment of the antibody include Fab, F(ab')2, Fv, single-chain Fv (scFv) in which Fv molecules of the heavy chain and the light chain are ligated via an appropriate linker, a diabody (diabodies), a linear antibody, and a polyspecific antibody composed of the antibody fragment. Further, Fab' which is a monovalent fragment in a variable region of an antibody obtained by treating F(ab')2 under reducing conditions is also regarded as a fragment of the antibody.

Further, the antibody of the invention may be a polyspecific antibody with specificity for at least two different antigens. In general, such a molecule binds to two antigens (that is, a bispecific antibody), however, the "polyspecific antibody" as used herein includes an antibody having specificity for two or more (for example, three) antigens.

The polyspecific antibody of the invention may be a full-length antibody or a fragment of such an antibody (for example, a F(ab')2 bispecific antibody). The bispecific antibody can be produced by ligating the heavy and light chains (HL pairs) of two types of antibodies, or can also be produced by fusing hybridomas which produce different monoclonal antibodies to prepare bispecific antibody-producing fused cells (Millstein et al., Nature (1983) 305, pp. 537-539).

The antibody of the invention may be a single-chain antibody (also referred to as scFv). The single-chain antibody can be obtained by ligating the heavy chain variable region and the light chain variable region of an antibody via a polypeptide linker (Pluckthun, The Pharmacology of Monoclonal Antibodies, 113 (edited by Rosenburg and Moore, Springer Verlag, New York, pp. 269-315 (1994), Nature Biotechnology (2005), 23, pp. 1126-1136). Further, a BiscFv fragment produced by ligating two scFv molecules via a polypeptide linker can also be used as the bispecific antibody.

Methods of producing a single-chain antibody are known in this technical field (see, for example, US patent Nos. 4,946,778, 5,260,203, 5,091,513, 5,455,030, etc.). In this scFv, the heavy chain variable region and the light chain variable region are ligated via a linker which does not form a conjugate, preferably via a polypeptide linker (Huston, J. S. et al., Proc. Natl. Acad. Sci. USA (1988), 85, pp. 5879-5883). In the scFv, the heavy chain variable region and the light chain variable region may be derived from the same antibody or different antibodies. As the polypeptide linker to be used for ligating the variable regions, for example, a given single-chain peptide composed of 12 to 19 residues is used.

DNA encoding scFv can be obtained by performing amplification using a DNA encoding the entire amino acid sequence, or a desired partial amino acid sequence, selected from the heavy chain or the heavy chain variable region of the above-mentioned antibody and the light chain or the light chain variable region thereof as a template by a PCR method using a primer pair that defines both ends thereof, and further performing amplification by combining a DNA encoding a polypeptide linker portion and a primer pair that defines both ends thereof so as to ligate both of the ends to the heavy chain and the light chain, respectively.

Further, once DNA encoding scFv is produced, an expression vector containing the same and a host transformed by the expression vector can be obtained according to common procedures. Further, by using the resulting host, scFv can be obtained according to common procedures. An antibody fragment thereof can be produced in a host by obtaining a gene and expressing the gene in the same manner as described above.

The antibody of the invention may be multimerized to increase its affinity for an antigen. The antibody to be multimerized may be one type of antibody or a plurality of antibodies which recognize a plurality of epitopes of the same antigen. As a method of multimerization of the antibody, binding of the IgG CH3 domain to two scFv molecules, binding to streptavidin, introduction of a helix-turn-helix motif and the like can be exemplified.

The antibody of the invention may be a polyclonal antibody which is a mixture of plural types of anti-Siglec-15 antibodies having different amino acid sequences. As one example of the polyclonal antibody, a mixture of plural types of antibodies having different CDRs can be exemplified. As such a polyclonal antibody, a mixture of cells which produce different antibodies is cultured, and an antibody purified from the resulting culture can be used (see WO 2004/061104).

The antibody of the invention may be an antibody having a homology of 80% to 99% in comparison with the heavy chain and/or light chain of the above-mentioned antibody. By combining a sequence having a high homology with the above-mentioned heavy chain amino acid sequence with a sequence having a high homology with the above-mentioned light chain amino acid sequence, it is possible to select an antibody having antigen binding affinity, an activity of inhibiting osteoclast formation, and/or an activity of inhibiting osteoclastic bone resorption comparable to those of each of the above-mentioned antibodies. The homology is generally a homology of 80% or more, preferably a homology of 90% or more, more preferably a homology of 95% or more, and most preferably a homology of 99% or more. Further, it is also possible to select an antibody having all sorts of activities comparable to those of each of the above-mentioned antibodies by combining an amino acid sequence including substitution, deletion, and/or addition of one to several amino acid residues in the heavy and/or light chain amino acid sequence. The number of amino acid residues to be substituted, deleted, and/or added is generally 10 or less, preferably 5 to 6 or fewer, more preferably 2 to 3 or fewer, and most preferably 1.

Incidentally, it is known that any lysine residue at the carboxyl terminus of the heavy chain of an antibody produced by a mammalian cultured cell is deleted (Journal of Chromatography A, 705: 129-134 (1995)), and it is also known that the following two amino acid residues: glycine and lysine at the carboxyl terminus of such a heavy chain are deleted, and any proline residue located at the carboxyl terminus is newly amidated (Analytical Biochemistry, 360: 75-83 (2007)). However, such deletion and modification of the heavy chain sequence do not affect the antigen binding affinity and effector function (complement activation, antibody-dependent cytotoxic activity, etc.) of the antibody. Therefore, the invention also includes an antibody subjected to the above-mentioned modifications, and a deletion variant in which one or two amino acids at the carboxyl terminus of the heavy chain have been deleted, a deletion variant obtained by amidation of the same (for example, a heavy chain in which a proline residue at the carboxyl-terminal site has been amidated), and the like can be exemplified. However, the deletion variant in which a carboxyl-terminal residue of the heavy chain of the antibody according to the invention has been deleted is not limited to the above-mentioned variants as long as it has antigen binding affinity and effector function. The two heavy chains constituting the antibody of the invention may comprise a full-length heavy chain and any one heavy chain selected from the group consisting of the above-mentioned deletion variants, or may comprise any two heavy chains selected therefrom in combination. The relative amount of each deletion variant can be affected by the type of mammalian cultured cell used in production of the antibody according to the invention and the culture conditions, however, one example is where, as the main component of the antibody according to the invention, both of the two heavy chains include deletion of one amino acid residue at the carboxyl terminus.

The homology between two amino acid sequences can be determined using Blast algorithm version 2.2.2 (Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25: 3389-3402) with default parameters. The Blast algorithm can also be used through the Internet by accessing the site www.ncbi.nlm.nih.gov/blast. Incidentally, two types of percentage values of identity (or identities) and positivity (or positivities) are calculated by the Blast algorithm. The former is a value when amino acid residues match each other in two amino acid sequences for which a degree of homology is to be determined, and the latter is a value obtained by also considering amino acid residues having a similar chemical structure. In this specification, the value of the identity when amino acid residues match each other is used as the homology value.

An antibody bound to any of various types of molecules such as polyethylene glycol (PEG) as a modifying substance of the antibody can also be used.

Further, the antibody of the invention may be in the form of a conjugate formed between any of these antibodies and another medicinal agent (an immunoconjugate). Examples of such an antibody include one in which the antibody is conjugated to a radioactive material or a compound having a pharmacological effect (Nature Biotechnology (2005) 23, pp. 1137-1146).

The obtained antibody can be purified to homogeneity. The separation and purification of the antibody can be performed employing a conventional protein separation and purification method. For example, the antibody can be separated and purified by appropriately selecting and combining column chromatography, filter filtration, ultrafiltration, salt precipitation, dialysis, preparative polyacrylamide gel electrophoresis, isoelectric focusing electrophoresis, and the like (Strategies for Protein Purification and Characterization: A Laboratory Course Manual, Daniel R. Marshak et al. eds., Cold Spring Harbor Laboratory Press (1996); Antibodies: A Laboratory Manual. Ed Harlow and David Lane, Cold Spring Harbor Laboratory (1988)), but the method is not limited thereto.

Examples of such chromatography include affinity chromatography, ion exchange chromatography, hydrophobic chromatography, gel filtration chromatography, reverse phase chromatography, and adsorption chromatography.

Such chromatography can be performed employing liquid chromatography such as HPLC or FPLC.

As a column to be used in affinity chromatography, a Protein A column and a Protein G column can be exemplified.

For example, as a column using a Protein A column, Hyper D, POROS, Sepharose FF (Pharmacia) and the like can be exemplified.

Further, by using a carrier having an antigen immobilized thereon, the antibody can also be purified utilizing the binding activity of the antibody to the antigen.

### 4. Medicament containing anti-Siglec-15 antibody

From the anti-Siglec-15 antibodies obtained by the method described in the above section "3. Production of anti-Siglec-15 antibody", an antibody which neutralizes the biological activity of Siglec-15 can be obtained. Such an antibody which neutralizes the biological activity of Siglec-15 inhibits the biological activity of Siglec-15 in vivo, i.e., the differentiation and/or maturation of osteoclasts, and therefore can be used as a therapeutic and/or prophylactic agent for abnormal bone metabolism caused by abnormal differentiation and/or maturation of osteoclasts as a medicament. The abnormal bone metabolism may be any disorder characterized by net bone loss (osteopenia or osteolysis). In general, the treatment and/or prophylaxis by the anti-Siglec-15 antibody are/is applied to a case where inhibition of bone resorption is required. Examples of the abnormal bone metabolism which can be treated and/or prevented by the anti-Siglec-15 antibody include osteoporosis (postmenopausal osteoporosis, senile osteoporosis, secondary osteoporosis due to the use of a therapeutic agent such as a steroid or an immunosuppressant, or osteoporosis accompanying rheumatoid arthritis), bone destruction accompanying rheumatoid arthritis, cancerous hypercalcemia, bone destruction accompanying multiple myeloma or cancer metastasis to bone, giant cell tumor, osteopenia, tooth loss due to periodontitis, osteolysis around a prosthetic joint, bone destruction in chronic osteomyelitis, bone Paget's disease, renal osteodystrophy, and osteogenesis imperfecta, however, the abnormal bone metabolism is not limited thereto as long as it is a disease accompanied by net bone loss caused by osteoclasts. Examples of the anti-Siglec-15 antibody to be used as the above-mentioned medicament include a chimeric antibody and a humanized antibody produced from the #24A3 antibody by the method described in "3. Production of anti-Siglec-15 antibody". Further, a chimeric antibody, a humanized antibody, and a human antibody sharing the same epitope as the #24A3 antibody can also be used as a medicament. Whether a certain anti-Siglec-15 antibody shares the same epitope as the #24A3 antibody can be confirmed by observing whether or not these antibodies bind to the same specific partial peptide of Siglec-15. Further, it can also be determined that if a certain anti-Siglec-15 antibody competes with the #24A3 antibody for binding to Siglec-15, these antibodies share the same epitope.

The in vitro activity of the anti-Siglec-15 antibody of neutralizing the biological activity of Siglec-15 can be determined by, for example, the activity of inhibiting differentiation of the cells which overexpress Siglec-15 into osteoclasts. For example, the anti-Siglec-15 antibody is added to a mouse monocyte-derived cell line RAW 264.7 cells or RAW 264 cells at various concentrations, and the activity of inhibiting differentiation into osteoclasts by stimulation with RANKL or TNF-α can be determined. Further, the anti-Siglec-15 antibody is added to bone marrow-derived primary cultured cells at various concentrations, and the activity of inhibiting differentiation into osteoclasts by stimulation with RANKL, TNF-α, or active vitamin D₃ can be determined. Further, the anti-Siglec-15 antibody is added to normal human osteoclast precursor cells (Normal Human Natural Osteoclast Precursor Cells, available from Sanko Junyaku Co., Ltd., Cat. No. 2T-110) at various concentrations, and the activity of inhibiting differentiation into osteoclasts by stimulation with RANKL and M-CSF can be determined. Such an inhibitory effect on osteoclast differentiation can be determined by using the inhibition of tartrate-resistant acid phosphatase (TRAP) activity of osteoclasts as an index. Further, the inhibitory effect on osteoclast differentiation can also be determined by using the inhibition of formation of TRAP-positive multinucleated osteoclasts, i.e., the inhibition of cell fusion of osteoclasts as an index. Further, in an experiment utilizing a pit assay (Takada et al., Bone and Mineral, (1992) 17, 347-359) using femur- and/or tibia-derived cells, the in vitro activity of inhibiting bone resorption by osteoclasts can be determined by adding the anti-Siglec-15 antibody to femur- and/or tibia-derived cells at various concentrations, and observing pit formation on a dentine slice. As a system for determining the in vitro activity of inhibiting bone resorption by osteoclasts, it is also possible to use a plate coated with europium-conjugated human collagen. The in vivo therapeutic or prophylactic effect of the anti-Siglec-15 antibody on abnormal bone metabolism using an experimental animal can be confirmed by, for example, administering the anti-Siglec-15 antibody to an animal model of osteoporosis or a transgenic animal which overexpresses Siglec-15 and measuring any change in osteoclasts. As the animal model of osteoporosis, ovariectomized rats and ovariectomized monkeys can be exemplified. The inhibitory effect of the anti-Siglec-15 antibody on bone resorption activity can be determined by administering the anti-Siglec-15 antibody to such an experimental animal, and then measuring the bone mineral density or a bone metabolism marker. Examples of the bone metabolism marker include, but are not limited to, bone resorption markers such as urinary deoxypyridinoline, urinary N-telopeptide of type I collagen (NTX), urinary C-telopeptide of type I collagen (CTX), blood NTX, blood CTX, and blood tartrate-resistant acid phosphatase (TRAP5b), and bone formation markers such as blood bone alkaline phosphatase (BAP), blood osteocalcin (BGP), and procollagen type I C-peptide (P1NP).

The thus obtained antibody which neutralizes the biological activity of Siglec-15 is useful as a medicament, particularly as a pharmaceutical composition for the treatment or prophylaxis of abnormal bone metabolism such as osteoporosis, bone destruction accompanying rheumatoid arthritis, or bone destruction accompanying cancer metastasis to bone, or as an antibody for immunological diagnosis of such a disease.

In the treatment of rheumatoid arthritis (RA), a major problem is bone loss accompanying the occurrence of the disease. It has been reported that in this bone loss accompanying RA, osteoclasts play a primary role. The cytokines considered to be most important as the cause of osteoclast induction (differentiation and maturation), and activation and bone destruction in RA are RANKL and TNF-α (Romas E. et al., Bone 30, pp. 340-346, 2002). OCIF/OPG which is a decoy receptor for RANKL can inhibit osteoclast formation induced by RANKL but does not inhibit osteoclast formation induced by TNF-α. On the other hand, the anti-Siglec-15 antibody according to the invention effectively inhibited osteoclast formation induced by both RANKL and TNF-α. Therefore, it is expected that the anti-Siglec-15 antibody of the invention can inhibit bone loss and bone destruction induced by TNF-α in RA or the like more strongly than an RANKL blocker (OCIF/OPG, an anti-RANKL antibody, or the like).

As one example, for the treatment or prophylaxis of abnormal bone metabolism, the anti-Siglec-15 antibody can be administered alone or in combination with at least one other therapeutic agent for a bone disease. As another example, the anti-Siglec-15 antibody can be administered in combination with a therapeutically effective amount of a therapeutic agent for abnormal bone metabolism. Examples of the other therapeutic agent which can be administered in combination with the anti-Siglec-15 antibody include, but are not limited to: bisphosphonates (for example, alendronate, etidronate, ibandronate, incadronate, pamidronate, risedronate, and zoledronate), active vitamin D₃, calcitonin and derivatives thereof, hormones such as estradiol, SERMs (selective estrogen receptor modulators), ipriflavone, vitamin K₂ (menatetrenone), calcium preparations, PTH (parathyroid hormone), nonsteroidal anti-inflammatory agents (for example, celecoxib and rofecoxib), soluble TNF receptors (for example, etanercept), anti-TNF-α antibodies or antigen binding fragments of the antibodies (for example, infliximab), anti-PTHrP (parathyroid hormone-related protein) antibodies or antigen binding fragments of the antibodies, IL-1 receptor antagonists (for example, anakinra), anti-IL-6 receptor antibodies or antigen binding fragments of the antibodies (for example, tocilizumab), anti-RANKL antibodies or antigen binding fragments of the antibodies (for example, denosumab), and OCIF (osteoclastogenesis inhibitory factor). Depending on the state of abnormal bone metabolism or the intended degree of the treatment and/or prophylaxis, two or three, or more other therapeutic agents can be administered, and these other therapeutic agents can be administered all together by encapsulating them in the same preparation. The other therapeutic agents and the anti-Siglec-15 antibody can also be administered all together by encapsulating them in the same preparation. In addition, the anti-Siglec-15 antibody and the other therapeutic agents can also be administered all together by encapsulating them in separate preparations. Further, the other medicinal agents and the anti-Siglec-15 antibody can also be separately administered successively, that is, after the other therapeutic agents are administered, a therapeutic agent containing the anti-Siglec-15 antibody or an antigen binding fragment of the antibody as an active ingredient may be administered, or after a therapeutic agent containing the anti-Siglec-15 antibody or an antigen binding fragment of the antibody as an active ingredient is administered, the other therapeutic agents may be administered. In the case of administration in gene therapy, a gene of a proteinous therapeutic agent for a bone disease and a gene of the anti-Siglec-15 antibody can be inserted downstream of the same promoter region or different promoter regions, and can be introduced into the same vector or different vectors.

By conjugating a therapeutic agent for a bone disease to the anti-Siglec-15 antibody or a fragment thereof, a targeted drug conjugate as described in M. C. Garnet "Targeted drug conjugates: principles and progress", Advanced Drug Delivery Reviews, (2001) 53, 171-216 can be produced. For achieving this purpose, other than the antibody molecule, any antibody fragment can be applied as long as it does not completely lose the ability to recognize osteoclasts, and examples thereof include fragments such as Fab, F(ab')2, and Fv. In the invention, the antibody and the fragment can be used in the same manner. The conjugate formed by the anti-Siglec-15 antibody or a fragment thereof and a therapeutic agent for a bone disease can be any of various forms described in M. C. Garnet "Targeted drug conjugates: principles and progress", Advanced Drug Delivery Reviews, (2001) 53, 171-216, G. T. Hermanson "Bioconjugate Techniques" Academic Press, California (1996), Putnam and J. Kopecek "Polymer Conjugates with Anticancer Activity" Advances in Polymer Science (1995) 122, 55-123 and the like. That is, a conjugate in which the anti-Siglec-15 antibody and a therapeutic agent for a bone disease are conjugated to each other chemically and directly or via a spacer such as an oligopeptide and a conjugate formed via an appropriate drug carrier can be exemplified. Examples of the drug carrier include a liposome and a water-soluble polymer. More specific examples of the conjugate formed via such a drug carrier include a conjugate in which the antibody and a therapeutic agent for a bone disease are incorporated in a liposome and the liposome and the antibody are conjugated to each other, and a conjugate in which a therapeutic agent for a bone disease is conjugated to a water-soluble polymer (a compound having a molecular weight of about 1,000 to 100,000) chemically and directly or via a spacer such as an oligopeptide and the antibody is conjugated to the water-soluble polymer. The conjugation of the antibody (or a fragment thereof) to a therapeutic agent for a bone disease or a drug carrier such as a liposome or a water-soluble polymer can be effected by methods known to those skilled in the art such as the method described in G. T. Hermanson "Bioconjugate Techniques" Academic Press, California (1996), Putnam and J. Kopecek "Polymer Conjugates with Anticancer Activity" Advances in Polymer Science (1995) 122, 55-123. The incorporation of a therapeutic agent for a bone disease in a liposome can be effected by methods known to those skilled in the art such as the method described in D. D. Lasic "Liposomes: From Physics to Applications" Elsevier Science Publishers B. V., Amsterdam (1993) or the like. The conjugation of a therapeutic agent for a bone disease to a water-soluble polymer can be effected by a method known to those skilled in the art such as the method described in D. Putnam and J. Kopecek "Polymer Conjugates with Anticancer Activity" Advances in Polymer Science (1995) 122, 55-123. A conjugate of the antibody (or a fragment thereof) and a proteinous therapeutic agent for a bone disease (or a fragment thereof) can be produced by methods known to those skilled in the art through genetic engineering other than the above-mentioned method.

The invention also provides a pharmaceutical composition containing a therapeutically and/or prophylactically effective amount of the anti-Siglec-15 antibody and a pharmaceutically acceptable diluent, carrier, solubilizing agent, emulsifying agent, preservative, and/or adjuvant.

The invention also provides a pharmaceutical composition containing a therapeutically and/or prophylactically effective amount of the anti-Siglec-15 antibody, a therapeutically and/or prophylactically effective amount of at least one therapeutic agent for a bone disease, and a pharmaceutically acceptable diluent, carrier, solubilizing agent, emulsifying agent, preservative, and/or adjuvant. Examples of the therapeutic agent for a bone disease include, but are not limited to, bisphosphonates (for example, alendronate, etidronate, ibandronate, incadronate, pamidronate, risedronate, and zoledronate), active vitamin D₃, calcitonin and derivatives thereof, hormones such as estradiol, SERMs (selective estrogen receptor modulators), ipriflavone, vitamin K₂ (menatetrenone), calcium preparations, PTH (parathyroid hormone), nonsteroidal anti-inflammatory agents (for example, celecoxib and rofecoxib), soluble TNF receptors (for example, etanercept), anti-TNF-α antibodies or antigen binding fragments of the antibodies (for example, infliximab), anti-PTHrP (parathyroid hormone-related protein) antibodies or antigen binding fragments of the antibodies, IL-1 receptor antagonists (for example, anakinra), anti-IL-6 receptor antibodies or antigen binding fragments of the antibodies (for example, tocilizumab), anti-RANKL antibodies or antigen binding fragments of the antibodies (for example, denosumab) and OCIF (osteoclastogenesis inhibitory factor).

A substance to be used in a preparation acceptable in the pharmaceutical composition according to the invention is preferably non-toxic to a person to whom the pharmaceutical composition is to be administered in terms of the dose and concentration.

The pharmaceutical composition of the invention can contain a substance for pharmaceutical use which is capable of changing or maintaining the pH, osmotic pressure, viscosity, transparency, color, isotonicity, aseptic condition, stability, solubility, release rate, absorption rate, or permeability thereof. Examples of such a substance for pharmaceutical use include, but are not limited to, amino acids such as glycine, alanine, glutamine, asparagine, arginine, and lysine; antimicrobial agents; antioxidants such as ascorbic acid, sodium sulfate, and sodium hydrogen sulfite; buffers such as phosphate, citrate, borate buffers, sodium hydrogen carbonate, and Tris-HCl solutions; fillers such as mannitol and glycine; chelating agents such as ethylenediamine tetraacetate (EDTA); complexing agents such as caffeine, polyvinylpyrrolidine, β-cyclodextrin, and hydroxypropyl-β-cyclodextrin; expanders such as glucose, mannose, and dextrin; other carbohydrates such as monosaccharides and disaccharides; coloring agents; flavors; diluents; emulsifying agents; hydrophilic polymers such as polyvinylpyrrolidine; preservatives such as low molecular weight polypeptides, salt forming counter ions, benzalkonium chloride, benzoic acid, salicylic acid, thimerosal, phenethyl alcohol, methylparaben, propylparaben, chlorhexidine, sorbic acid, and hydrogen peroxide; solvents such as glycerin, propylene glycol, and polyethylene glycol; sugar alcohols such as mannitol and sorbitol; suspending agents; surfactants such as sorbitan ester, polysorbates (such as polysorbate 20 and polysorbate 80), Triton, tromethamine, lecithin, and cholesterol; stability enhancing agents such as sucrose and sorbitol; elasticity enhancing agents such as sodium chloride, potassium chloride, and mannitol and sorbitol; transport agents; excipients; and/or pharmaceutical adjuvants. The amount of these substances to be added for pharmaceutical use is preferably from 0.01 to 100 times, particularly preferably from 0.1 to 10 times the weight of the anti-Siglec-15 antibody. Those skilled in the art can appropriately determine a preferred formulation of the pharmaceutical composition in a preparation depending on the disease to which the composition is applied, the route of administration to be applied, or the like.

The excipient or carrier in the pharmaceutical composition may be in the form of a liquid or a solid. An appropriate excipient or carrier may be injectable water, physiological saline, an artificial cerebral spinal fluid, or other substance commonly used for parenteral administration. Further, neutral physiological saline or physiological saline containing serum albumin can also be used as a carrier. The pharmaceutical composition may contain a Tris buffer at pH 7.0 to 8.5, an acetate buffer at pH 4.0 to 5.5, or a citrate buffer at pH 3.0 to 6.2. Further, such a buffer may be supplemented with sorbitol or other compounds. Examples of the pharmaceutical composition of the invention include a pharmaceutical composition containing the anti-Siglec-15 antibody and a pharmaceutical composition containing the anti-Siglec-15 antibody and at least one therapeutic agent for a bone disease. The pharmaceutical composition of the invention is prepared in the form of a lyophilized product or a liquid as a medicament having a selected composition and a required purity. The pharmaceutical composition containing the anti-Siglec-15 antibody and the pharmaceutical composition containing the anti-Siglec-15 antibody and at least one therapeutic agent for abnormal bone metabolism can also be formed into a lyophilized product using an appropriate excipient such as sucrose.

The pharmaceutical composition of the invention can be prepared for parenteral administration or for gastrointestinal absorption through oral administration. The composition and concentration of a preparation can be determined depending on the administration method. The higher the affinity of the anti-Siglec-15 antibody contained in the pharmaceutical composition of the invention is for Siglec-15, that is, the lower the dissociation constant (Kd value) thereof is for Siglec-15, the more the anti-Siglec-15 antibody can exhibit its drug efficacy even when decreasing the dose for humans. Hence, the dose of the pharmaceutical composition of the invention for humans can also be determined based on this consideration. As for the dose, in the case where a human anti-Siglec-15 antibody is administered to humans, the antibody may be administered at a dose of about 0.1 to 100 mg/kg once per one to 180 days.

Examples of the dosage form of the pharmaceutical composition of the invention include injections including infusions, suppositories, transnasal agents, sublingual agents, and percutaneous absorbents.

### Examples

Hereinafter, the invention will be more specifically described with reference to the Examples, however, the invention is not limited thereto. Note that the respective operations regarding gene manipulation in the following Examples were performed according to the methods described in "Molecular Cloning" (written by Sambrook, J., Fritsch, E. F. and Maniatis, T., published by Cold Spring Harbor Laboratory Press in 1989), or in the case of using commercially available reagents or kits, they are used according to the protocols attached thereto unless otherwise stated.

### Example 1

### Preparation of culture solution containing soluble human Siglec-15 protein and purification thereof

An entry clone was produced by amplifying human Siglec-15 extracellular domain cDNA by PCR and integrating the cDNA into a pDNOR221 vector (manufactured by Invitrogen Corporation). From this entry clone, an expression plasmid (soluble human Siglec-15/pDONM) was obtained by recombination into pDONM which is a destination vector designed such that a V5 epitope tag and a 6 x His tag are added to the C terminus of the insert. The soluble human Siglec-15/pDONM was transfected into 293-F cells, and the cells were subjected to spinner culture at a CO₂ concentration of 6 to 12% for 96 hours (4 days) at 37°C. The culture solution was collected and centrifuged to prepare a culture supernatant containing the soluble human Siglec-15 protein. The thus obtained culture supernatant was subjected to partial purification using a Ni-Sepharose HP column (manufactured by Amersham Biosciences, Inc.), followed by fractionation using a Resource Q column (manufactured by Amersham Biosciences, Inc.). Then, by performing SDS-polyacrylamide electrophoresis (under reducing conditions) and silver staining, detection and purity assay of the soluble human Siglec-15 protein were performed. As a result, it was confirmed that a protein having a molecular weight of about 35 kDa (soluble human Siglec-15 protein) was efficiently purified and concentrated in the protein fraction which was not adsorbed to the Resource Q column.

### Example 2

Establishment of rat anti-human Siglec-15 monoclonal antibody #24A3-producing hybridoma and preparation of purified antibody

Rats were immunized with the purified soluble human Siglec-15 protein produced in Example 1. After collecting the blood for a test and confirming that the antibody titer was increased, the rats were euthanized, and then the spleen was excised. Cell fusion was performed according to a common method of fusing mouse (rat) spleen cells and myeloma cells. Thirty five clones having a high antibody titer were selected by screening, and then first and second cloning procedures were performed. As a result, the present inventors succeeded in the establishment of #24A3 as a hybridoma having a high antibody titer. The established hybridoma was intraperitoneally implanted in nude mice at 1 x 10⁷ cells per mouse. After the implantation, the ascites was collected when sufficient accumulation of ascites was observed, and an IgG fraction was subjected to purification. By the above-mentioned operation, a rat anti-human Siglec-15 monoclonal antibody #24A3 was prepared.

### Example 3

### Evaluation of binding activity of rat anti-human Siglec-15 monoclonal antibody #24A3 to human Siglec-15 protein

### 3-1) Expression and purification of human Siglec-15 V-set domain

A DNA encoding a protein in which a His tag and a Factor Xa recognition sequence were attached to the N-terminal side of a human Siglec-15 V-set domain (a polypeptide comprising amino acid residues 39 to 165 of an amino acid sequence with the accession number of NP_998767 in the NCBI Protein database) was integrated into vector pDEST14 (Invitrogen, Corporation, Cat. No. 11801-016). By using this plasmid, Escherichia coli Rosetta-gamiB (DE3) (Novagen, Inc., Cat. No. 71136-4) was transformed and cultured in TB medium (Invitrogen, Corporation, Cat. No. 22711-022). After culturing, the bacterial cells were homogenized by ultrasound, the resulting homogenate was centrifuged, and the supernatant was subjected to purification using a HisTrap HP column (GE Healthcare Ltd., Cat. No. 17-5247-01). Thereafter, the His tag was cleaved with Factor Xa (New England BioLabs Inc., Cat. No. P8010L), and then the human Siglec-15 V-set domain was purified using a Mono S5/50 GL column (GE Healthcare Ltd., Cat. No. 17-5168-01) and a Superdex 75 10/300 column (GE Healthcare Ltd., Cat. No. 17-5174-01) until a single band with a molecular weight of 14 kDa was obtained by electrophoresis.

### 3-2) Measurement of dissociation constant between rat #32A3 and human Siglec-15 V-set domain

The dissociation constant between the rat #24A3 antibody and the human Siglec-15 V-set domain was measured using Biacore T200 (GE Healthcare Bio-Sciences Ltd.) by immobilizing the antibody as a ligand and using the antigen as an analyte. Incidentally, the rat #32A1 antibody was used as a control antibody. The rat #32A1 antibody is described in WO 09/48072 and WO 10/117011 with respect to the preparation method thereof and the information of the heavy and light chain sequences thereof. Incidentally, the hybridoma #32A1 which produces the rat #32A1 antibody was deposited at the International Patent Organism Depositary of the National Institute of Advanced Industrial Science and Technology (located at Central 6, 1-1-1 Higashi, Tsukuba-shi, Ibaraki-ken, 305-8566, Japan) on August 28, 2008, and has been given an accession number of FERM BP-10999 under the name of anti-Siglec-15 Hybridoma #32A1.

The #32A1 or #24A3 antibody was bound to a sensor chip CM5 (GE Healthcare Bio-Sciences Ltd.) at about 50 RU by an amine coupling method via an anti-mouse IgG antibody (GE Healthcare Bio-Sciences Ltd.) immobilized thereon. As a running buffer, HBS-EP+ (10 mM HEPES pH 7.4, 0.15 M NaCl, 3 mM EDTA, 0.05% surfactant P20) was used. On the chip having the antibody bound thereto, a dilution series of an antigen solution (0.003 to 2 nM) was added at a flow rate of 90 µL/min for 233 seconds, and subsequently, the dissociation phase was monitored for 3600 seconds. As a regeneration solution, 3 M MgCl₂ was added at a flow rate of 10 µL/min for 30 seconds. In the analysis of data, a 1:1 binding model of analysis software (Biacore T200 Evaluation software, version 1.0) was used, and an association rate constant (kon), a dissociation rate constant (koff), and a dissociation constant (KD; KD = koff/kon) were calculated. According to the results, #32A1 had a KD value of 1. 5E-10 [M], and #24A3 had a KD value of 7.0E-11 [M], and therefore, #24A3 had about a 2-fold greater affinity than #32A1.

### Example 4

### Amplification of cDNA encoding variable region of rat anti-human Siglec-15 monoclonal antibody #24A3 and base sequence analysis thereof

### 4-1) Identification of N-terminal amino acid sequences of heavy and light chains of #24A3

In order to identify the N-terminal amino acid sequences of the heavy and light chains of #24A3, #24A3 prepared in Example 2 was separated by SDS-PAGE. The protein in the gel was transferred from the gel after separation to a Sequi-Blot PVDF membrane (Bio-Rad Laboratories, Inc.). The membrane was washed with a washing buffer (25 mM NaCl, 10 mM sodium borate buffer pH 8.0), and thereafter stained by being immersed in a dye solution (50% methanol, 20% acetic acid, 0.05% Coomassie brilliant blue) for 5 minutes, followed by destaining with 90% methanol. The portions of the band corresponding to the heavy chain (the band with smaller mobility) and the band corresponding to the light chain (the band with larger mobility) visualized on the PVDF membrane were excised. The band corresponding to the heavy chain was incubated at 37°C for 30 minutes in a small amount of a 0.5% polyvinylpyrrolidone/100 mM acetic acid solution, followed by washing with water. Subsequently, by using a Pfu pyroglutamate aminopeptidase kit (TaKaRa Bio, Inc.), the modified N-terminal residue was removed, followed by washing with water and air-drying. Then, an attempt was made to identify their respective N-terminal amino acid sequences by an automatic Edman method (see Edman et al. (1967) Eur. J. Biochem. 1, 80) using Procise cLC Protein Sequencer Model 492cLC (Applied Biosystems, Inc.). According to the results, the N-terminal amino acid sequence of the band corresponding to the heavy chain of #24A3 was VQLQQSGAELTKP, and the N-terminal amino acid sequence of the band corresponding to the light chain thereof was DIVMTQSPTSMSIS.

### 4-2) Preparation of mRNA from #24A3-producing hybridoma

In order to amplify cDNA containing a variable region of #24A3, mRNA was prepared from the #24A3-producing hybridoma using a mRNA isolation kit (Roche Applied Science, Inc.).

### 4-3) Synthesis of cDNA (5'-RACE-Ready cDNA)

The synthesis of cDNA (5' -RACE-Ready cDNA) was performed using 100 ng of the mRNA prepared in the above 4-2) with SMARTer RACE cDNA Amplification Kit (Clontech Laboratories, Inc.).

### 4-4) Amplification of cDNA containing #24A3 heavy chain variable region by 5'-RACE PCR and sequence determination

As primers for amplifying the cDNA of the #24A3 heavy chain gene variable region by PCR, UPM (Universal Primer A Mix, attached to the SMARTer RACE cDNA Amplification Kit) and an oligonucleotide having a sequence of 5'-CTCCAGAGTTCCAGGTCACGGTGACTGGC-3' (RG2AR3) were used. As the UPM, one attached to the SMARTer RACE cDNA Amplification Kit (Clontech Laboratories, Inc.) was used, and the RG2AR3 was designed based on the sequence of the rat heavy chain (IgG2a) constant region in the database.

The cDNA containing the #24A3 heavy chain variable region was amplified by 5' -RACE PCR using this combination of primers and the cDNA synthesized in the above 4-3) (5' -RACE-Ready cDNA) as a template. The PCR was performed using KOD -Plus- (TOYOBO, Co., Ltd.) as a polymerase according to the protocol attached to the SMARTer RACE cDNA Amplification Kit (Clontech Laboratories, Inc.) using a Touchdown PCR program.

The cDNA containing the heavy chain variable region amplified by 5'-RACE PCR was purified using MinElute PCR Purification Kit (QIAGEN, Inc.), and then cloned using Zero Blunt TOPO PCR Cloning Kit (Invitrogen Corporation), and the sequence analysis of the nucleotide sequence of the cloned cDNA containing the heavy chain variable region was performed.

As the sequence primers, an oligonucleotide having a sequence of 5'-CTCCAGAGTTCCAGGTCACGGTGACTGGC-3' (RG2AR3) designed based on the sequence of the rat heavy chain constant region in the database and NUP (Nested Universal Primer A: attached to the SMARTer RACE cDNA Amplification Kit) were used.

The sequence analysis was performed using a gene sequencer (ABI PRISM 3700 DNA Analyzer (Applied Biosystems) or Applied Biosystems 3730x1 Analyzer (Applied Biosystems)), and in the sequence reaction, GeneAmp 9700 (Applied Biosystems, Inc.) was used.

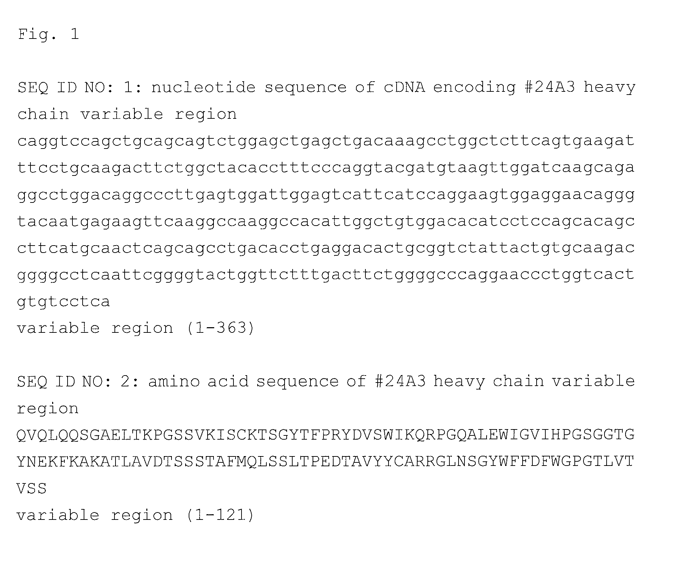

The determined nucleotide sequence of the cDNA encoding the #24A3 heavy chain variable region is represented by SEQ ID NO: 1 in the Sequence Listing, and the amino acid sequence thereof is represented by SEQ ID NO: 2. The nucleotide sequence of SEQ ID NO: 1 and the amino acid sequence of SEQ ID NO: 2 are also shown in Fig. 1.

The amino acid sequence of the #24A3 heavy chain variable region determined from the nucleotide sequence matched the N-terminal amino acid sequence determined in the above 4-1).

### 4-5) Amplification of cDNA containing #24A3 light chain variable region by 5'-RACE PCR and sequence determination

As primers for amplifying the cDNA of the #24A3 light chain gene variable region by PCR, UPM (Universal Primer A Mix, attached to the SMARTer RACE cDNA Amplification Kit) and an oligonucleotide having a sequence of 5'-TCAGTAACACTGTCCAGGACACCATCTC-3' (RKR5) were used. As the UPM, one attached to the SMARTer RACE cDNA Amplification Kit (Clontech Laboratories, Inc.) was used, and the RKR3 was designed based on the sequence of the rat light chain constant region in the database.

The cDNA containing the #24A3 light chain variable region was amplified by 5' -RACE PCR using this combination of primers and the cDNA synthesized in the above 4-3) (5' -RACE-Ready cDNA) as a template. The PCR was performed using KOD -Plus- (TOYOBO, Co., Ltd.) as a polymerase according to the protocol attached to the SMARTer RACE cDNA Amplification Kit (ClontechH Laboratories, Inc.) using a Touchdown PCR program.

The cDNA containing the light chain variable region amplified by 5'-RACE PCR was purified using MinElute PCR Purification Kit (QIAGEN, Inc.), and then cloned using Zero Blunt TOPO PCR Cloning Kit (Invitrogen Corporation), and the sequence analysis of the nucleotide sequence of the cloned cDNA containing the light chain variable region was performed.

As the sequence primers, an oligonucleotide having a sequence of 5'-TCAGTAACACTGTCCAGGACACCATCTC-3' (RKR5) designed based on the sequence of the rat light chain constant region in the database and NUP (Nested Universal Primer A: attached to the SMARTer RACE cDNA Amplification Kit) were used.

The sequence analysis was performed using a gene sequencer (ABI PRISM 3700 DNA Analyzer (Applied Biosystems) or Applied Biosystems 3730x1 Analyzer (Applied Biosystems)), and in the sequence reaction, GeneAmp 9700 (Applied Biosystems, Inc.) was used.

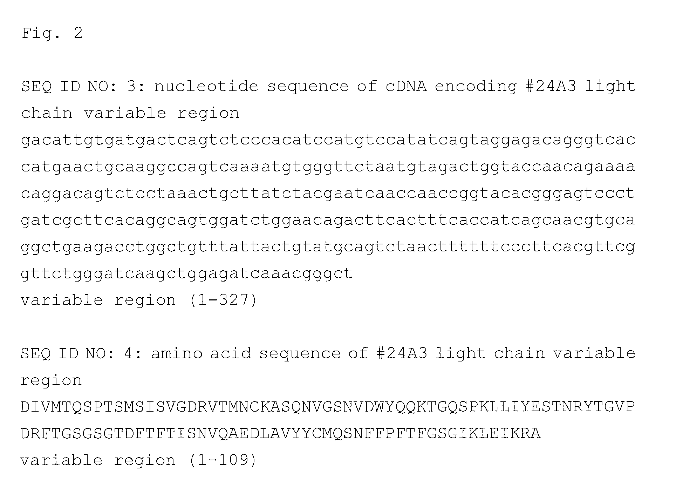

The determined nucleotide sequence of the cDNA encoding the #24A3 light chain variable region is represented by SEQ ID NO: 3 in the Sequence Listing, and the amino acid sequence thereof is represented by SEQ ID NO: 4. The nucleotide sequence of SEQ ID NO: 3 and the amino acid sequence of SEQ ID NO: 4 are also shown in Fig. 2. The amino acid sequence of the #24A3 light chain variable region determined from the nucleotide sequence matched the N-terminal amino acid sequence determined in the above 4-1).

### Example 5

### Production of gene expression construct of human chimeric antibody of rat anti-human Siglec-15 monoclonal antibody #24A3

### 5-1) Construction of chimeric and humanized light chain expression vector pCMA-LK

A fragment of about 5.4 kb obtained by digesting a plasmid pcDNA3.3-TOPO/LacZ (Invitrogen Corporation) with the restriction enzymes XbaI and PmeI and a DNA fragment containing a nucleotide sequence encoding a human κ chain secretory signal and a human κ chain constant region represented by SEQ ID NO: 5 were ligated using In-Fusion Advantage PCR Cloning Kit (Clontech Laboratories, Inc.), whereby pcDNA3.3/LK was produced.

By using pcDNA3.3/LK as a template and also using the following primer set, PCR was performed, and the obtained fragment of about 3.8 kb was phosphorylated, followed by self-ligation, whereby a chimeric and humanized light chain expression vector pCMA-LK having a signal sequence, a cloning site, and a human κ chain constant region downstream of the CMV promoter was constructed.
Primer set
5'-tataccgtcgacctctagctagagcttggc-3' (3.3-F1)
5'-gctatggcagggcctgccgccccgacgttg-3' (3.3-R1)

### 5-2) Construction of chimeric and humanized IgG2 type heavy chain expression vector pCMA-G2

A DNA fragment, obtained by digesting pCMA-LK with XbaI and PmeI and removing the κ chain secretory signal and the human κ chain constant region, and a DNA fragment (SEQ ID NO: 6) containing a nucleotide sequence encoding a human heavy chain secretory signal and a human IgG2 constant region were ligated using In-Fusion Advantage PCR Cloning Kit (Clontech Laboratories, Inc.), whereby a chimeric and humanized IgG2 type heavy chain expression vector pCMA-G2 having a signal sequence, a cloning site, and a human IgG2 heavy chain constant region downstream of the CMV promoter was constructed.

### 5-3) Construction of human chimeric #24A3 light chain expression vector

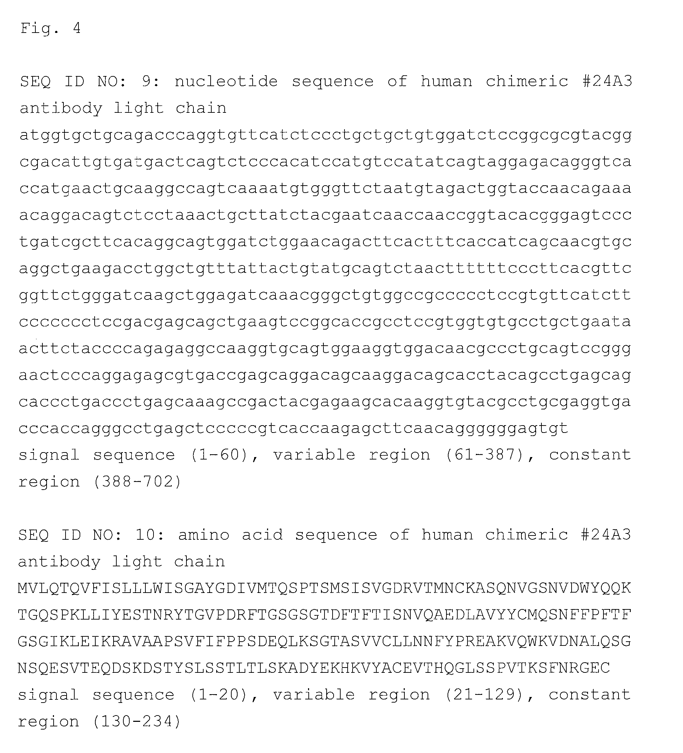

By using the cDNA containing the #24A3 light chain variable region obtained in Example 4) as a template and also using KOD -Plus- (TOYOBO, Co., Ltd.) and the following primer set, a DNA fragment containing the cDNA encoding the light chain variable region was amplified and then inserted into the chimeric and humanized antibody light chain expression vector pCMA-LK for universal use at the site cleaved with the restriction enzyme BsiWI using In-Fusion Advantage PCR Cloning Kit (Clontech Laboratories, Inc.), whereby a human chimeric #24A3 light chain expression vector was constructed. The thus obtained expression vector was named "pCMA-LK/#24A3". The produced human chimeric #24A3 light chain gene has a nucleotide sequence represented by SEQ ID NO: 9 in the Sequence Listing and encodes an amino acid sequence represented by SEQ ID NO: 10 in the Sequence Listing. The sequence comprising nucleotides 1 to 60 of the nucleotide sequence of SEQ ID NO: 9, the sequence comprising nucleotides 61 to 387 thereof, and the sequence comprising nucleotides 388 to 702 thereof encode a signal sequence, a light chain variable region sequence, and a light chain constant region sequence, respectively. The amino acid sequence represented by amino acid numbers 1 to 20 of SEQ ID NO: 10 corresponds to a secretory signal, the amino acid sequence of SEQ ID NOs: 21 to 129 corresponds to a light chain variable region, and the amino acid sequence represented by amino acid numbers 130 to 234 thereof corresponds to a light chain constant region. The nucleotide sequence of SEQ ID NO: 9 and the amino acid sequence of SEQ ID NO: 10 are also shown in FIG. 4.
Primer set for human chimeric #24A3 light chain
5'-GATCTCCGGCGCGTACGGCGACATTGTGATGACTCAGTCTCCCACATCC -3' (24A3L-F)
5'-GGAGGGGGCGGCCACAGCCCGTTTGATCTCCAGCTTGATCCCAG-3' (24A3L-R)

### 5-4) Construction of human chimeric #24A3 heavy chain expression vector

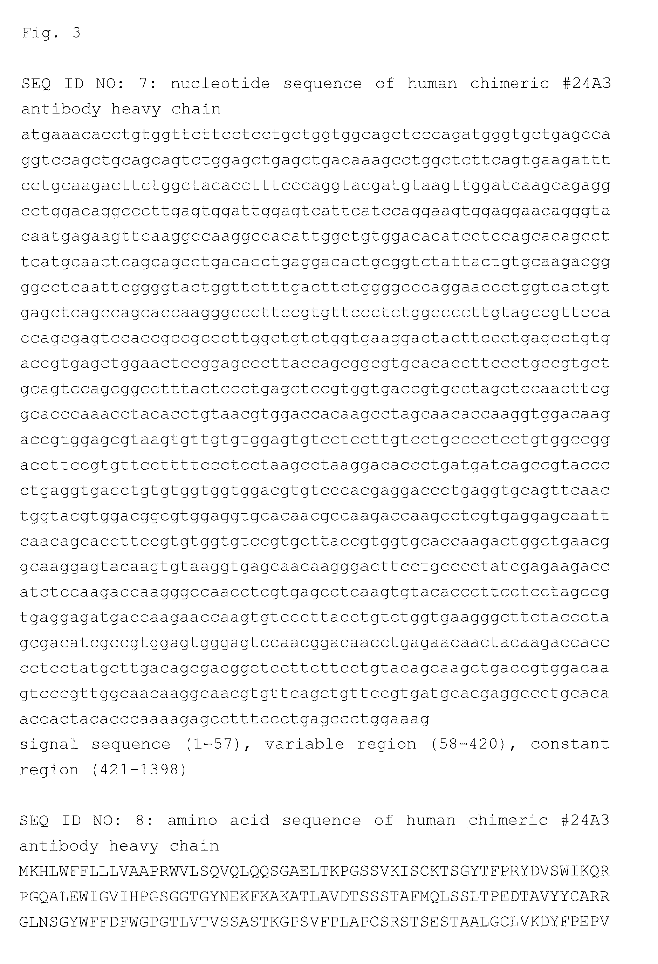

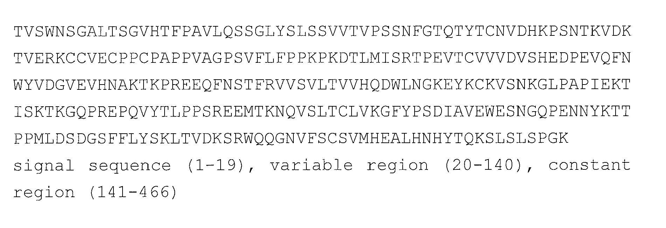

By using the cDNA containing the #24A3 heavy chain variable region obtained in Example 4) as a template and also using KOD -Plus- (TOYOBO, Co., Ltd.) and the following primer set, a DNA fragment containing the cDNA encoding the heavy chain variable region was amplified and then inserted into the chimeric and humanized IgG2 type heavy chain expression vector pCMA-G2 at the site cleaved with the restriction enzyme BlpI using In-Fusion Advantage PCR Cloning Kit (Clontech Laboratories, Inc.), whereby a human chimeric #24A3 heavy chain expression vector was constructed. The thus obtained expression vector was named "pCMA-G2/#24A3". The produced human chimeric #24A3 heavy chain gene has a nucleotide sequence represented by SEQ ID NO: 7 in the Sequence Listing and encodes an amino acid sequence represented by SEQ ID NO: 8 in the Sequence Listing. The sequence comprising nucleotides 1 to 57 of the nucleotide sequence of SEQ ID NO: 7, the sequence comprising nucleotides 58 to 420 thereof, and the sequence comprising nucleotides 421 to 1398 thereof encode a signal sequence, a heavy chain variable region sequence, and a heavy chain constant region sequence, respectively. The amino acid sequence represented by amino acid numbers 1 to 19 of SEQ ID NO: 8 corresponds to a secretory signal, the amino acid sequence represented by amino acid numbers 20 to 140 thereof corresponds to a heavy chain variable region, and the amino acid sequence represented by amino acid numbers 141 to 466 thereof corresponds to a heavy chain constant region. The nucleotide sequence of SEQ ID NO: 7 and the amino acid sequence of SEQ ID NO: 8 are also shown in FIG. 3.
Primer set for human chimeric #24A3 heavy chain
5'-CCAGATGGGTGCTGAGCCAGGTCCAGCTGCAGCAGTCTGGAGCTGAG-3 ' (24A3H-F)
5'-CTTGGTGCTGGCTGAGCTCACAGTGACCAGGGTTCCTGGGCCCCAG-3' (24A3RR)

### Example 6

### Preparation of human chimeric antibody of rat anti-human Siglec-15 monoclonal antibody #24A3

### 6-1) Production of human chimeric antibody of rat anti-human Siglec-15 monoclonal antibody #24A3

FreeStyle 293F cells (Invitrogen Corporation) were subcultured and cultured according to the protocol. 1.2 x 10⁹ cells of FreeStyle 293F cells (Invitrogen Corporation) in logarithmic growth phase were seeded in a 3-L Fernbach Erlenmeyer Flask (Corning Incorporated) and prepared at 1.0 x 10⁶ cells/ml by dilution with FreeStyle 293 expression medium (Invitrogen Corporation), and then shaking culture was performed at 90 rpm for 1 hour at 37°C in an 8% CO₂ incubator. 3.6 mg of polyethyleneimine (Polyscience #24765) was dissolved in 20 ml of Opti-Pro SFM medium (Invitrogen Corporation). Subsequently, the heavy chain expression vector (0.4 mg) and the light chain expression vector (0.8 mg) prepared using NucleoBond Xtra (TaKaRa Bio, Inc.) were suspended in 20 ml of Opti-Pro SFM medium (Invitrogen Corporation). Then, 20 ml of the obtained expression vectors/Opti-Pro SFM mixture was added to 20 ml of the obtainedpolyethyleneimine/Opti-Pro SFM mixture, and the resulting mixture was gently stirred and then left for 5 minutes. Thereafter, the mixture was added to the FreeStyle 293F cells, and shaking culture was performed at 90 rpm for 7 days at 37°C in an 8% CO₂ incubator. The resulting culture supernatant was filtered through a disposable capsule filter (Advantec #CCS-045-E1H).

A human chimeric antibody of the rat anti-human Siglec-15 monoclonal antibody #24A3 obtained by a combination of pCMA-G2/#24A3 and pCMA-LK/#24A3 was named "c#24A3".

### 6-2) Purification of human chimeric antibody of rat anti-human Siglec-15 monoclonal antibody #24A3

The antibody was purified from the culture supernatant obtained in the above 6-1) by a two-step process including rProtein A affinity chromatography (at 4 to 6°C) and ceramic hydroxyapatite (at room temperature). A buffer replacement step after the purification by rProtein A affinity chromatography and after the purification by ceramic hydroxyapatite was performed at 4 to 6°C. First, the culture supernatant was applied to MabSelect SuRe (manufactured by GE Healthcare Bio-Sciences Ltd., HiTrap column) equilibrated with PBS. After all culture solution was poured into the column, the column was washed with PBS in an amount twice or more the volume of the column. Subsequently, elution was performed with a 2 M arginine hydrochloride solution (pH 4.0), and a fraction containing the antibody was collected. After the collected fraction was subjected to buffer replacement with PBS by dialysis (Thermo Scientific, Inc., Slide-A-Lyzer Dialysis Cassette), an antibody solution prepared by 5-fold dilution with a buffer containing 5 mM sodium phosphate and 50 mM MES at pH 7.0 was applied to a ceramic hydroxyapatite column (Bio-Rad Laboratories, Inc. (Japan), Bio-Scale CHT Type-I hydroxyapatite column) equilibrated with a buffer containing 5 mM NaPi, 50 mM MES, and 30 mM NaCl at pH 7.0. Then, linear concentration gradient elution with sodium chloride was performed, and a fraction containing the antibody was collected, and the collected fraction was subjected to buffer replacement with HBSor (25 mM histidine/5% sorbitol, pH 6.0) by dialysis (Thermo Scientific, Inc., Slide-A-Lyzer Dialysis Cassette). Finally, the resulting solution was concentrated using Centrifugal UF Filter Device VIVASPIN 20 (fractional molecular weight UF: 10 K, Sartorius Co. , Ltd. , at 4°C), the concentration of IgG was adjusted to 10 mg/ml, and the thus obtained solution was used as a purified sample.

### Example 7

### Designing of humanized antibody of rat anti-mouse Siglec-15 monoclonal antibody #24A3

### a) Designing of humanized version of #24A3

### a)-i) Molecular modeling of variable region of #24A3

The molecular modeling of the variable region of #24A3 was performed by a method generally known as homology modeling (Methods in Enzymology, 203, 121-153, (1991)). The primary sequences (three-dimensional structures derived from the X-ray crystal structures are available) of the variable regions of human immunoglobulin registered in Protein Data Bank (Nuc. Acid Res. 35, D301-D303 (2007)) were compared with the variable region of #24A3 determined above. As a result, 12E8 was selected as having the highest sequence homology with the variable region of the light chain of #24A3 among antibodies having a similar deletion in the framework. Further, 2OSL was selected as having the highest sequence homology with the variable region of the heavy chain of #24A3. The three-dimensional structure of the framework region was generated by obtaining the "framework model" by combining the coordinates of 12E8 and 2OSL corresponding to the light chain and heavy chain of #24A3, respectively. Then, the representative conformations of the respective CDRs were integrated into the framework model.

Finally, in order to obtain a probable molecular model of the variable region of #24A3 in terms of energy, an energy calculation was performed for excluding disadvantageous interatomic contact. The above procedure was performed using a commercially available protein conformational analysis program Discovery Studio (Accelrys, Inc.).

### a)-ii) Designing of amino acid sequence of humanized #24A3

A humanized #24A3 antibody was constructed according to a method generally known as CDR grafting (Proc. Natl. Acad. Sci. USA 86, 10029-10033 (1989)). An acceptor antibody was selected in two ways based on the amino acid homology within the framework region. The sequence of the framework region of #24A3 was compared with the amino acid sequences of all the human frameworks in the Kabat Database (Nuc. Acid Res. 29, 205-206 (2001)). As a result, an HuMc3 antibody was selected as an acceptor based on a sequence homology of 74% in the framework region. The amino acid residues in the framework region of HuMc3 were aligned with the amino acid residues of #24A3, and the positions where different amino acids were used were identified. The positions of these residues were analyzed using the three-dimensional model of #24A3 constructed above. Then, donor residues to be grafted onto the acceptor were selected according to the criteria provided by Queen et al. (Proc. Natl. Acad. Sci. USA 86, 10029-10033 (1989)). By transferring some selected donor residues to the acceptor antibody, humanized #32A1 sequences were constructed as described in the following Example. Further, a CDR-modified humanized #24A3 sequence including substitution of one to three amino acid residues in each CDR of #24A3 with other amino acid residues was also constructed as described in the following Example.

### b) Humanization of #24A3 heavy chain

### b)-i) h#24A3-H1-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H1-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, threonine (amino acid number 43) with alanine, proline (amino acid number 49) with threonine, isoleucine (amino acid number 56) with valine, lysine (amino acid number 57) with arginine, arginine (amino acid number 59) with alanine, alanine (amino acid number 63) with glycine, isoleucine (amino acid number 67) with methionine, lysine (amino acid number 86) with arginine, alanine (amino acid number 87) with valine, leucine (amino acid number 89) with isoleucine, alanine (amino acid number 90) with threonine, valine (amino acid number 91) with alanine, serine (amino acid number 95) with threonine, phenylalanine (amino acid number 99) with tyrosine, glutamine (amino acid number 101) with glutamic acid, threonine (amino acid number 106) with arginine, proline (amino acid number 107) with serine, and proline (amino acid number 132) with glutamine.

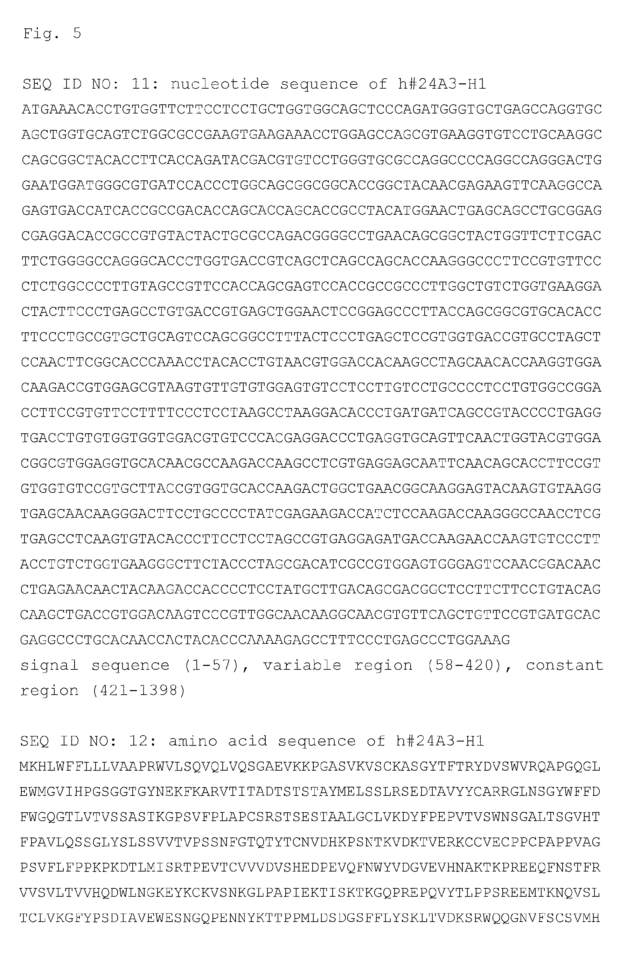

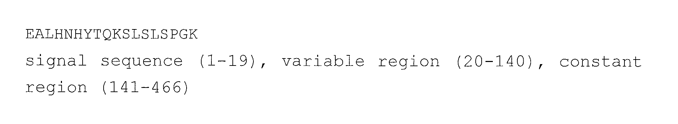

The amino acid sequence of the h#24A3-H1-type heavy chain is represented by SEQ ID NO: 12 in the Sequence Listing. The sequence comprising amino acid residues 1 to 19 of the amino acid sequence of SEQ ID NO: 12, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 12 is represented by SEQ ID NO: 11 in the Sequence Listing. The sequence comprising nucleotides 1 to 57 of the nucleotide sequence of SEQ ID NO: 11, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 11 and the amino acid sequence of SEQ ID NO: 12 are also shown in Fig. 5.

### b)-ii) h#24A3-H2-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H2-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, threonine (amino acid number 43) with alanine, isoleucine (amino acid number 56) with valine, lysine (amino acid number 57) with arginine, arginine (amino acid number 59) with alanine, alanine (amino acid number 63) with glycine, isoleucine (amino acid number 67) with methionine, lysine (amino acid number 86) with arginine, alanine (amino acid number 87) with valine, leucine (amino acid number 89) with isoleucine, alanine (amino acid number 90) with threonine, valine (amino acid number 91) with alanine, serine (amino acid number 95) with threonine, phenylalanine (amino acid number 99) with tyrosine, glutamine (amino acid number 101) with glutamic acid, threonine (amino acid number 106) with arginine, proline (amino acid number 107) with serine, and proline (amino acid number 132) with glutamine.

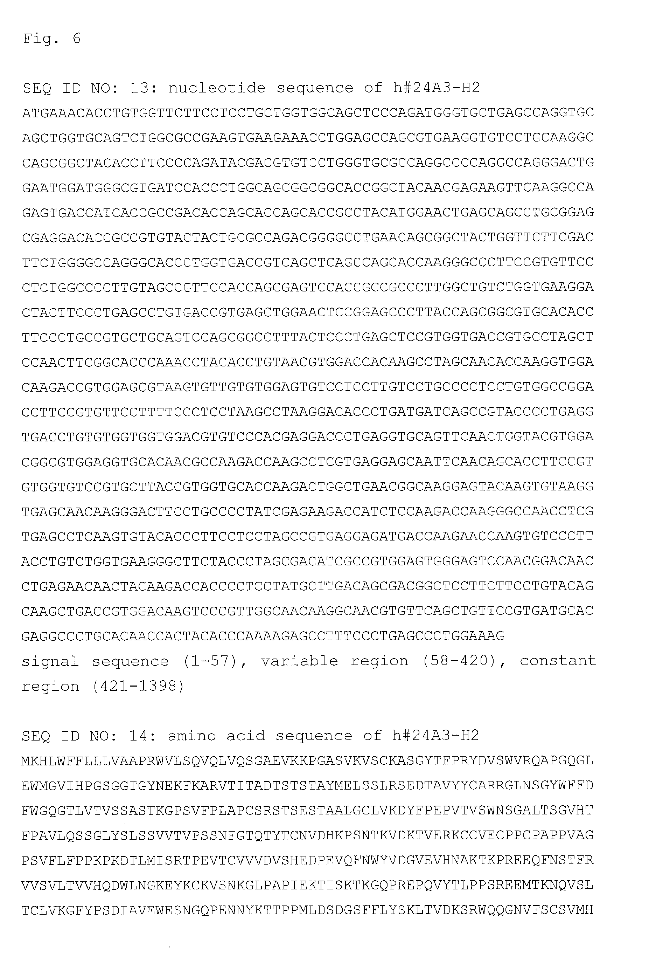

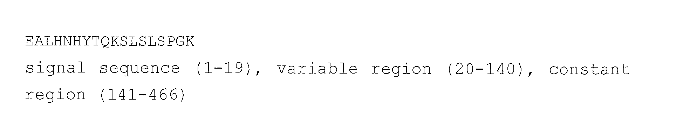

The amino acid sequence of the h#24A3-H2-type heavy chain is represented by SEQ ID NO: 14 in the Sequence Listing. The sequence comprising amino acid residues 1 to 19 of the amino acid sequence of SEQ ID NO: 14, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 14 is represented by SEQ ID NO: 13 in the Sequence Listing. The sequence comprising nucleotides 1 to 57 of the nucleotide sequence of SEQ ID NO: 13, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 13 and the amino acid sequence of SEQ ID NO: 14 are also shown in Fig. 6.

### b)-iii) h#24A3-H3-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H3-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, threonine (amino acid number 43) with alanine, lysine (amino acid number 57) with arginine, arginine (amino acid number 59) with alanine, alanine (amino acid number 63) with glycine, lysine (amino acid number 86) with arginine, leucine (amino acid number 89) with isoleucine, alanine (amino acid number 90) with threonine, serine (amino acid number 95) with threonine, phenylalanine (amino acid number 99) with tyrosine, glutamine (amino acid number 101) with glutamic acid, threonine (amino acid number 106) with arginine, proline (amino acid number 107) with serine, and proline (amino acid number 132) with glutamine.

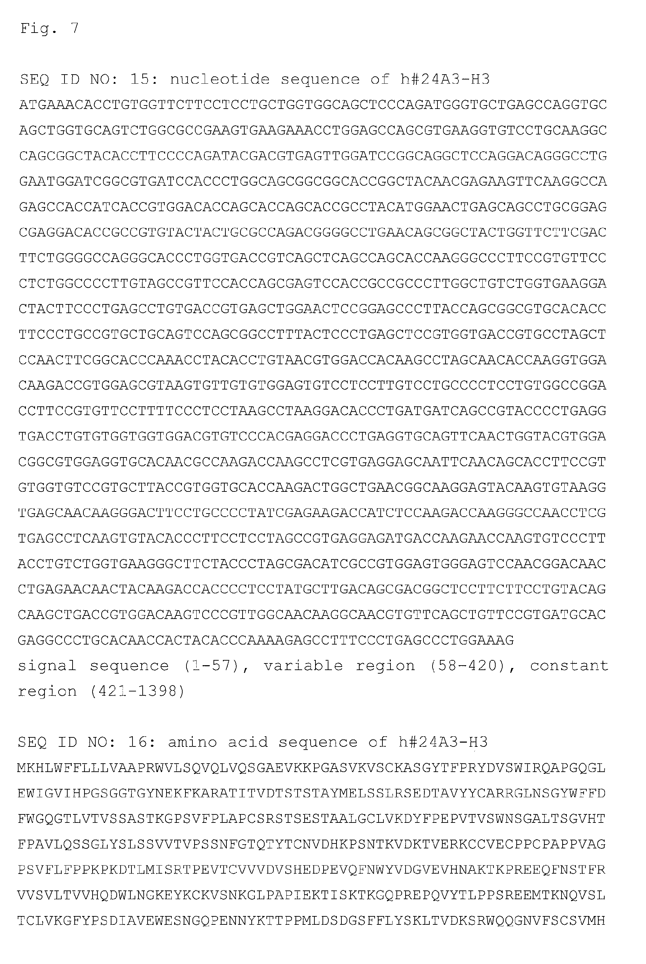

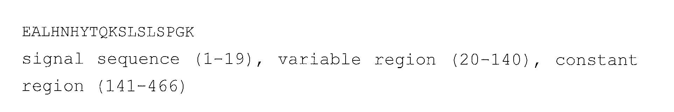

The amino acid sequence of the h#24A3-H3-type heavy chain is represented by SEQ ID NO: 16 in the Sequence Listing. The sequence comprising amino acid residues 1 to 19 of the amino acid sequence of SEQ ID NO: 16, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 16 is represented by SEQ ID NO: 15 in the Sequence Listing. The sequence comprising nucleotides 1 to 57 of the nucleotide sequence of SEQ ID NO: 15, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 15 and the amino acid sequence of SEQ ID NO: 16 are also shown in Fig. 7.

### b)-iv) h#24A3-H4-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H4-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, arginine (amino acid number 59) with alanine, alanine (amino acid number 63) with glycine, lysine (amino acid number 86) with arginine, alanine (amino acid number 90) with threonine, serine (amino acid number 95) with threonine, phenylalanine (amino acid number 99) with tyrosine, glutamine (amino acid number 101) with glutamic acid, threonine (amino acid number 106) with arginine, proline (amino acid number 107) with serine, and proline (amino acid number 132) with glutamine.

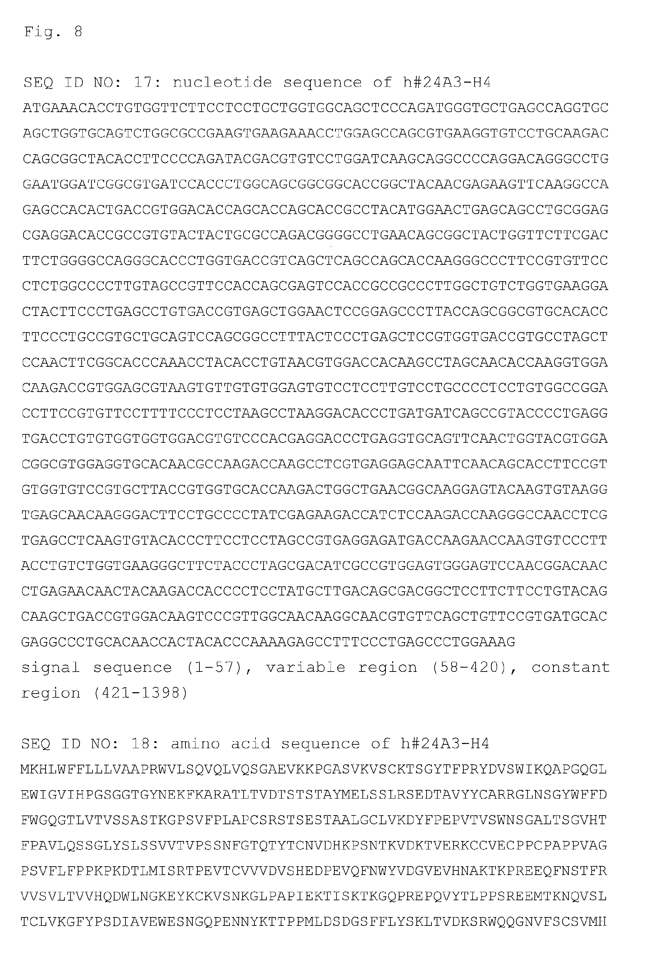

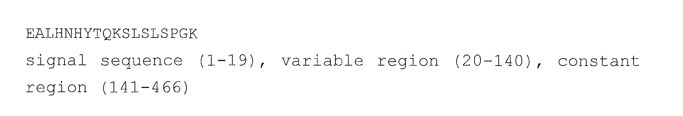

The amino acid sequence of the h#24A3-H4-type heavy chain is represented by SEQ ID NO: 18 in the Sequence Listing. The sequence comprising amino acid residues 1 to 19 of the amino acid sequence of SEQ ID NO: 18, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 18 is represented by SEQ ID NO: 17 in the Sequence Listing. The sequence comprising nucleotides 1 to 57 of the nucleotide sequence of SEQ ID NO: 17, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 17 and the amino acid sequence of SEQ ID NO: 18 are also shown in Fig. 8.

### b)-v) h#24A3-H5-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H5-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, serine (amino acid number 95) with threonine, phenylalanine (amino acid number 99) with tyrosine, glutamine (amino acid number 101) with glutamic acid, threonine (amino acid number 106) with arginine, and proline (amino acid number 107) with serine.

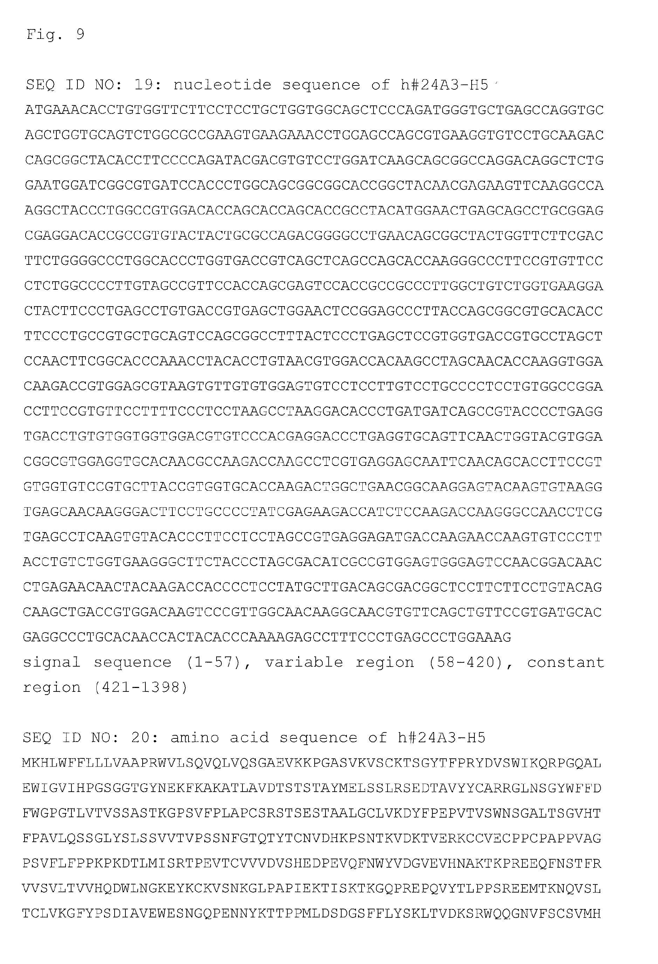

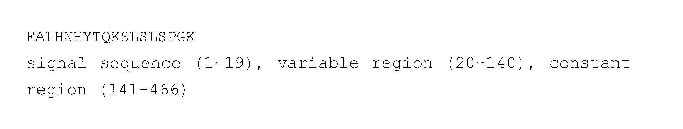

The amino acid sequence of the h#24A3-H5-type heavy chain is represented by SEQ ID NO: 20 in the Sequence Listing. The sequence comprising amino acid residues 1 to 19 of the amino acid sequence of SEQ ID NO: 20, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 20 is represented by SEQ ID NO: 19 in the Sequence Listing. The sequence comprising nucleotides 1 to 57 of the nucleotide sequence of SEQ ID NO: 19, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 19 and the amino acid sequence of SEQ ID NO: 20 are also shown in Fig. 9.

### b)-vi) h#24A3-H6-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H6-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, serine (amino acid number 95) with threonine, glutamine (amino acid number 101) with glutamic acid, threonine (amino acid number 106) with arginine, and proline (amino acid number 107) with serine.

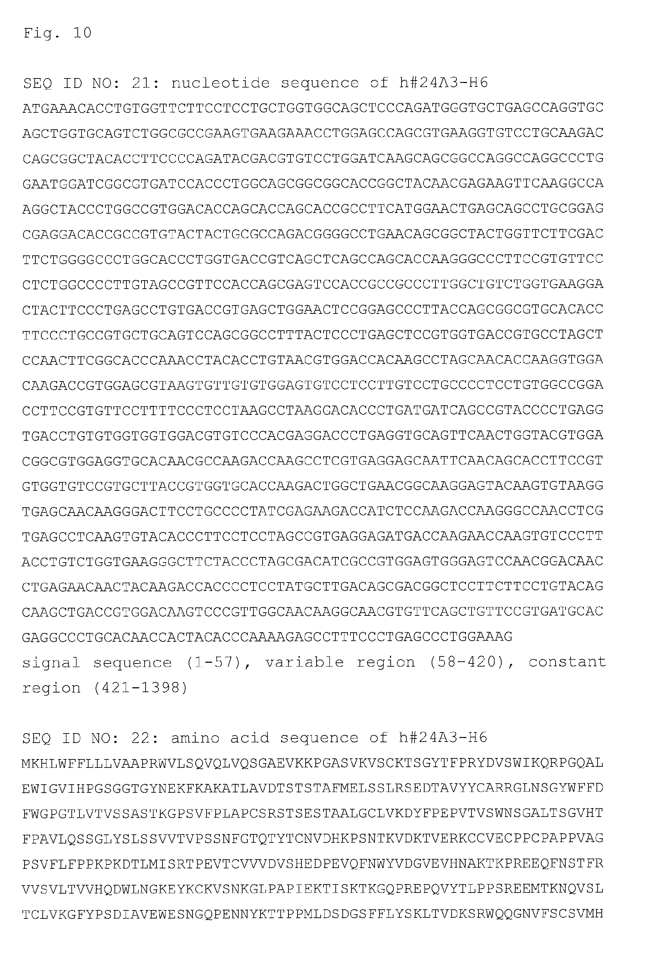

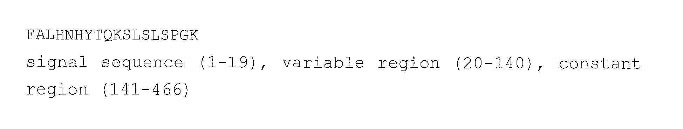

The amino acid sequence of the h#24A3-H6-type heavy chain is represented by SEQ ID NO: 22 in the Sequence Listing. The sequence comprising amino acid residues 1 to 19 of the amino acid sequence of SEQ ID NO: 22, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 22 is represented by SEQ ID NO: 21 in the Sequence Listing. The sequence comprising nucleotides 1 to 57 of the nucleotide sequence of SEQ ID NO: 21, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 21 and the amino acid sequence of SEQ ID NO: 22 are also shown in Fig. 10.

### c) Humanization of #24A3 light chain

### c)-i) h#24A3-L1-type light chain:

A humanized #24A3 light chain designed by substituting the following amino acid residues (amino acid numbers) of the human chimeric #24A3 light chain represented by SEQ ID NO: 10 in the Sequence Listing with the following amino acid residues, respectively, was named "h#24A3-L1-type light chain": threonine (amino acid number 29) with aspartic acid, methionine (amino acid number 31) with leucine, serine (amino acid number 32) with alanine, isoleucine (amino acid number 33) with valine, valine (amino acid number 35) with leucine, aspartic acid (amino acid number 37) with glutamic acid, valine (amino acid number 39) with alanine, methionine (amino acid number 41) with isoleucine, threonine (amino acid number 60) with proline, serine (amino acid number 63) with proline, threonine (amino acid number 83) with serine, phenylalanine (amino acid number 93) with leucine, asparagine (amino acid number 97) with serine, valine (amino acid number 98) with leucine, leucine (amino acid number 103) with valine, serine (amino acid number 120) with glutamine, isoleucine (amino acid number 122) with threonine, leucine (amino acid number 124) with valine, and alanine (amino acid number 129) with threonine.

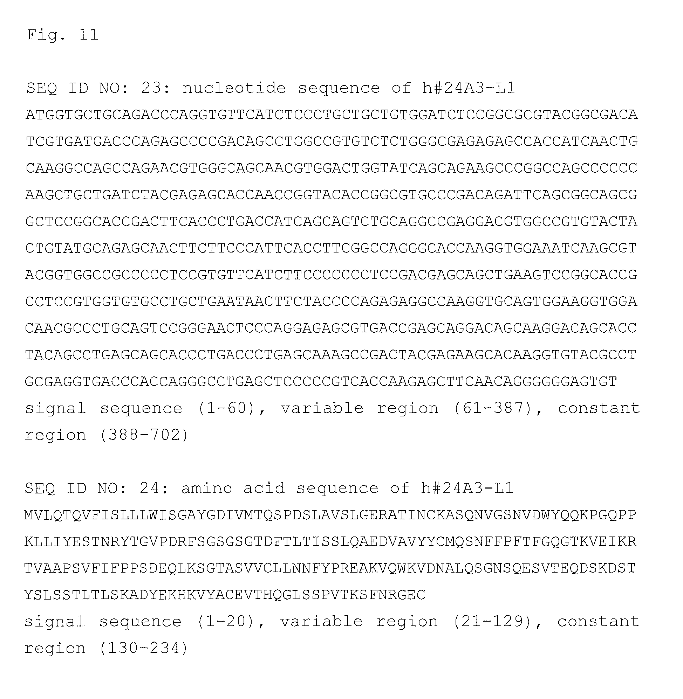

The amino acid sequence of the h#24A3-L1-type light chain is represented by SEQ ID NO: 24 in the Sequence Listing. The sequence comprising amino acid residues 1 to 20 of the amino acid sequence of SEQ ID NO: 24, the sequence comprising amino acid residues 21 to 129 thereof, and the sequence comprising amino acid residues 130 to 234 thereof correspond to the signal sequence, the light chain variable region, and the light chain constant region, respectively. A nucleotide sequence encoding the amino acid sequence of SEQ ID NO: 24 is represented by SEQ ID NO: 23 in the Sequence Listing. The sequence comprising nucleotides 1 to 60 of the nucleotide sequence of SEQ ID NO: 23, the sequence comprising nucleotides 61 to 387 thereof, and the sequence comprising nucleotides 388 to 702 thereof encode the signal sequence, the light chain variable region sequence, and the light chain constant region sequence, respectively. The nucleotide sequence of SEQ ID NO: 23 and the amino acid sequence of SEQ ID NO: 24 are also shown in Fig. 11.

### c)-ii) h#24A3-L2-type light chain:

A humanized #24A3 light chain designed by substituting the following amino acid residues (amino acid numbers) of the human chimeric #24A3 light chain represented by SEQ ID NO: 10 in the Sequence Listing with the following amino acid residues, respectively, was named "h#24A3-L2-type light chain": threonine (amino acid number 29) with aspartic acid, methionine (amino acid number 31) with leucine, serine (amino acid number 32) with alanine, isoleucine (amino acid number 33) with valine, valine (amino acid number 35) with leucine, aspartic acid (amino acid number 37) with glutamic acid, valine (amino acid number 39) with alanine, methionine (amino acid number 41) with isoleucine, threonine (amino acid number 60) with proline, threonine (amino acid number 83) with serine, asparagine (amino acid number 97) with serine, valine (amino acid number 98) with leucine, leucine (amino acid number 103) with valine, serine (amino acid number 120) with glutamine, isoleucine (amino acid number 122) with threonine, leucine (amino acid number 124) with valine, and alanine (amino acid number 129) with threonine.

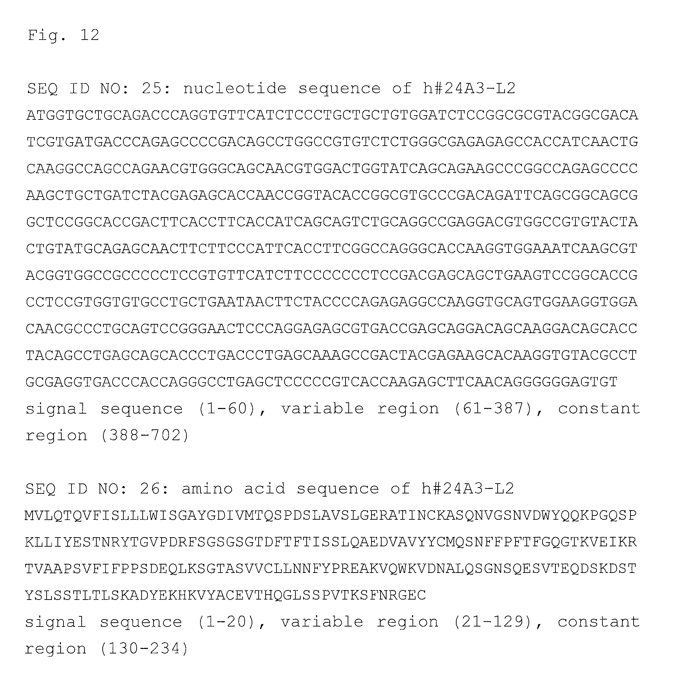

The amino acid sequence of the h#24A3-L2-type light chain is represented by SEQ ID NO: 26 in the Sequence Listing. The sequence comprising amino acid residues 1 to 20 of the amino acid sequence of SEQ ID NO: 26, the sequence comprising amino acid residues 21 to 129 thereof, and the sequence comprising amino acid residues 130 to 234 thereof correspond to the signal sequence, the light chain variable region, and the light chain constant region, respectively. A nucleotide sequence encoding the amino acid sequence of SEQ ID NO: 26 is represented by SEQ ID NO: 25 in the Sequence Listing. The sequence comprising nucleotides 1 to 60 of the nucleotide sequence of SEQ ID NO: 25, the sequence comprising nucleotides 61 to 387 thereof, and the sequence comprising nucleotides 388 to 702 thereof encode the signal sequence, the light chain variable region sequence, and the light chain constant region sequence, respectively. The nucleotide sequence of SEQ ID NO: 25 and the amino acid sequence of SEQ ID NO: 26 are also shown in Fig. 12.

### c)-iii) h#24A3-L3-type light chain:

A humanized #24A3 light chain designed by substituting the following amino acid residues (amino acid numbers) of the human chimeric #24A3 light chain represented by SEQ ID NO: 10 in the Sequence Listing with the following amino acid residues, respectively, was named "h#24A3-L3-type light chain": threonine (amino acid number 29) with aspartic acid, methionine (amino acid number 31) with leucine, serine (amino acid number 32) with alanine, isoleucine (amino acid number 33) with valine, valine (amino acid number 35) with leucine, aspartic acid (amino acid number 37) with glutamic acid, valine (amino acid number 39) with alanine, methionine (amino acid number 41) with isoleucine, threonine (amino acid number 60) with proline, asparagine (amino acid number 97) with serine, valine (amino acid number 98) with leucine, leucine (amino acid number 103) with valine, serine (amino acid number 120) with glutamine, isoleucine (amino acid number 122) with threonine, leucine (amino acid number 124) with valine, and alanine (amino acid number 129) with threonine.

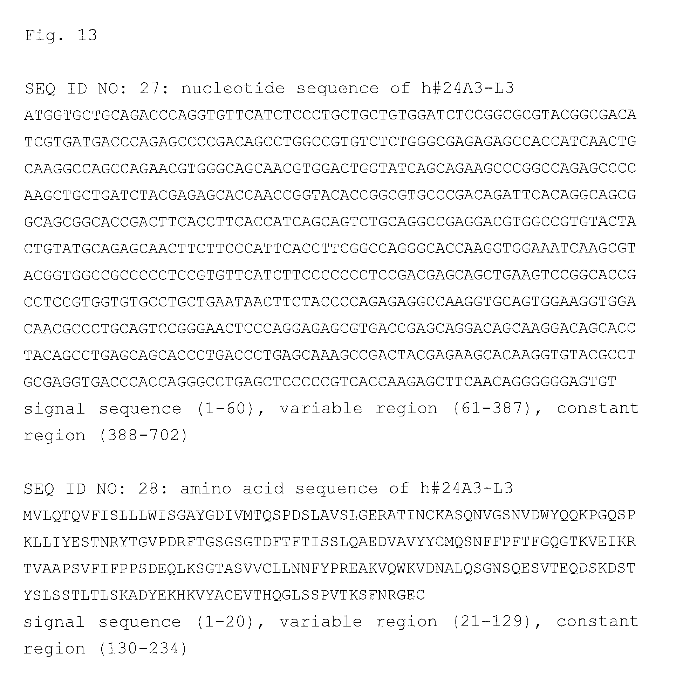

The amino acid sequence of the h#24A3-L3-type light chain is represented by SEQ ID NO: 28 in the Sequence Listing. The sequence comprising amino acid residues 1 to 20 of the amino acid sequence of SEQ ID NO: 28, the sequence comprising amino acid residues 21 to 129 thereof, and the sequence comprising amino acid residues 130 to 234 thereof correspond to the signal sequence, the light chain variable region, and the light chain constant region, respectively. A nucleotide sequence encoding the amino acid sequence of SEQ ID NO: 28 is represented by SEQ ID NO: 27 in the Sequence Listing. The sequence comprising nucleotides 1 to 60 of the nucleotide sequence of SEQ ID NO: 27, the sequence comprising nucleotides 61 to 387 thereof, and the sequence comprising nucleotides 388 to 702 thereof encode the signal sequence, the light chain variable region sequence, and the light chain constant region sequence, respectively. The nucleotide sequence of SEQ ID NO: 27 and the amino acid sequence of SEQ ID NO: 28 are also shown in Fig. 13.

### c)-iv) h#24A3-L4-type light chain:

A humanized #24A3 light chain designed by substituting the following amino acid residues (amino acid numbers) of the human chimeric #24A3 light chain represented by SEQ ID NO: 10 in the Sequence Listing with the following amino acid residues, respectively, was named "h#24A3-L4-type light chain": threonine (amino acid number 29) with aspartic acid, methionine (amino acid number 31) with leucine, serine (amino acid number 32) with alanine, isoleucine (amino acid number 33) with valine, valine (amino acid number 35) with leucine, aspartic acid (amino acid number 37) with glutamic acid, valine (amino acid number 39) with alanine, methionine (amino acid number 41) with isoleucine, asparagine (amino acid number 97) with serine, valine (amino acid number 98) with leucine, leucine (amino acid number 103) with valine, isoleucine (amino acid number 122) with threonine, leucine (amino acid number 124) with valine, and alanine (amino acid number 129) with threonine.

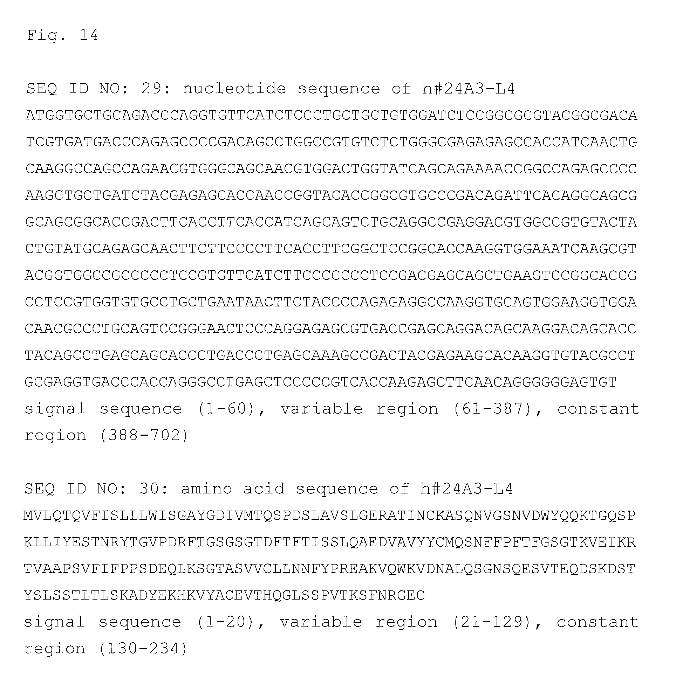

The amino acid sequence of the h#24A3-L4-type light chain is represented by SEQ ID NO: 30 in the Sequence Listing. The sequence comprising amino acid residues 1 to 20 of the amino acid sequence of SEQ ID NO: 30, the sequence comprising amino acid residues 21 to 129 thereof, and the sequence comprising amino acid residues 130 to 234 thereof correspond to the signal sequence, the light chain variable region, and the light chain constant region, respectively. A nucleotide sequence encoding the amino acid sequence of SEQ ID NO: 30 is represented by SEQ ID NO: 29 in the Sequence Listing. The sequence comprising nucleotides 1 to 60 of the nucleotide sequence of SEQ ID NO: 29, the sequence comprising nucleotides 61 to 387 thereof, and the sequence comprising nucleotides 388 to 702 thereof encode the signal sequence, the light chain variable region sequence, and the light chain constant region sequence, respectively. The nucleotide sequence of SEQ ID NO: 29 and the amino acid sequence of SEQ ID NO: 30 are also shown in Fig. 14.

### d) Humanization of h#24A3 heavy chain (2)

### d)-i) h#24A3-H2b-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H2b-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, threonine (amino acid number 43) with alanine, isoleucine (amino acid number 56) with valine, lysine (amino acid number 57) with arginine, arginine (amino acid number 59) with alanine, alanine (amino acid number 63) with glycine, isoleucine (amino acid number 67) with methionine, lysine (amino acid number 86) with arginine, alanine (amino acid number 87) with valine, leucine (amino acid number 89) with isoleucine, alanine (amino acid number 90) with threonine, valine (amino acid number 91) with alanine, serine (amino acid number 95) with threonine, phenylalanine (amino acid number 99) with tyrosine, glutamine (amino acid number 101) with glutamic acid, threonine (amino acid number 106) with arginine, proline (amino acid number 107) with serine, serine (amino acid number 122) with lysine, and proline (amino acid number 132) with glutamine.

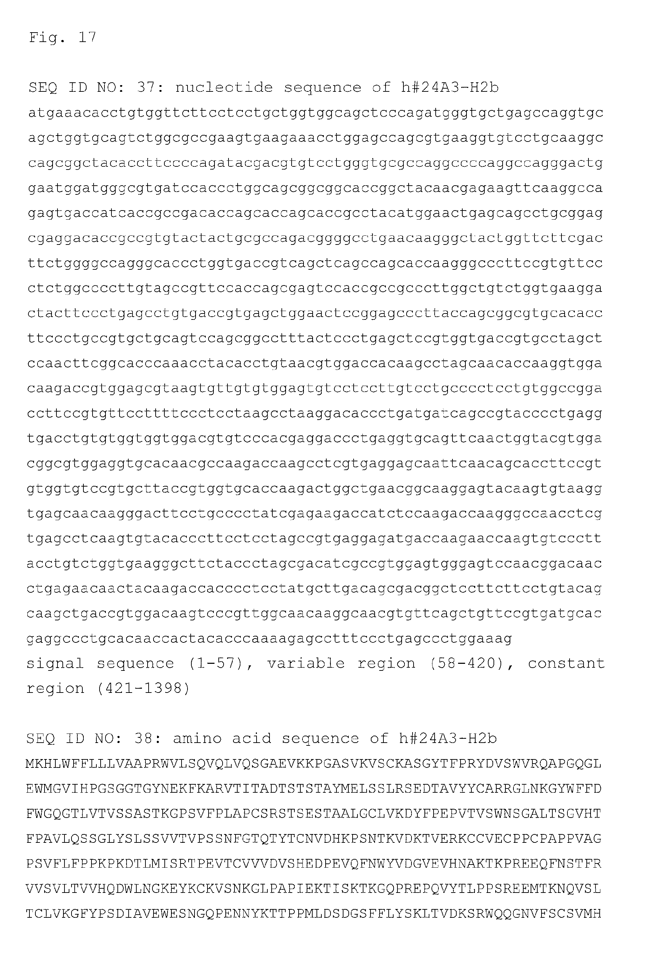

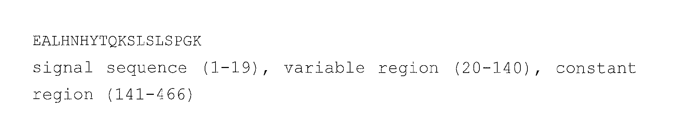

The amino acid sequence of the h#24A3-H2b-type heavy chain is represented by SEQ ID NO: 38 in the Sequence Listing. The sequence comprising amino acid residues 1 to 19 of the amino acid sequence of SEQ ID NO: 38, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 38 is represented by SEQ ID NO: 37 in the Sequence Listing. The sequence comprising nucleotides 1 to 57 of the nucleotide sequence of SEQ ID NO: 37, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 37 and the amino acid sequence of SEQ ID NO: 38 are also shown in Fig. 17.

### d)-ii) h#24A3-H2c-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H2c-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, threonine (amino acid number 43) with alanine, isoleucine (amino acid number 56) with valine, lysine (amino acid number 57) with arginine, arginine (amino acid number 59) with alanine, alanine (amino acid number 63) with glycine, lysine (amino acid number 86) with arginine, alanine (amino acid number 87) with valine, leucine (amino acid number 89) with isoleucine, alanine (amino acid number 90) with threonine, valine (amino acid number 91) with alanine, serine (amino acid number 95) with threonine, phenylalanine (amino acid number 99) with tyrosine, glutamine (amino acid number 101) with glutamic acid, threonine (amino acid number 106) with arginine, proline (amino acid number 107) with serine, serine (amino acid number 122) with lysine, and proline (amino acid number 132) with glutamine.

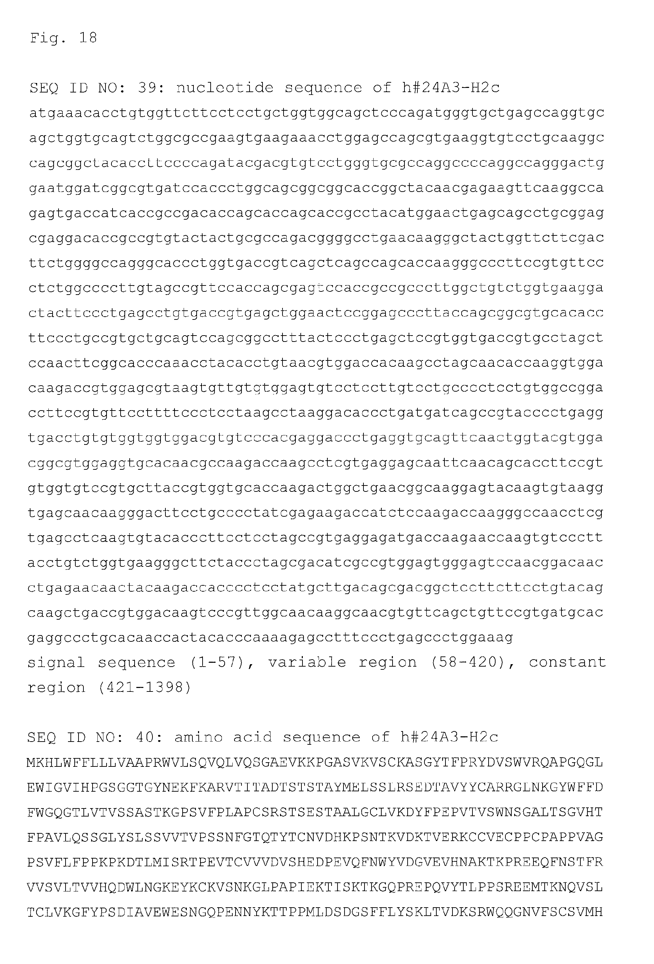

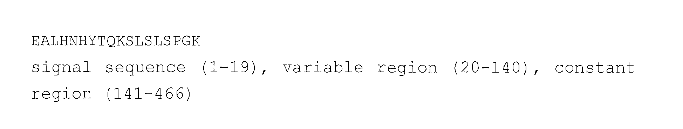

The amino acid sequence of the h#24A3-H2c-type heavy chain is represented by SEQ ID NO: 40 in the Sequence Listing. The sequence comprising amino acid residues 1 to 19 of the amino acid sequence of SEQ ID NO: 40, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 40 is represented by SEQ ID NO: 39 in the Sequence Listing. The sequence comprising nucleotides 1 to 57 of the nucleotide sequence of SEQ ID NO: 39, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 39 and the amino acid sequence of SEQ ID NO: 40 are also shown in Fig. 18.

### d)-iii) h#24A3-H2d-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H2d-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, threonine (amino acid number 43) with alanine, isoleucine (amino acid number 56) with valine, lysine (amino acid number 57) with arginine, arginine (amino acid number 59) with alanine, alanine (amino acid number 63) with glycine, isoleucine (amino acid number 67) with methionine, lysine (amino acid number 86) with arginine, leucine (amino acid number 89) with isoleucine, alanine (amino acid number 90) with threonine, valine (amino acid number 91) with alanine, serine (amino acid number 95) with threonine, phenylalanine (amino acid number 99) with tyrosine, glutamine (amino acid number 101) with glutamic acid, threonine (amino acid number 106) with arginine, proline (amino acid number 107) with serine, serine (amino acid number 122) with lysine, and proline (amino acid number 132) with glutamine.

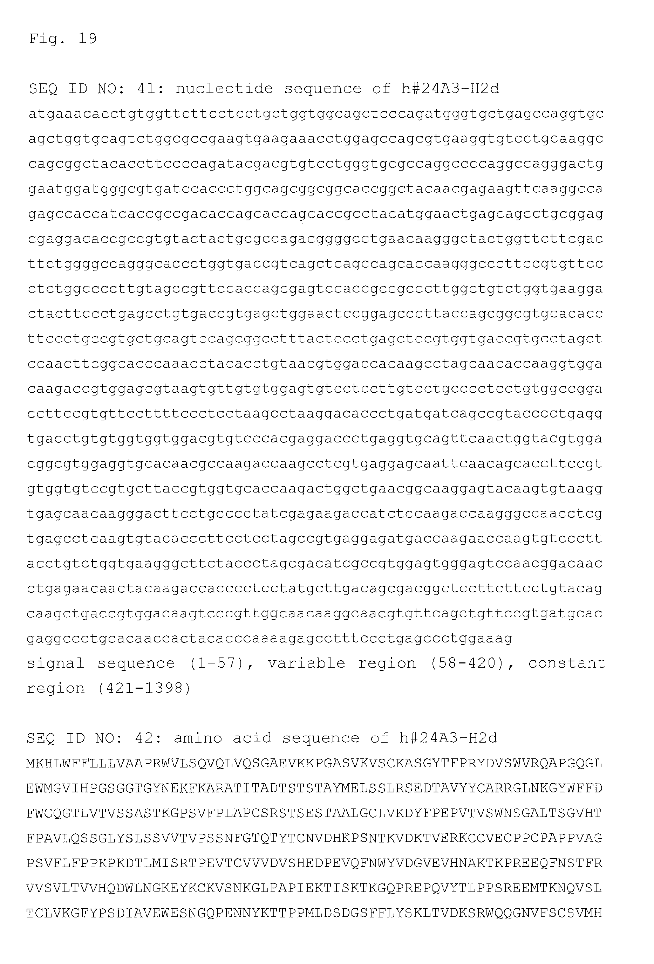

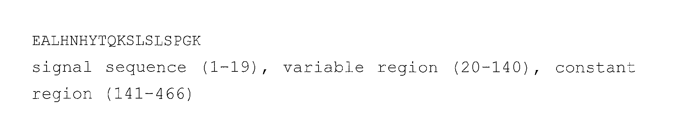

The amino acid sequence of the h#24A3-H2d-type heavy chain is represented by SEQ ID NO: 42 in the Sequence Listing. The sequence comprising amino acid residues 1 to 19 of the amino acid sequence of SEQ ID NO: 42, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 42 is represented by SEQ ID NO: 41 in the Sequence Listing. The sequence comprising nucleotides 1 to 57 of the nucleotide sequence of SEQ ID NO: 41, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 41 and the amino acid sequence of SEQ ID NO: 42 are also shown in Fig. 19.

### d)-iiii) h#24A3-H2e-type heavy chain:

A humanized #24A3 heavy chain designed by substituting the following amino acid residues (amino acid numbers) of the human chimeric #24A3 heavy chain represented by SEQ ID NO: 8 in the Sequence Listing with the following amino acid residues, respectively, was named "h#24A3-H2e-type heavy chain": glutamine (amino acid number 24) with valine, leucine (amino acid number 30) with valine, threonine (amino acid number 31) with lysine, serine (amino acid number 35) with alanine, isoleucine (amino acid number 39) with valine, threonine (amino acid number 43) with alanine, isoleucine (amino acid number 56) with valine, lysine (amino acid number 57) with arginine, arginine (amino acid number 59) with alanine, alanine (amino acid number 63) with glycine, isoleucine (amino acid number 67) with methionine, lysine (amino acid number 86) with arginine, alanine (amino acid number 87) with valine, leucine (amino acid number 89) with isoleucine, alanine (amino acid number 90) with threonine, serine (amino acid number 95) with threonine, phenylalanine (amino acid number 99) with tyrosine, glutamine (amino acid number 101) with glutamic acid, threonine (amino acid number 106) with arginine, proline (amino acid number 107) with serine, serine (amino acid number 122) with lysine, and proline (amino acid number 132) with glutamine.

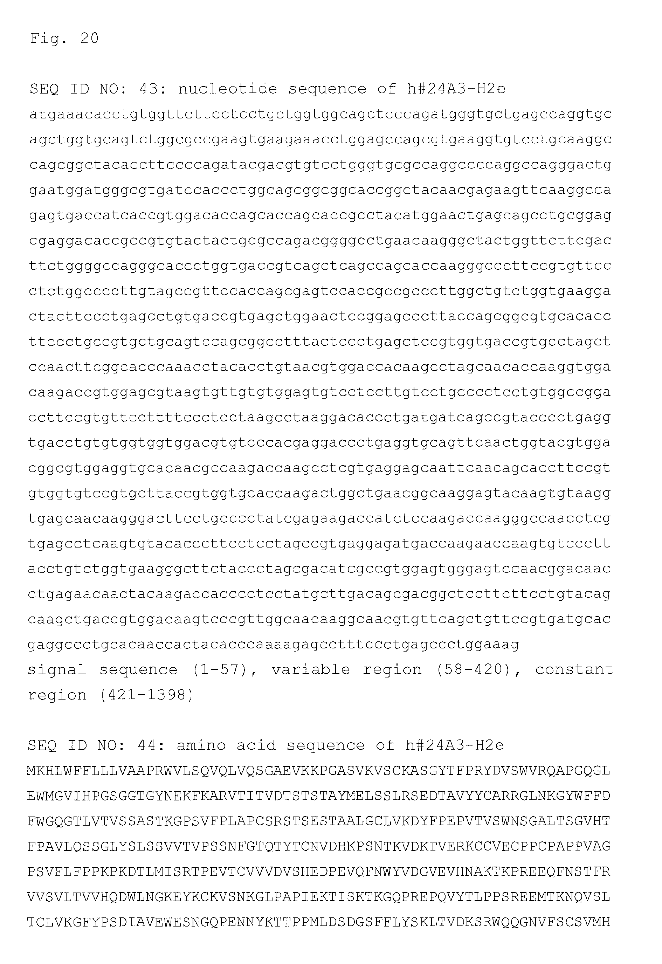

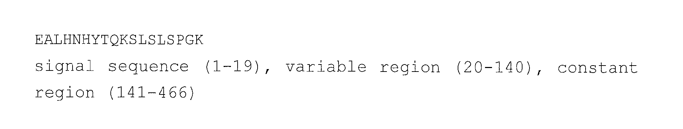

The amino acid sequence of the h#24A3-H2e-type heavy chain is represented by SEQ ID NO: 44 in the Sequence Listing. The sequence comprising amino acid residues 1 to 19 of the amino acid sequence of SEQ ID NO: 44, the sequence comprising amino acid residues 20 to 140 thereof, and the sequence comprising amino acid residues 141 to 466 thereof correspond to the signal sequence, the heavy chain variable region, and the heavy chain constant region, respectively. A nucleotide sequence encoding the amino acid sequence of SEQ ID NO: 44 is represented by SEQ ID NO: 43 in the Sequence Listing. The sequence comprising nucleotides 1 to 57 of the nucleotide sequence of SEQ ID NO: 43, the sequence comprising nucleotides 58 to 420 thereof, and the sequence comprising nucleotides 421 to 1398 thereof encode the signal sequence, the heavy chain variable region sequence, and the heavy chain constant region sequence, respectively. The nucleotide sequence of SEQ ID NO: 43 and the amino acid sequence of SEQ ID NO: 44 are also shown in Fig. 20.

### e) Humanization of h#24A3 light chain (2)

### e)-i) h#24A3-L2b-type light chain

A humanized #24A3 light chain designed by substituting the following amino acid residues (amino acid numbers) of the human chimeric #24A3 light chain represented by SEQ ID NO: 10 in the Sequence Listing with the following amino acid residues, respectively, was named "h#24A3-L2b-type light chain": threonine (amino acid number 29) with aspartic acid, methionine (amino acid number 31) with leucine, serine (amino acid number 32) with alanine, isoleucine (amino acid number 33) with valine, valine (amino acid number 35) with leucine, aspartic acid (amino acid number 37) with glutamic acid, valine (amino acid number 39) with alanine, methionine (amino acid number 41) with isoleucine, threonine (amino acid number 60) with proline, threonine (amino acid number 83) with serine, phenylalanine (amino acid number 93) with leucine, asparagine (amino acid number 97) with serine, valine (amino acid number 98) with leucine, leucine (amino acid number 103) with valine, serine (amino acid number 120) with glutamine, isoleucine (amino acid number 122) with threonine, leucine (amino acid number 124) with valine, and alanine (amino acid number 129) with threonine.

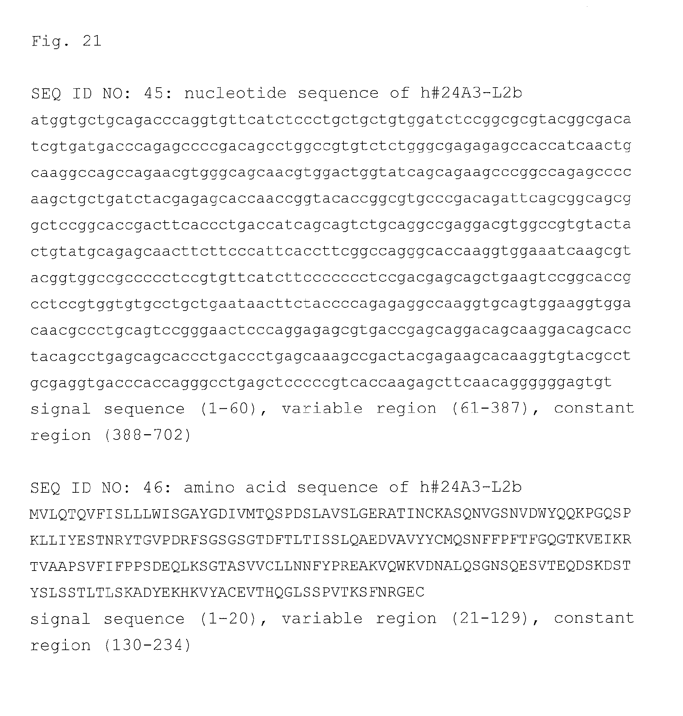

The amino acid sequence of the h#24A3-L2b-type light chain is represented by SEQ ID NO: 46 in the Sequence Listing. The sequence comprising amino acid residues 1 to 20 of the amino acid sequence of SEQ ID NO: 46, the sequence comprising amino acid residues 21 to 129 thereof, and the sequence comprising amino acid residues 130 to 234 thereof correspond to the signal sequence, the light chain variable region, and the light chain constant region, respectively. A nucleotide sequence encoding the amino acid sequence of SEQ ID NO: 46 is represented by SEQ ID NO: 45 in the Sequence Listing. The sequence comprising nucleotides 1 to 60 of the nucleotide sequence of SEQ ID NO: 45, the sequence comprising nucleotides 61 to 387 thereof, and the sequence comprising nucleotides 388 to 702 thereof encode the signal sequence, the light chain variable region sequence, and the light chain constant region sequence, respectively. The nucleotide sequence of SEQ ID NO: 45 and the amino acid sequence of SEQ ID NO: 46 are also shown in Fig. 21.

### e)-ii) h#24A3-L3b-type light chain

A humanized #24A3 light chain designed by substituting the following amino acid residues (amino acid numbers) of the human chimeric #24A3 light chain represented by SEQ ID NO: 10 in the Sequence Listing with the following amino acid residues, respectively, was named "h#24A3-L3b-type light chain": threonine (amino acid number 29) with aspartic acid, methionine (amino acid number 31) with leucine, serine (amino acid number 32) with alanine, isoleucine (amino acid number 33) with valine, valine (amino acid number 35) with leucine, aspartic acid (amino acid number 37) with glutamic acid, valine (amino acid number 39) with alanine, methionine (amino acid number 41) with isoleucine, threonine (amino acid number 60) with proline, phenylalanine (amino acid number 93) with leucine, asparagine (amino acid number 97) with serine, valine (amino acid number 98) with leucine, leucine (amino acid number 103) with valine, serine (amino acid number 120) with glutamine, isoleucine (amino acid number 122) with threonine, leucine (amino acid number 124) with valine, and alanine (amino acid number 129) with threonine.

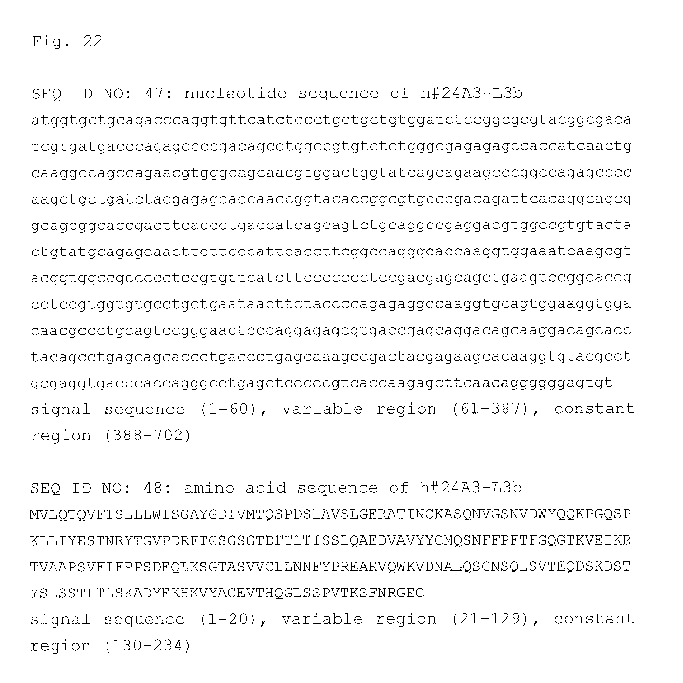

The amino acid sequence of the h#24A3-L3b-type light chain is represented by SEQ ID NO: 48 in the Sequence Listing. The sequence comprising amino acid residues 1 to 20 of the amino acid sequence of SEQ ID NO: 48, the sequence comprising amino acid residues 21 to 129 thereof, and the sequence comprising amino acid residues 130 to 234 thereof correspond to the signal sequence, the light chain variable region, and the light chain constant region, respectively. A nucleotide sequence encoding the amino acid sequence of SEQ ID NO: 48 is represented by SEQ ID NO: 47 in the Sequence Listing. The sequence comprising nucleotides 1 to 60 of the nucleotide sequence of SEQ ID NO: 47, the sequence comprising nucleotides 61 to 387 thereof, and the sequence comprising nucleotides 388 to 702 thereof encode the signal sequence, the light chain variable region sequence, and the light chain constant region sequence, respectively. The nucleotide sequence of SEQ ID NO: 47 and the amino acid sequence of SEQ ID NO: 48 are also shown in Fig. 22.

### Example 8

### Production of expression vector for humanized antibody of rat anti-human Siglec-15 monoclonal antibody #24A3

### 8-1) Construction of expression vector for heavy chain of humanized antibody of rat anti-human Siglec-15 monoclonal antibody #24A3

A DNA containing a gene encoding any of the heavy chain variable regions of the humanized antibodies of the rat anti-human Siglec-15 monoclonal antibody #24A3 described in Example 7-b) was synthesized (GENEART, Inc. Artificial Gene Synthesis Service) and inserted into the chimeric and humanized IgG2 type heavy chain expression vector for universal use (pCMA-G2) at the site cleaved with the restriction enzyme BlpI, whereby an expression vector for a heavy chain of a humanized antibody of the rat anti-human Siglec-15 monoclonal antibody #24A3 was constructed.

### 8-2) Construction of expression vector for light chain of humanized antibody of rat anti-human Siglec-15 monoclonal antibody #24A3

A DNA containing a gene encoding any of the light chain variable regions of the humanized antibodies of the rat anti-human Siglec-15 monoclonal antibody #24A3 described in Example 7-c) was synthesized (GENEART, Inc. Artificial Gene Synthesis Service) and inserted into the chimeric and humanized light chain expression vector for universal use (pCMA-LK) at the site cleaved with the restriction enzyme BsiWI, whereby an expression vector for a light chain of a humanized antibody of the rat anti-human Siglec-15 monoclonal antibody #24A3 was constructed.

### 8-3) Construction of expression vector for heavy chain of humanized antibody of rat anti-human Siglec-15 monoclonal antibody #24A3 (2)

### 8-3-1) Construction of #24A3-H2b-type heavy chain expression vector

By using the #24A3-H2-type heavy chain expression vector produced in 8-1) as a template, and also using the following primer set and KOD -Plus- Mutagenesis Kit (TOYOBO Co., Ltd.), a mutation was introduced, whereby a h#24A3-H2b-type heavy chain expression vector was constructed.
Primer set
5'-AAGGGCTACTGGTTCTTCGACTTCTGGGGCCAG-3' (H2b-F)
5'-GTTCAGGCCCCGTCTGGCGCAGTAGTACAC-3' (H2b-R)

### 8-3-2) Construction of h#24A3-H2c-type heavy chain expression vector

By using the h#24A3-H2b-type heavy chain expression vector produced in 8-3-1) as a template, and also using the following primer set and KOD -Plus- Mutagenesis Kit (TOYOBO Co., Ltd.), a mutation was introduced, whereby a h#24A3-H2c-type heavy chain expression vector was constructed.
Primer set
5'-GGCGTGATCCACCCTGGCAGCGGCGGCACC-3' (H2c-F)
5'-GATCCATTCCAGTCCCTGGCCTGGGGCCTGG-3' (H2c-R)

### 8-3-3) Construction of h#24A3-H2d-type heavy chain expression vector

By using the h#24A3-H2b-type heavy chain expression vector produced in 8-3-1) as a template, and also using the following primer set and KOD -Plus- Mutagenesis Kit (TOYOBO Co., Ltd.), a mutation was introduced, whereby a h#24A3-H2d-type heavy chain expression vector was constructed.
Primer set
5'-ACCATCACCGCCGACACCAGCACCAGCACC-3' (H2d-F)
5'-GGCTCTGGCCTTGAACTTCTCGTTGTAGCCGG-3' (H2d-R)

### 8-3-4) Construction of h#24A3-H2e-type heavy chain expression vector

By using the h#24A3-H2b-type heavy chain expression vector produced in 8-3-1) as a template, and also using the following primer set and KOD -Plus- Mutagenesis Kit (TOYOBO Co., Ltd.), a mutation was introduced, whereby a h#24A3-H2d-type heavy chain expression vector was constructed.
Primer set
5'-GACACCAGCACCAGCACCGCCTACATGGAAC-3' (H2e-F)
5'-CACGGTGATGGTCACTCTGGCCTTGAACTTCTC-3' (H2e-R)

### 8-4) Construction of expression vector for light chain of humanized antibody of rat anti-human Siglec-15 monoclonal antibody #24A3 (2)

### 8-4-1) Construction of h#24A3-L2b-type light chain expression vector

By using the h#24A3-L2-type light chain expression vector produced in 8-2) as a template, and also using the following primer set and KOD -Plus- Mutagenesis Kit (TOYOBO Co., Ltd.), a mutation was introduced, whereby a h#24A3-L2b-type light chain expression vector was constructed.
Primer set
5'-CTGACCATCAGCAGTCTGCAGGCCGAGGACGTG-3' (L2b-F)
5'-GGTGAAGTCGGTGCCGGAGCCGCTGCCGCTG-3' (L2b-R)

### 8-4-2) Construction of h#24A3-L3b-type light chain expression vector

By using the h#24A3-L3-type light chain expression vector produced in 8-2) as a template, and also using the following primer set and KOD -Plus- Mutagenesis Kit (TOYOBO Co., Ltd.), a mutation was introduced, whereby a h#24A3-L3b-type light chain expression vector was constructed.
Primer set
5'-CTGACCATCAGCAGTCTGCAGGCCGAGGACGTG-3' (L2b-F)
5'-GGTGAAGTCGGTGCCGCTGCCGCTGCCTGT-3' (L3b-R)

### Example 9

### Preparation of humanized antibody of rat anti-human Siglec-15 monoclonal antibody #24A3

### 9-1) Production of humanized antibody of rat anti-human Siglec-15 monoclonal antibody #24A3

FreeStyle 293F cells (Invitrogen Corporation) can be subcultured and cultured according to the protocol. 1.2 x 10⁹ cells of FreeStyle 293F cells (Invitrogen Corporation) in logarithmic growth phase were seeded in a 3-L Fernbach Erlenmeyer Flask (Corning Incorporated) and prepared at 1.0 x 10⁶ cells/ml by dilution with FreeStyle 293 expression medium (Invitrogen Corporation), and then shaking culture was performed at 90 rpm for 1 hour at 37°C in an 8% CO₂ incubator. 3.6 mg of polyethyleneimine (Polyscience #24765) was dissolved in 20 ml of Opti-Pro SFM medium (Invitrogen Corporation). Subsequently, the heavy chain expression vector (0.4 mg) and the light chain expression vector (0.8 mg) prepared using NucleoBond Xtra (TaKaRa Bio, Inc.) were suspended in 20 ml of Opti-Pro SFM medium (Invitrogen Corporation). Then, 20 ml of the obtained expression vectors/Opti-Pro SFM mixture was added to 20 ml of the obtainedpolyethyleneimine/Opti-Pro SFM mixture, and the resulting mixture was gently stirred and then left for 5 minutes. Thereafter, the mixture was added to the FreeStyle 293F cells, and shaking culture was performed at 90 rpm for 7 days at 37°C in an 8% CO₂ incubator. The resulting culture supernatant was filtered through a disposable capsule filter (Advantec #CCS-045-E1H).

By combining any of the expression vectors for a heavy chain of a humanized antibody of the rat anti-human Siglec-15 monoclonal antibody #24A3 produced in Examples 8-1 and 8-3 with any of the expression vectors for a light chain of a humanized antibody of the rat anti-human Siglec-15 monoclonal antibody #24A3 produced in Examples 8-2 and 8-4, a humanized antibody of the rat anti-human Siglec-15 monoclonal antibody #24A3 was obtained.

### 9-2) Purification of humanized antibody of rat anti-human Siglec-15 monoclonal antibody #24A3

The antibody was purified from the culture supernatant obtained in the above 9-1) by a two-step process including rProtein A affinity chromatography (at 4 to 6°C) and ceramic hydroxyapatite (at room temperature). A buffer replacement step after the purification by rProtein A affinity chromatography and after the purification by ceramic hydroxyapatite was performed at 4 to 6°C. First, the culture supernatant was applied to MabSelect SuRe (manufactured by GE Healthcare Bio-Sciences Ltd., HiTrap column) equilibrated with PBS. After all culture solution was poured into the column, the column was washed with PBS in an amount twice or more the volume of the column. Subsequently, elution was performed with a 2 M arginine hydrochloride solution (pH 4.0), and a fraction containing the antibody was collected. After the collected fraction was subjected to buffer replacement with PBS by dialysis (Thermo Scientific, Inc., Slide-A-Lyzer Dialysis Cassette), an antibody solution prepared by 5-fold dilution with a buffer containing 5 mM sodium phosphate and 50 mM MES at pH 7.0 was applied to a ceramic hydroxyapatite column (Bio-Rad Laboratories, Inc. (Japan), Bio-Scale CHT Type-I hydroxyapatite column) equilibrated with a buffer containing 5 mM NaPi, 50 mM MES, and 30 mM NaCl at pH 7.0. Then, linear concentration gradient elution with sodium chloride was performed, and a fraction containing the antibody was collected. The collected fraction was subjected to buffer replacement with HBSor (25 mM histidine/5% sorbitol, pH 6. 0) by dialysis (Thermo Scientific, Inc., Slide-A-Lyzer Dialysis Cassette). Finally, the resulting solution was concentrated using Centrifugal UF Filter Device VIVASPIN 20 (fractional molecular weight UF: 10 K, Sartorius Co., Ltd., at 4°C), the concentration of IgG was adjusted to 20 mg/ml, and the thus obtained solution was used as a purified sample.

Incidentally, in this specification, for example, the antibody having the h#24A3-H1 heavy chain and the h#24A3-L1 light chain is referred to as "h#24A3-H1/L1" or "h#24A3-H1/L1 antibody". Specific examples of the antibody prepared according to the method of the above 9-1) and 9-2) include h#24A3-H1/L1, h#24A3-H1/L2, h#24A3-H1/L3, h#24A3-H2/L1, h#24A3-H2/L2, h#24A3-H2/L3, h#24A3-H3/L1, h#24A3-H3/L2, h#24A3-H3/L3, h#24A3-H4/L1, h#24A3-H4/L2, h#24A3-H4/L3, h#24A3-H5/L1, h#24A3-H5/L2, h#24A3-H5/L3, h#24A3-H6/L4, h#24A3-H2b/L1, h#24A3-H2b/L2b, h#24A3-H2b/L3b, h#24A3-H2c/L1, h#24A3-H2c/L2b, h#24A3-H2c/L3b, h#24A3-H2d/L1, h#24A3-H2d/L2b, h#24A3-H2d/L3b, h#24A3-H2e/L1, h#24A3-H2e/L2b, and h#24A3-H2e/L3b.

### Example 10

Evaluation of binding activity of chimeric antibody and humanized antibody of rat anti-human Siglec-15 monoclonal antibody #24A3 to mouse Siglec-15 protein

### 10-1) Evaluation of binding activity of chimeric antibody

The dissociation constant between the human chimeric #24A3 antibody and the human Siglec-15 V-set domain was measured using Biacore T200 (GE Healthcare Bio-Sciences Ltd.) by immobilizing the antibody as a ligand and using the antigen as an analyte. Incidentally, a human chimeric #32A1 antibody was used as a control antibody. The human chimeric #32A1 antibody is described in WO 10/117011 with respect to the preparation method thereof and information on the heavy and light chain sequences thereof. The human chimeric #24A3 antibody or human chimeric #32A1 antibody was bound to a sensor chip CM5 (GE Healthcare Bio-Sciences Ltd.) at about 50 RU by an amine coupling method via an anti-human IgG antibody (GE Healthcare Bio-Sciences Ltd.) immobilized thereon. As a running buffer, HBS-EP+ (10 mM HEPES pH 7.4, 0.15 M NaCl, 3 mM EDTA, 0.05% surfactant P20) was used. On the chip having the antibody bound thereto, a dilution series of an antigen solution (0.003 to 2 nM) was added at a flow rate of 90 µL/min for 233 seconds, and subsequently, the dissociation phase was monitored for 3600 seconds. As a regeneration solution, 3 M MgCl₂ was added at a flow rate of 10 µL/min for 30 seconds. In the analysis of data, a 1:1 binding model of analysis software (Biacore T200 Evaluation software, version 1.0) was used, and an association rate constant (kon), a dissociation rate constant (koff), and a dissociation constant (KD; KD = koff/kon) were calculated. According to the results, the human chimeric #24A3 antibody had a KD value of 1.7E-11 [M], and the human chimeric #32A1 antibody had a KD value of 1. 4E-10 [M]. The human chimeric #24A3 antibody had affinity comparable to, or higher than, that of the rat #24A3 antibody. Further, the human chimeric #32A1 antibody had affinity comparable to that of the rat #32A1 antibody.

### 10-2) Evaluation of binding activity of humanized antibody

In the same manner as in the above 10-1), the binding activity of the humanized antibodies of the rat anti-human Siglec-15 monoclonal antibody #24A3 to the mouse Siglec-15 protein was evaluated. The results are shown in Table 1.

**(Table 1)**

| Antibody | KD [M] |
|---|---|
| h#24A3-H2/L1 | 3.1E-11 |
| h#24A3-H2/L2 | 2.8E-11 |
| h#24A3-H2/L3 | 2.7E-11 |
| h#24A3-H2b/L1 | 9.8E-12 |
| h#24A3-H2b/L2b | 8.5E-12 |
| h#24A3-H2b/L3b | 7.7E-12 |
| h#24A3-H2c/L1 | 1.0E-11 |
| h#24A3-H2c/L2b | 1.3E-11 |
| h#24A3-H2c/L3b | 1.5E-11 |
| h#24A3-H2d/L1 | 4.5E-12 |
| h#24A3-H2d/L2b | 1.0E-11 |
| h#24A3-H2d/L3b | 7.5E-12 |
| h#24A3-H2e/L1 | 6.9E-12 |
| h#24A3-H2e/L2b | 1.8E-11 |
| h#24A3-H2e/L3b | 1.6E-11 |

The humanized #24A3 antibodies had affinity comparable to, or higher than, that of the human chimeric #24A3 antibody.

### Example 11

### Effect of chimeric antibody and humanized antibody of rat anti-human Siglec-15 monoclonal antibody #24A3 on differentiation of normal human osteoclasts

### 11-1) Effect of chimeric antibody on differentiation of normal human osteoclasts

Tartrate-resistant acid phosphatase (TRAP) is a marker whose expression increases with the differentiation of osteoclasts and is considered to be secreted from mature osteoclasts. Therefore, by measuring the activity of TRAP in a culture supernatant of osteoclasts, the effect of a test substance on differentiation of osteoclasts can be evaluated in vitro.

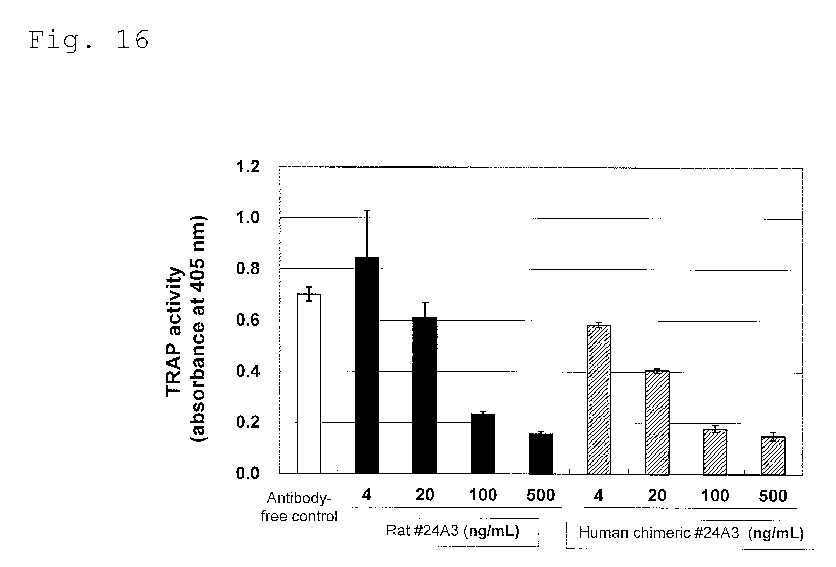

Normal human osteoclast precursor cells (Normal Human Natural Osteoclast Precursor Cells, purchased from Sanko Junyaku Co., Ltd., Cat. No. 2T-110) were seeded in a 96-well plate at 1 x 10⁴ cells/well according to the protocol attached to the cells. Incidentally, as the medium, a basal medium for osteoclast precursor cells (OPBM, purchased from Sanko Junyaku Co., Ltd., Cat. No. PT-8201) supplemented with an OPGM supplement set (purchased from Sanko Junyaku Co., Ltd., Cat. No. PT-9501) containing fetal bovine serum (final concentration: 10%), human RANKL (final concentration: 68.4 ng/ml), human M-CSF (final concentration: 33 ng/ml), and the like was used. To this culture supernatant, each of the rat #24A3 antibody obtained in Example 2 and the human chimeric #24A3 antibody prepared in Example 6 was added to give a final concentration of 4, 20, 100, or 500 ng/ml, and the cells were cultured for 5 days in a CO₂ incubator. A 75-µL aliquot of the culture supernatant was collected, and 75 µL of a substrate solution (a 0.2 M sodium acetate buffer (pH 5.0) containing 11 mg/ml p-nitrophenyl phosphate and 100 mM sodium tartrate) was added thereto, and the resulting mixture was incubated at room temperature for 30 minutes. Then, 50 µL of a 2 N sodium hydroxide solution was added thereto to stop the enzymatic reaction, and the absorbance at 405 nm was measured and used as the index of TRAP activity (Fig. 16). As a result, the TRAP activity was inhibited in a concentration-dependent manner by the human chimeric #24A3 antibody within the range from 4 ng/ml to 500 ng/ml, and thus, it was confirmed that the human chimeric #24A3 antibody inhibits the differentiation of human osteoclasts. Further, the inhibitory activity of this human chimeric antibody was comparable to, or higher than, that of the original rat #24A3 antibody, and therefore, it was considered that a decrease in the activity by chimerization with a human source did not occur.

### 11-2) Effect of humanized antibody on differentiation of normal human osteoclasts

The effect of the chimeric antibody and the humanized antibody of the rat anti-human Siglec-15 monoclonal antibody #24A3 on differentiation of normal human osteoclasts can be examined by the method described in the above 11-1).

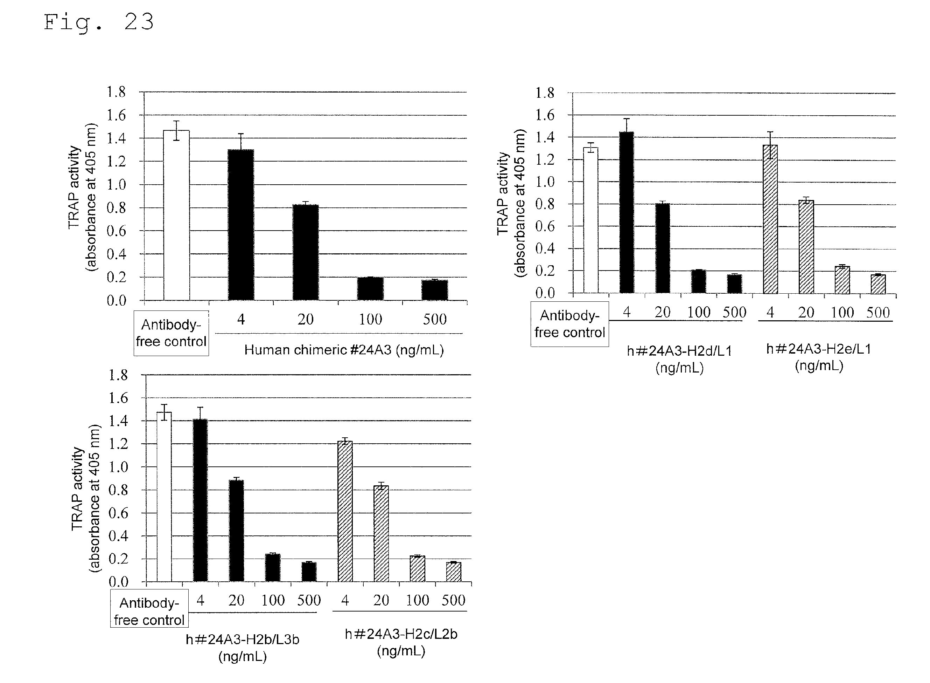

Normal human osteoclast precursor cells were seeded in a 96-well plate at 1 x 10⁴ cells/well. Incidentally, human RANKL in the medium was prepared to give a final concentration of 66 ng/ml. In this culture supernatant, each of the humanized #24A3 antibodies (h#24A3-H2d/L1, h#24A3-H2e/L1, h#24A3-H2b/L3b, and h#24A3-H2c/L2b) obtained in Example 9 and the human chimeric #24A3 antibody prepared in Example 6 was added to give a final concentration of 4, 20, 100, or 500 ng/ml, and the cells were cultured for 6 days in a CO₂ incubator. A 75-µL aliquot of the culture supernatant was collected, and 75 µL of a substrate solution (a 0.2 M sodium acetate buffer (pH 5.0) containing 11 mg/ml p-nitrophenyl phosphate and 100 mM sodium tartrate) was added thereto, and the resulting mixture was incubated at room temperature for 20 minutes . Then, 50 µL of a 2 N sodium hydroxide solution was added thereto to stop the enzymatic reaction, and the absorbance at 405 nm was measured and used as the index of TRAP activity (Fig. 23). As a result, the TRAP activity was inhibited in a concentration-dependent manner by h#24A3-H2d/L1, h#24A3-H2e/L1, and h#24A3-H2b/L3b within the range from 20 ng/ml to 500 ng/ml, and also by h#24A3-H2c/L2b within the range from 4 ng/ml to 500 ng/ml. Further, the inhibitory activity of these antibodies was almost comparable to that of the human chimeric #24A3 antibody, and therefore, it was considered that a decrease in the activity by humanization hardly occurred. From the above results, it was indicated that the humanized #24A3 antibody inhibits the differentiation of human osteoclasts.

### Example 12

### Effect of humanized antibody of rat anti-human Siglec-15 monoclonal antibody #24A3 on bone resorption activity of normal human osteoclasts (in vitro evaluation of biological activity)

It is known that osteoclasts release a protease such as cathepsin K and degrade type I collagen which is a constitutional component of bone tissue. OsteoLyse Assay Kit (manufactured by Lonza, Inc., Cat. No. PA-1500) provides a 96-well plate coated with europium-conjugated human collagen (96-well OsteoLyse cell culture plate), and it is possible to evaluate the bone resorption activity of osteoclasts in vitro by measuring the amount of fluorescent collagen fragments released in the supernatant when osteoclasts are cultured in the plate. The inhibitory effect of the humanized antibody of the rat anti-human Siglec-15 monoclonal antibody #24A3 on the bone resorption activity of normal human osteoclasts can be evaluated by the method shown below.

Normal human osteoclast precursor cells (Normal Human Natural Osteoclast Precursor Cells, purchased from Sanko Junyaku Co., Ltd., Cat. No. 2T-110) are seeded in a 96-well OsteoLyse cell culture plate at 1 x 10⁴ cells/well according to the protocol attached to the cells. Incidentally, as the medium, a basal medium for osteoclast precursor cells (OPBM, purchased from Sanko Junyaku Co., Ltd., Cat. No. PT-8201) supplemented with an OPGM supplement set (purchased from Sanko Junyaku Co., Ltd., Cat. No. PT-9501) containing fetal bovine serum (final concentration: 10%), human RANKL (final concentration: 63.8 ng/ml), human M-CSF (final concentration: 33 ng/ml), and the like is used. To this culture supernatant, the humanized antibody of the rat anti-human Siglec-15 monoclonal antibody #24A3 prepared in Example 10 is added to give a final concentration of 0.8 to 500 ng/ml, and the cells are cultured for 3 to 7 days in a CO₂ incubator. A 10-µL aliquot of the culture supernatant is collected, and 200 µL of Fluorophore Releasing Reagent included in the OsteoLyse Assay Kit is added thereto, and fluorescence intensity is measured (Excitation: 340 nm, Emission: 615 nm) using a fluorescence plate reader (ARVO MX, manufactured by Perkin Elmer Inc.), whereby the amount of free fluorescent collagen fragments released in the culture supernatant is determined.

### Example 13

### Biological evaluation of humanized antibody of rat anti-human Siglec-15 monoclonal antibody #24A3 using ovariectomized rats

The inhibitory effect of the humanized antibody obtained in Example 9 on decrease in bone mineral density and bone resorption activity in ovariectomized rats can be evaluated by the method described below.

### a) Protocol of animal experiment

The ovaries on both sides are removed from female F344 rats at the age of 12 weeks (obtained from Charles River Laboratories Japan, Inc.), and the rats are divided into a vehicle administration group and a humanized antibody of the rat anti-human Siglec-15 monoclonal antibody #24A3 administration group. Further, one group is also prepared as a sham operation group. In the antibody administration group, the antibody is subcutaneously administered singly or repeatedly from the next day of the operation at a dose of 0.1 to 30 mg/kg. After 4 weeks from the initiation of administration, urine is collected for 24 hours under fasting conditions, and the urine samples are stored at -80°C until measurement. After completion of the urine collection, the rats are euthanized, and the lumbar spine is excised from each rat.

### b) Measurement of lumbar spine bone mineral density

Soft tissues adhered to the excised lumbar spine are removed, and the 4th to 6th lumbar vertebrae are extracted. The bone mineral density is measured using a bone densitometer (DCS-600EX, manufactured by Aloka Co., Ltd.) and compared with those of the vehicle administration group and the sham operation group, whereby the effect of the humanized antibody is determined.

### c) Measurement of urinary deoxypyridinoline excretion

A variety of type I collagen crosslinked metabolites sharply reflect bone metabolic turnover, particularly bone resorption. Above all, deoxypyridinoline is localized mainly in bone collagen, and therefore is considered to be highly reliable as an index of bone resorption.

The cryopreserved urine sample is thawed, and insoluble matter is precipitated by a centrifugal operation, whereby a supernatant is obtained. The amount of deoxypyridinoline contained in this supernatant is measured using Osteolinks "DPD" (manufactured by DS Pharma Biomedical Co., Ltd.). Further, the content of creatinine in the supernatant is also measured, and the amount of deoxypyridinoline corrected for creatinine is calculated. The calculated amount of deoxypyridinoline is compared with those of the vehicle administration group and the sham operation group, whereby the effect of the humanized antibody is determined.

### Example 14

### Determination of thermal stability of humanized antibody of rat anti-human Siglec-15 monoclonal antibody #24A3

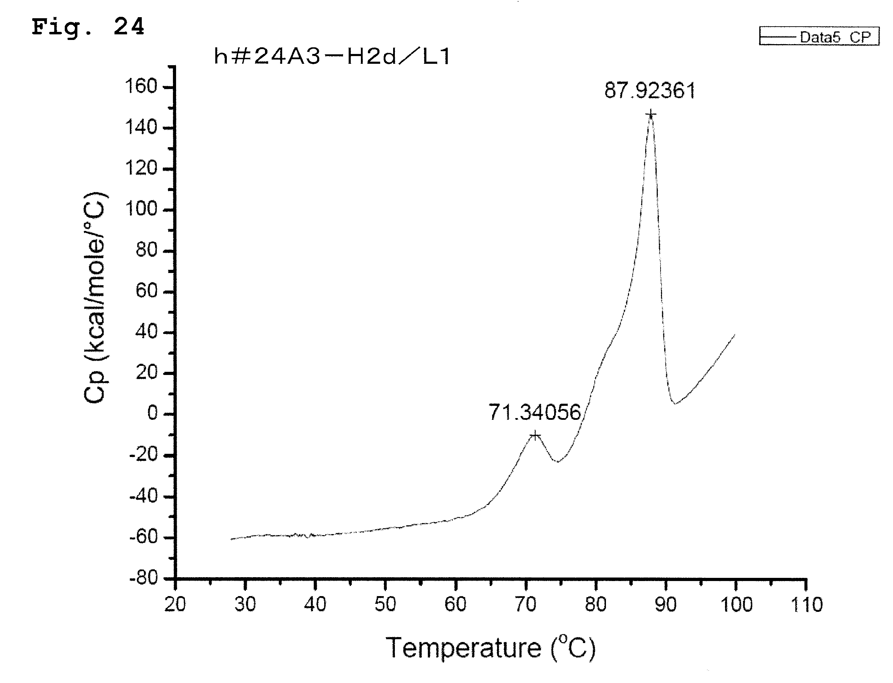

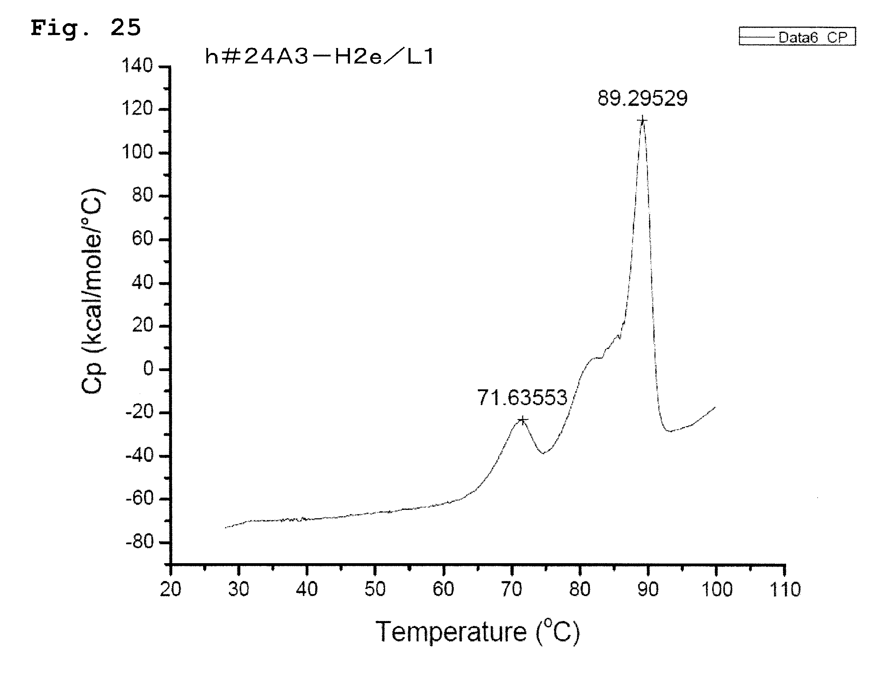

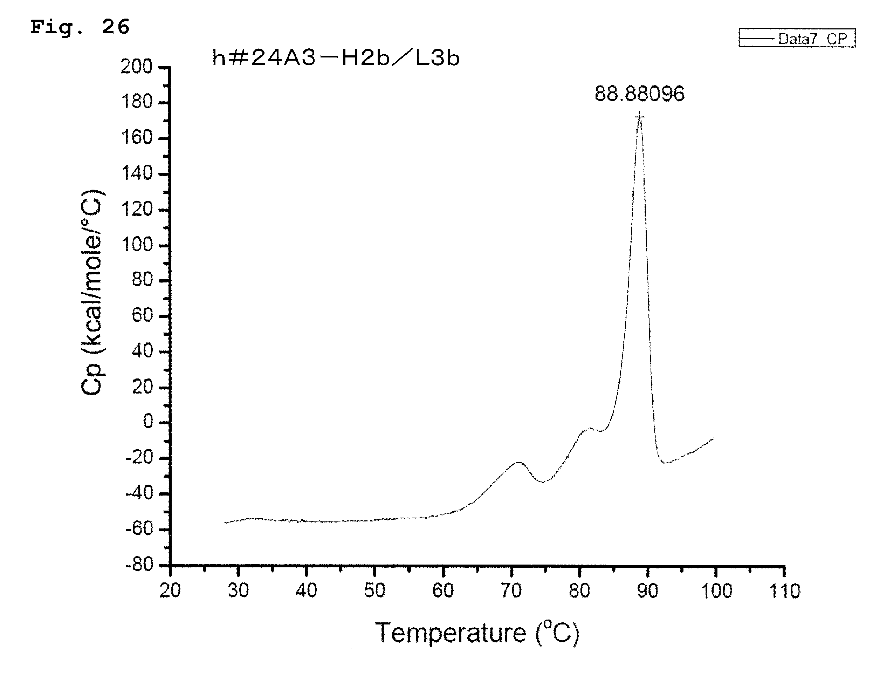

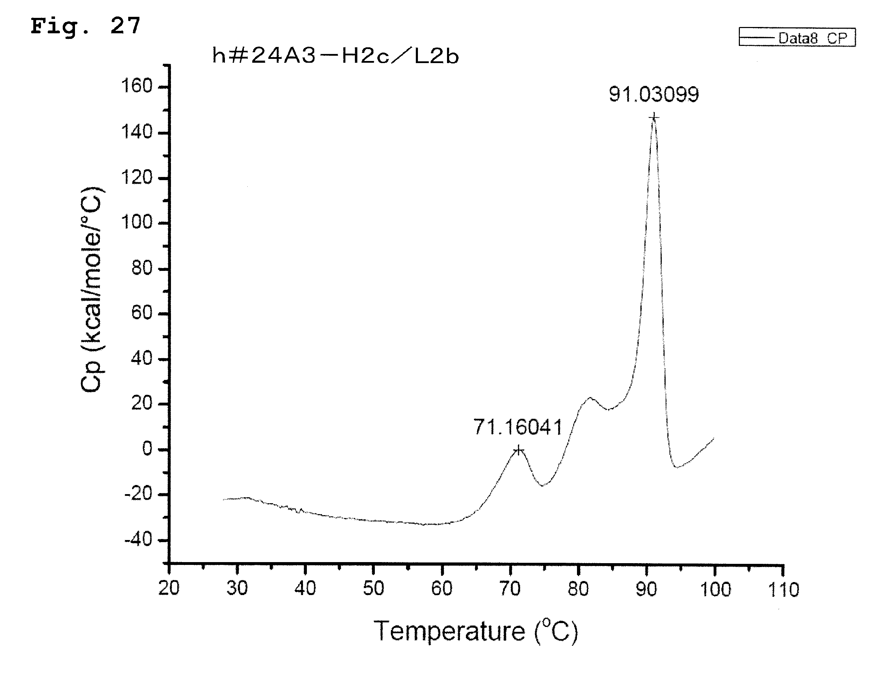

The determination of thermal stability was performed using differential scanning calorimetry (DSC). A sample was dissolved in HBSor buffer (prepared to contain 25 mM histidine and 5% sorbitol at pH 6.0) at a concentration of 0.5 mg/ml, and a 400 µl aliquot thereof was used as a sample solution for the DSC measurement. The conditions for the DSC measurement were set as follows. That is, the initial temperature was set to 20°C, the final temperature was set to 100°C, the temperature increase rate was set to 200°C/hour, the filter time was set to 2 seconds, and the feed back mode was set to low. As a reference solution, HBSor was used. As an instrument for the DSC measurement for all samples, VP-Capillary DSC platform manufactured by GE Healthcare Bio-Sciences Ltd. (USA) was used. The baseline (a scanning curve obtained by filling a sample cell with the reference solution) was subtracted from a scanning curve obtained for each sample solution, whereby baseline correction was performed. Subsequently, by using the molar concentration calculated from the molecular weight of each sample, the concentration was calibrated. Fig. 24 shows a thermogram for the h#24A3-H2d/L1 antibody, Fig. 25 shows a thermogram for the h#24A3-H2e/L1 antibody, Fig. 26 shows a thermogram for the h#24A3-H2b/L3b antibody, and Fig. 27 shows a thermogram for the h#24A3-H2c/L2b antibody. When the temperature showing a peak top with respect to the highest peak in each thermogram was taken as a thermal denaturation midpoint temperature (Tm), the Tm value of the h#24A3-H2d/L1 antibody was 87.9°C, the Tm value of the h#24A3-H2e/L1 antibody was 89.3°C, the Tm value of the h#24A3-H2b/L3b antibody was 88.9°C, and the Tm value of the h#24A3-H2c/L2b antibody was 91.0°C.

### Industrial Applicability

The chimeric or humanized anti-Siglec-15 antibody of the invention has an ability to inhibit osteoclast differentiation or bone resorption activity, and a pharmaceutical composition containing the anti-Siglec-15 antibody can be a therapeutic or prophylactic agent for a disease of abnormal bone metabolism.

### Sequence Listing Free Text

SEQ ID NO: 1: nucleotide sequence of cDNA encoding #24A3 heavy chain variable region
SEQ ID NO: 2: amino acid sequence of #24A3 heavy chain variable region
SEQ ID NO: 3: nucleotide sequence of cDNA encoding #24A3 light chain variable region
SEQ ID NO: 4: amino acid sequence of #24A3 light chain variable region
SEQ ID NO: 5: nucleotide sequence encoding human κ chain secretory signal and human κ chain constant region
SEQ ID NO: 6: nucleotide sequence encoding human heavy chain secretory signal and human IgG2 constant region
SEQ ID NO: 7 : nucleotide sequence of human chimeric #24A3 antibody heavy chain
SEQ ID NO: 8: amino acid sequence of human chimeric #24A3 antibody heavy chain
SEQ ID NO: 9: nucleotide sequence of human chimeric #24A3 antibody light chain
SEQ ID NO: 10: amino acid sequence of human chimeric #24A3 antibody light chain
SEQ ID NO: 11: nucleotide sequence of h#24A3-H1
SEQ ID NO: 12: amino acid sequence of h#24A3-H1
SEQ ID NO: 13: nucleotide sequence of h#24A3-H2
SEQ ID NO: 14: amino acid sequence of h#24A3-H2
SEQ ID NO: 15: nucleotide sequence of h#24A3-H3
SEQ ID NO: 16: amino acid sequence of h#24A3-H3
SEQ ID NO: 17: nucleotide sequence of h#24A3-H4
SEQ ID NO: 18: amino acid sequence of h#24A3-H4
SEQ ID NO: 19: nucleotide sequence of h#24A3-H5
SEQ ID NO: 20: amino acid sequence of h#24A3-H5
SEQ ID NO: 21: nucleotide sequence of h#24A3-H6
SEQ ID NO: 22: amino acid sequence of h#24A3-H6
SEQ ID NO: 23: nucleotide sequence of h#24A3-L1
SEQ ID NO: 24: amino acid sequence of h#24A3-L1
SEQ ID NO: 25: nucleotide sequence of h#24A3-L2
SEQ ID NO: 26: amino acid sequence of h#24A3-L2
SEQ ID NO: 27: nucleotide sequence of h#24A3-L3
SEQ ID NO: 28: amino acid sequence of h#24A3-L3
SEQ ID NO: 29: nucleotide sequence of h#24A3-L4
SEQ ID NO: 30: amino acid sequence of h#24A3-L4
SEQ ID NO: 31: amino acid sequence of CDRH1 of #24A3 antibody
SEQ ID NO: 32: amino acid sequence of CDRH2 of #24A3 antibody
SEQ ID NO: 33: amino acid sequence of CDRH3 of #24A3 antibody
SEQ ID NO: 34: amino acid sequence of CDRL1 of #24A3 antibody
SEQ ID NO: 35: amino acid sequence of CDRL2 of #24A3 antibody
SEQ ID NO: 36: amino acid sequence of CDRL3 of #24A3 antibody
SEQ ID NO: 37: nucleotide sequence of h#24A3-H2b
SEQ ID NO: 38: amino acid sequence of h#24A3-H2b
SEQ ID NO: 39: nucleotide sequence of h#24A3-H2c
SEQ ID NO: 40: amino acid sequence of h#24A3-H2c
SEQ ID NO: 41: nucleotide sequence of h#24A3-H2d
SEQ ID NO: 42: amino acid sequence of h#24A3-H2d
SEQ ID NO: 43: nucleotide sequence of h#24A3-H2e
SEQ ID NO: 44: amino acid sequence of h#24A3-H2e
SEQ ID NO: 45: nucleotide sequence of h#24A3-L2b
SEQ ID NO: 46: amino acid sequence of h#24A3-L2b
SEQ ID NO: 47: nucleotide sequence of h#24A3-L3b
SEQ ID NO: 48: amino acid sequence of h#24A3-L3b
SEQ ID NO: 49: amino acid sequence of mutated CDRH3 of #24A3 antibody

## Claims

1. An antibody or an antigen binding fragment of the antibody, **characterized in that**:
the heavy chain sequence contains a variable region having CDRH1, CDRH2, and CDRH3, and the CDRH1 comprises an amino acid sequence represented by SEQ ID NO: 31, the CDRH2 comprises an amino acid sequence represented by SEQ ID NO: 32, and the CDRH3 comprises either an amino acid sequence represented by SEQ ID NO: 33 or an amino acid sequence including substitution of one to three amino acids therein; and
the light chain sequence contains a variable region having CDRL1, CDRL2, and CDRL3, and the CDRL1 comprises an amino acid sequence represented by SEQ ID NO: 34, the CDRL2 comprises an amino acid sequence represented by SEQ ID NO: 35, and the CDRL3 comprises an amino acid sequence represented by SEQ ID NO: 36.

2. An antibody or an antigen binding fragment of the antibody, **characterized in that**:
the heavy chain sequence contains a variable region having CDRH1, CDRH2, and CDRH3, and the CDRH1 comprises an amino acid sequence represented by SEQ ID NO: 31, the CDRH2 comprises an amino acid sequence represented by SEQ ID NO: 32, and the CDRH3 comprises an amino acid sequence represented by SEQ ID NO: 33 or an amino acid sequence represented by SEQ ID NO: 49; and
the light chain sequence contains a variable region having CDRL1, CDRL2, and CDRL3, and the CDRL1 comprises an amino acid sequence represented by SEQ ID NO: 34, the CDRL2 comprises an amino acid sequence represented by SEQ ID NO: 35, and the CDRL3 comprises an amino acid sequence represented by SEQ ID NO: 36.

3. The antibody or an antigen binding fragment of the antibody according to claim 1 or 2, **characterized by** comprising a heavy chain variable region sequence comprising amino acid residues 1 to 121 of an amino acid sequence represented by SEQ ID NO: 2 and a light chain variable region sequence comprising amino acid residues 1 to 109 of an amino acid sequence represented by SEQ ID NO: 4.

4. The antigen binding fragment of the antibody according to claims 1 to 3, which is selected from the group consisting of Fab, F(ab')2, Fab' and Fv.

5. The antibody according to claims 1 to 3, **characterized by** being an scFv.

6. The antibody or an antigen binding fragment of the antibody according to any one of claims 1 to 4, **characterized in that** the antibody is a chimeric antibody.

7. The antibody or an antigen binding fragment of the antibody according to claim 6, **characterized by** comprising a heavy chain sequence comprising amino acid residues 20 to 466 of an amino acid sequence represented by SEQ ID NO: 8 and a light chain sequence comprising amino acid residues 21 to 234 of an amino acid sequence represented by SEQ ID NO: 10.

8. The antibody or an antigen binding fragment of the antibody according to claim 1, 2 or 4, **characterized in that** the antibody is humanized.

9. The antibody according to claim 6 or 8, wherein the heavy chain has a constant region of a human immunoglobulin G2 heavy chain and the light chain has a constant region of a human immunoglobulin κ light chain.

10. An antibody which inhibits osteoclast formation and/or osteoclastic bone resorption or an antigen binding fragment of the antibody, **characterized by** comprising:
(a) a heavy chain variable region sequence selected from the group consisting of the following amino acid sequences:
a1) an amino acid sequence comprising amino acid residues 20 to 140 of an amino acid sequence represented by SEQ ID NO: 12;
a2) an amino acid sequence comprising amino acid residues 20 to 140 of an amino acid sequence represented by SEQ ID NO: 14;
a3) an amino acid sequence comprising amino acid residues 20 to 140 of an amino acid sequence represented by SEQ ID NO: 16;
a4) an amino acid sequence comprising amino acid residues 20 to 140 of an amino acid sequence represented by SEQ ID NO: 18;
a5) an amino acid sequence comprising amino acid residues 20 to 140 of an amino acid sequence represented by SEQ ID NO: 20;
a6) an amino acid sequence comprising amino acid residues 20 to 140 of an amino acid sequence represented by SEQ ID NO: 22;
a7) an amino acid sequence comprising amino acid residues 20 to 140 of an amino acid sequence represented by SEQ ID NO: 38;
a8) an amino acid sequence comprising amino acid residues 20 to 140 of an amino acid sequence represented by SEQ ID NO: 40;
a9) an amino acid sequence comprising amino acid residues 20 to 140 of an amino acid sequence represented by SEQ ID NO: 42;
a10) an amino acid sequence comprising amino acid residues 20 to 140 of an amino acid sequence represented by SEQ ID NO: 44;
a11) an amino acid sequence having a homology of at least 95% with any one of the amino acid sequences selected from a1) to a10) ;
a12) an amino acid sequence having a homology of at least 99% with any one of the amino acid sequences selected from a1) to a10); and
a13) an amino acid sequence including substitution, deletion, or addition of one to several amino acid residues in any one of the amino acid sequences selected from a1) to a10) ; and
(b) a light chain variable region sequence selected from the group consisting of the following amino acid sequences:
b1) an amino acid sequence comprising amino acid residues 21 to 129 of an amino acid sequence represented by SEQ ID NO: 24;
b2) an amino acid sequence comprising amino acid residues 21 to 129 of an amino acid sequence represented by SEQ ID NO: 26;
b3) an amino acid sequence comprising amino acid residues 21 to 129 of an amino acid sequence represented by SEQ ID NO: 28;
b4) an amino acid sequence comprising amino acid residues 21 to 129 of an amino acid sequence represented by SEQ ID NO: 30;
b5) an amino acid sequence comprising amino acid residues 21 to 129 of an amino acid sequence represented by SEQ ID NO: 46;
b6) an amino acid sequence comprising amino acid residues 21 to 129 of an amino acid sequence represented by SEQ ID NO: 48;
b7) an amino acid sequence having a homology of at least 95% with any one of the amino acid sequences selected from b1) to b6) ;
b8) an amino acid sequence having a homology of at least 99% with any one of the amino acid sequences selected from b1) to b6); and
b9) an amino acid sequence including substitution, deletion, or addition of one to several amino acid residues in any one of the amino acid sequences selected from b1) to b6).

11. The antibody or an antigen binding fragment of the antibody according to claim 10, **characterized by** comprising a heavy chain variable region sequence comprising amino acid residues 20 to 140 of an amino acid sequence represented by SEQ ID NO: 14 and a light chain variable region sequence comprising amino acid residues 21 to 129 of an amino acid sequence represented by SEQ ID NO: 24.

12. The antibody or an antigen binding fragment of the antibody according to claim 10, **characterized by** comprising a heavy chain variable region sequence comprising amino acid residues 20 to 140 of an amino acid sequence represented by SEQ ID NO: 14 and a light chain variable region sequence comprising amino acid residues 21 to 129 of an amino acid sequence represented by SEQ ID NO: 26.

13. The antibody or an antigen binding fragment of the antibody according to claim 10, **characterized by** comprising a heavy chain variable region sequence comprising amino acid residues 20 to 140 of an amino acid sequence represented by SEQ ID NO: 14 and a light chain variable region sequence comprising amino acid residues 21 to 129 of an amino acid sequence represented by SEQ ID NO: 28.

14. The antibody or an antigen binding fragment of the antibody according to claim 10, **characterized by** comprising a heavy chain variable region sequence comprising amino acid residues 20 to 140 of an amino acid sequence represented by SEQ ID NO: 38 and a light chain variable region sequence comprising amino acid residues 21 to 129 of an amino acid sequence represented by SEQ ID NO: 48.

15. The antibody or an antigen binding fragment of the antibody according to claim 10, **characterized by** comprising a heavy chain variable region sequence comprising amino acid residues 20 to 140 of an amino acid sequence represented by SEQ ID NO: 40 and a light chain variable region sequence comprising amino acid residues 21 to 129 of an amino acid sequence represented by SEQ ID NO: 46.

16. The antibody or an antigen binding fragment of the antibody according to claim 10, **characterized by** comprising a heavy chain variable region sequence comprising amino acid residues 20 to 140 of an amino acid sequence represented by SEQ ID NO: 42 and a light chain variable region sequence comprising amino acid residues 21 to 129 of an amino acid sequence represented by SEQ ID NO: 24.

17. The antibody or an antigen binding fragment of the antibody according to claim 10, **characterized by** comprising a heavy chain variable region sequence comprising amino acid residues 20 to 140 of an amino acid sequence represented by SEQ ID NO: 44 and a light chain variable region sequence comprising amino acid residues 21 to 129 of an amino acid sequence represented by SEQ ID NO: 24.

18. The antibody or an antigen binding fragment of the antibody according to claim 10, **characterized by** comprising a heavy chain sequence comprising amino acid residues 20 to 466 of an amino acid sequence represented by SEQ ID NO: 14 and a light chain sequence comprising amino acid residues 21 to 234 of an amino acid sequence represented by SEQ ID NO: 24.

19. The antibody or an antigen binding fragment of the antibody according to claim 10, **characterized by** comprising a heavy chain sequence comprising amino acid residues 20 to 466 of an amino acid sequence represented by SEQ ID NO: 14 and a light chain sequence comprising amino acid residues 21 to 234 of an amino acid sequence represented by SEQ ID NO: 26.

20. The antibody or an antigen binding fragment of the antibody according to claim 10, **characterized by** comprising a heavy chain sequence comprising amino acid residues 20 to 466 of an amino acid sequence represented by SEQ ID NO: 14 and a light chain sequence comprising amino acid residues 21 to 234 of an amino acid sequence represented by SEQ ID NO: 28.

21. The antibody or an antigen binding fragment of the antibody according to claim 10, **characterized by** comprising a heavy chain sequence comprising amino acid residues 20 to 466 of an amino acid sequence represented by SEQ ID NO: 38 and a light chain sequence comprising amino acid residues 21 to 234 of an amino acid sequence represented by SEQ ID NO: 48.

22. The antibody or an antigen binding fragment of the antibody according to claim 10, **characterized by** comprising a heavy chain sequence comprising amino acid residues 20 to 466 of an amino acid sequence represented by SEQ ID NO: 40 and a light chain sequence comprising amino acid residues 21 to 234 of an amino acid sequence represented by SEQ ID NO: 46.

23. The antibody or a functional fragment of the antibody according to claim 10, **characterized by** comprising a heavy chain sequence comprising amino acid residues 20 to 466 of an amino acid sequence represented by SEQ ID NO: 42 and a light chain sequence comprising amino acid residues 21 to 234 of an amino acid sequence represented by SEQ ID NO: 24.

24. The antibody or an antigen binding fragment of the antibody according to claim 10, **characterized by** comprising a heavy chain sequence comprising amino acid residues 20 to 466 of an amino acid sequence represented by SEQ ID NO: 44 and a light chain sequence comprising amino acid residues 21 to 234 of an amino acid sequence represented by SEQ ID NO: 24.

25. The antibody according to any one of claims 11 to 24, wherein the antibody comprises a heavy chain including a deletion of one to several amino acids from the carboxyl group-terminal end thereof.

26. A pharmaceutical composition, **characterized by** comprising at least one of the antibodies or antigen binding fragments of the antibodies according to claims 1 to 25.

27. The pharmaceutical composition according to claim 26, **characterized by** being a therapeutic and/or prophylactic agent for abnormal bone metabolism.

28. A pharmaceutical composition for the treatment and/or prophylaxis of abnormal bone metabolism, **characterized by** comprising at least one of the antibodies or antigen binding fragments of the antibodies according to claims 1 to 25 and at least one selected from the group consisting of bisphosphonates, active vitamin D₃, calcitonin and derivatives thereof, hormones such as estradiol, SERMs (selective estrogen receptor modulators), ipriflavone, vitamin K₂ (menatetrenone), calcium preparations, PTH (parathyroid hormone), nonsteroidal anti-inflammatory agents, soluble TNF receptors, anti-TNF-α antibodies or antigen binding fragments of the antibodies, anti-PTHrP (parathyroid hormone-related protein) antibodies or antigen binding fragments of the antibodies, IL-1 receptor antagonists, anti-IL-6 receptor antibodies or antigen binding fragments of the antibodies, anti-RANKL antibodies or antigen binding fragments of the antibodies, and OCIF (osteoclastogenesis inhibitory factor).

29. The pharmaceutical composition according to claim 27 or 28, wherein the abnormal bone metabolism is selected from the group consisting of osteoporosis, bone destruction accompanying rheumatoid arthritis, cancerous hypercalcemia, bone destruction accompanying multiple myeloma or cancer metastasis to bone, giant cell tumor, osteopenia, tooth loss due to periodontitis, osteolysis around a prosthetic joint, bone destruction in chronic osteomyelitis, bone Paget's disease, renal osteodystrophy, and osteogenesis imperfecta.

30. The pharmaceutical composition according to claim 29, **characterized in that** the abnormal bone metabolism is osteoporosis, bone destruction accompanying rheumatoid arthritis, or bone destruction accompanying cancer metastasis to bone.

31. The pharmaceutical composition according to claim 30, **characterized in that** the abnormal bone metabolism is osteoporosis.

32. The pharmaceutical composition according to claim 31, **characterized in that** the osteoporosis is postmenopausal osteoporosis, senile osteoporosis, secondary osteoporosis due to the use of a therapeutic agent such as a steroid or an immunosuppressant, or osteoporosis accompanying rheumatoid arthritis.

33. A method for the treatment and/or prophylaxis of abnormal bone metabolism, **characterized by** administering at least one of the antibodies or antigen binding fragments of the antibodies according to claims 1 to 25 or the pharmaceutical composition according to claim 27 or 28.

34. A method for the treatment and/or prophylaxis of abnormal bone metabolism, **characterized by** simultaneously or successively administering at least one of the antibodies or antigen binding fragments of the antibodies according to claims 1 to 25 or the pharmaceutical composition according to claim 27 and at least one selected from the group consisting of bisphosphonates, active vitamin D₃, calcitonin and derivatives thereof, hormones such as estradiol, SERMs (selective estrogen receptor modulators), ipriflavone, vitamin K₂ (menatetrenone), calcium preparations, PTH (parathyroid hormone), nonsteroidal anti-inflammatory agents, soluble TNF receptors, anti-TNF-α antibodies or antigen binding fragments of the antibodies, anti-PTHrP (parathyroid hormone-related protein) antibodies or antigen binding fragments of the antibodies, IL-1 receptor antagonists, anti-IL-6 receptor antibodies or antigen binding fragments of the antibodies, anti-RANKL antibodies or antigen binding fragments of the antibodies, and OCIF (osteoclastogenesis inhibitory factor).

35. The method for the treatment and/or prophylaxis according to claim 33 or 34, **characterized in that** the abnormal bone metabolism is osteoporosis, bone destruction accompanying rheumatoid arthritis, or bone destruction accompanying cancer metastasis to bone.

36. The method for the treatment and/or prophylaxis according to claim 35, **characterized in that** the abnormal bone metabolism is osteoporosis.

37. The method for the treatment and/or prophylaxis according to claim 36, **characterized in that** the osteoporosis is postmenopausal osteoporosis, senile osteoporosis, secondary osteoporosis due to the use of a therapeutic agent such as a steroid or an immunosuppressant, or osteoporosis accompanying rheumatoid arthritis.

38. A polynucleotide encoding the antibody according to any one of claims 1 to 25.

39. The polynucleotide according to claim 38, **characterized by** comprising a nucleotide sequence comprising nucleotides 1 to 363 of a nucleotide sequence represented by SEQ ID NO: 1 and a nucleotide sequence comprising nucleotides 1 to 327 of a nucleotide sequence represented by SEQ ID NO: 3.

40. The polynucleotide according to claim 39, **characterized by** comprising a nucleotide sequence comprising nucleotides 58 to 1398 of a nucleotide sequence represented by SEQ ID NO: 7 and a nucleotide sequence comprising nucleotides 61 to 702 of a nucleotide sequence represented by SEQ ID NO: 9.

41. The polynucleotide according to claim 38, **characterized by** comprising:
(a) a polynucleotide selected from the group consisting of the following nucleotide sequences:
a1) a nucleotide sequence comprising nucleotides 58 to 420 of a nucleotide sequence represented by SEQ ID NO: 11;
a2) a nucleotide sequence comprising nucleotides 58 to 420 of a nucleotide sequence represented by SEQ ID NO: 13;
a3) a nucleotide sequence comprising nucleotides 58 to 420 of a nucleotide sequence represented by SEQ ID NO: 15;
a4) a nucleotide sequence comprising nucleotides 58 to 420 of a nucleotide sequence represented by SEQ ID NO: 17;
a5) a nucleotide sequence comprising nucleotides 58 to 420 of a nucleotide sequence represented by SEQ ID NO: 19;
a6) a nucleotide sequence comprising nucleotides 58 to 420 of a nucleotide sequence represented by SEQ ID NO: 21;
a7) a nucleotide sequence comprising nucleotides 58 to 420 of a nucleotide sequence represented by SEQ ID NO: 37;
a8) a nucleotide sequence comprising nucleotides 58 to 420 of a nucleotide sequence represented by SEQ ID NO: 39;
a9) a nucleotide sequence comprising nucleotides 58 to 420 of a nucleotide sequence represented by SEQ ID NO: 41;
a10) a nucleotide sequence comprising nucleotides 58 to 420 of a nucleotide sequence represented by SEQ ID NO: 43;
a11) an amino acid sequence having a homology of at least 95% with any one of the nucleotide sequences selected from a1) to a10) ;
a12) an amino acid sequence having a homology of at least 99% with any one of the nucleotide sequences selected from a1) to a10);
a13) a nucleotide sequence of a polynucleotide which hybridizes to a polynucleotide comprising a nucleotide sequence complementary to any one of the nucleotide sequences selected from a1) to a10) under stringent conditions; and
a14) a nucleotide sequence including substitution, deletion, or addition of one to several amino acid residues in any one of the nucleotide sequences selected from a1) to a10) ; and
(b) a polynucleotide selected from the group consisting of the following nucleotide sequences:
b1) a nucleotide sequence comprising nucleotides 61 to 387 of a nucleotide sequence represented by SEQ ID NO: 23;
b2) a nucleotide sequence comprising nucleotides 61 to 387 of a nucleotide sequence represented by SEQ ID NO: 25;
b3) a nucleotide sequence comprising nucleotides 61 to 387 of a nucleotide sequence represented by SEQ ID NO: 27;
b4) a nucleotide sequence comprising nucleotides 61 to 387 of a nucleotide sequence represented by SEQ ID NO: 28;
b5) a nucleotide sequence comprising nucleotides 61 to 387 of a nucleotide sequence represented by SEQ ID NO: 45;
b6) a nucleotide sequence comprising nucleotides 61 to 387 of a nucleotide sequence represented by SEQ ID NO: 47;
b7) an amino acid sequence having a homology of at least 95% with any one of the nucleotide sequences selected from b1) to b6);
b8) an amino acid sequence having a homology of at least 99% with any one of the nucleotide sequences selected from b1) to b6);
b9) a nucleotide sequence of a polynucleotide which hybridizes to a polynucleotide comprising a nucleotide sequence complementary to any one of the nucleotide sequences selected from b1) to b6) under stringent conditions; and
b10) a nucleotide sequence including substitution, deletion, or addition of one to several nucleotides in any one of the nucleotide sequences selected from b1) to b6).

42. The polynucleotide according to claim 41, **characterized by** comprising a polynucleotide comprising a nucleotide sequence comprising nucleotides 58 to 420 of a nucleotide sequence represented by SEQ ID NO: 13, and a polynucleotide comprising a nucleotide sequence comprising nucleotides 61 to 387 of a nucleotide sequence represented by SEQ ID NO: 23.

43. The polynucleotide according to claim 41, **characterized by** comprising a polynucleotide comprising a nucleotide sequence comprising nucleotides 58 to 420 of a nucleotide sequence represented by SEQ ID NO: 13, and a polynucleotide comprising a nucleotide sequence comprising nucleotides 61 to 387 of a nucleotide sequence represented by SEQ ID NO: 25.

44. The polynucleotide according to claim 41, **characterized by** comprising a polynucleotide comprising a nucleotide sequence comprising nucleotides 58 to 420 of a nucleotide sequence represented by SEQ ID NO: 13, and a polynucleotide comprising a nucleotide sequence comprising nucleotides 61 to 387 of a nucleotide sequence represented by SEQ ID NO: 27.

45. The polynucleotide according to claim 41, **characterized by** comprising a polynucleotide comprising a nucleotide sequence comprising nucleotides 58 to 420 of a nucleotide sequence represented by SEQ ID NO: 37, and a polynucleotide comprising a nucleotide sequence comprising nucleotides 61 to 387 of a nucleotide sequence represented by SEQ ID NO: 47.

46. The polynucleotide according to claim 41, **characterized by** comprising a polynucleotide comprising a nucleotide sequence comprising nucleotides 58 to 420 of a nucleotide sequence represented by SEQ ID NO: 39, and a polynucleotide comprising a nucleotide sequence comprising nucleotides 61 to 387 of a nucleotide sequence represented by SEQ ID NO: 45.

47. The polynucleotide according to claim 41, **characterized by** comprising a polynucleotide comprising a nucleotide sequence comprising nucleotides 58 to 420 of a nucleotide sequence represented by SEQ ID NO: 41, and a polynucleotide comprising a nucleotide sequence comprising nucleotides 61 to 387 of a nucleotide sequence represented by SEQ ID NO: 23.

48. The polynucleotide according to claim 41, **characterized by** comprising a polynucleotide comprising a nucleotide sequence comprising nucleotides 58 to 420 of a nucleotide sequence represented by SEQ ID NO: 43, and a polynucleotide comprising a nucleotide sequence comprising nucleotides 61 to 387 of a nucleotide sequence represented by SEQ ID NO: 23.

49. The polynucleotide according to claim 41, **characterized by** comprising a polynucleotide comprising a nucleotide sequence comprising nucleotides 58 to 1398 of a nucleotide sequence represented by SEQ ID NO: 13, and a polynucleotide comprising a nucleotide sequence comprising nucleotides 61 to 702 of a nucleotide sequence represented by SEQ ID NO: 23.

50. The polynucleotide according to claim 41, **characterized by** comprising a polynucleotide comprising a nucleotide sequence comprising nucleotides 58 to 1398 of a nucleotide sequence represented by SEQ ID NO: 13, and a polynucleotide comprising a nucleotide sequence comprising nucleotides 61 to 702 of a nucleotide sequence represented by SEQ ID NO: 25.

51. The polynucleotide according to claim 41, **characterized by** comprising a polynucleotide comprising a nucleotide sequence comprising nucleotides 58 to 1398 of a nucleotide sequence represented by SEQ ID NO: 13, and a polynucleotide comprising a nucleotide sequence comprising nucleotides 61 to 702 of a nucleotide sequence represented by SEQ ID NO: 27.

52. The polynucleotide according to claim 41, **characterized by** comprising a polynucleotide comprising a nucleotide sequence comprising nucleotides 58 to 1398 of a nucleotide sequence represented by SEQ ID NO: 37, and a polynucleotide comprising a nucleotide sequence comprising nucleotides 61 to 702 of a nucleotide sequence represented by SEQ ID NO: 47.

53. The polynucleotide according to claim 41, **characterized by** comprising a polynucleotide comprising a nucleotide sequence comprising nucleotides 58 to 1398 of a nucleotide sequence represented by SEQ ID NO: 39, and a polynucleotide comprising a nucleotide sequence comprising nucleotides 61 to 702 of a nucleotide sequence represented by SEQ ID NO: 45.

54. The polynucleotide according to claim 41, **characterized by** comprising a polynucleotide comprising a nucleotide sequence comprising nucleotides 58 to 1398 of a nucleotide sequence represented by SEQ ID NO: 41, and a polynucleotide comprising a nucleotide sequence comprising nucleotides 61 to 702 of a nucleotide sequence represented by SEQ ID NO: 23.

55. The polynucleotide according to claim 41, **characterized by** comprising a polynucleotide comprising a nucleotide sequence comprising nucleotides 58 to 1398 of a nucleotide sequence represented by SEQ ID NO: 43, and a polynucleotide comprising a nucleotide sequence comprising nucleotides 61 to 702 of a nucleotide sequence represented by SEQ ID NO: 23.

56. A vector, comprising any one of the polynucleotides according to claims 38 to 55.

57. A transformed host cell, comprising any one of the polynucleotides according to claims 38 to 55.

58. A transformed host cell, comprising the vector according to claim 56.

59. A method of producing the antibody according to any one of claims 1 to 25, comprising culturing the host cell according to claim 57 or 58, and purifying the antibody from the resulting culture product.
